# EUROPEAN PATENT SPECIFICATION

(11) **EP 2 675 915 B1**
(45) Date of publication and mention of the grant of the patent: **10.12.2014**
(21) Application number: 12709357.3
(22) Date of filing: 13.02.2012
(51) Int. Cl.: C12Q 1/68

(54) **CD4+ T-CELL GENE SIGNATURE FOR RHEUMATOID ARTHRITIS (RA)**
CD4+-T-ZELLEN-GENSIGNATUR FÜR RHEUMATOIDE ARTHRITIS (RA)
SIGNATURE DE GÈNE DE CELLULE T CD4+ POUR LA POLYARTHRITE RHUMATOÏDE (PR)

(30) Priority: 14.02.2011 GB 201102563; 25.05.2011 GB 201108818
(43) Date of publication of application: 25.12.2013
(73) Proprietor: University of Newcastle Upon Tyne, Tyne&Wear NE1 7RU (GB)
(72) Inventor: ISAACS, John D., Newcastle upon Tyne Tyne&Wear NE1 7RU (GB); PRATT, Arthur G., Newcastle upon Tyne Tyne&Wear NE1 7RU (GB)
(74) Representative: Rutherford, Claire
(86) International application number: PCT/GB2012/050315
(87) International publication number: WO 2012/110793

(56) References cited:
- WO-A1-03/072827
- WO-A2-2004/110244
- WO-A2-2010/008852
- DE-A1- 10 328 033
- US-A1- 2003 224 386
- LI JINGYI ET AL: "Altered microRNA expression profile with miR-146a upregulation in CD4+ T cells from patients with rheumatoid arthritis", ARTHRITIS RESEARCH AND THERAPY, BIOMED CENTRAL, LONDON, GB, vol. 12, no. 3, 11 May 2010 (2010-05-11), page R81, XP021085209, ISSN: 1478-6354, DOI: 10.1186/AR3006
- T Namekawa ET AL: "Functional subsets of CD4 T cells in rheumatoid synovitis", Arthritis and rheumatism, 1 January 1998 (1998-01-01), pages 2108-2116, XP055027090, DOI: doi:10.1002/1529-01 Retrieved from the Internet: URL:http://onlinelibrary.wiley.com/doi/10. 1002/1529-0131%28199812%2941:12%3C2108::AI D-ART5%3E3.0.CO;2-Q/pdf [retrieved on 2012-05-14]
- Michael Schirmer: "Resistance to apoptosis and elevated expression of Bcl-2 in clonally expanded CD4+CD28- T cells from rheumatoid arthritis patients", The Journal of Immunology, 15 July 1998 (1998-07-15), pages 2 1018--1025, XP055027092, Retrieved from the Internet: URL:http://www.jimmunol.org/content/161/2/ 1018.full.pdf#page=1&view=FitH [retrieved on 2012-05-14]
- ELLIOTT S F ET AL: "Bcl-3 is an interleukin-1-responsive gene in chondrocytes and synovial fibroblasts that activates transcription of the matrix metalloproteinase 1 gene", ARTHRITIS & RHEUMATISM, JOHN WILEY & SONS, INC, US, vol. 46, no. 12, 1 December 2002 (2002-12-01), pages 3230-3239, XP002372708, ISSN: 0004-3591, DOI: 10.1002/ART.10675

### SEQUENCE LISTING

<110> University of Newcastle Upon Tyne
<120> CD4+ T-Cell gene signature for Rheumatoid Arthritis
<130> 119460.WO.01
<150> GB1102563.2
   <151> 2011-02-14
<150> GB1108818.4
   <151> 2011-05-25
<160> 13
<170> PatentIn version 3.5
<210> 1
   <211> 1813
   <212> DNA
   <213> Homo sapiens
<300>
   <308> NM_005178.2
   <309> 2007-01-28
   <313> (1)..(1813)
<400> 1
   ccgtccccgg cggccccatg ccccgatgcc ccgcgggggc catggacgag gggcccgtgg 60
   acctgcgcac ccggcccaag gccgccggac tcccgggcgc cgcgctgccg ctccgcaagc 120
   gcccgctgcg cgcgccctcc ccggagcccg ccgctccccg cggcgctgcg ggccttgtcg 180
   tccccctgga ccctctgcgc ggcggctgcg acctgccggc ggtccccggg cccccccacg 240
   gcctggcccg gccggaggcg ctttactacc ccggagcctt actgcctttg taccccactc 300
   gggccatggg ctccccgttt cctctggtga acctgcctac acccctatac cccatgatgt 360
   gccccatgga acaccccctt tctgctgaca tcgccatggc cacccgtgca gatgaggacg 420
   gagacacgcc tctccatatt gctgtggtgc agggtaacct gccagctgtg caccggctgg 480
   tcaacctctt ccagcagggg ggccgggagc tcgacatcta caacaaccta cggcagacac 540
   cgctccacct ggctgtgatc accacattac cgtctgtggt ccggctcctg gtgacagctg 600
   gtgccagccc catggcgctg gaccgccatg gccagacggc cgctcacctg gcgtgcgagc 660
   accgcagccc gacctgcctg cgagccctgc tggacagcgc agctccgggc acgttggacc 720
   tggaggcccg caattatgac gggctcaccg ccctgcacgt ggcagtgaac accgagtgcc 780
   aagaaaccgt gcagctcttg ctagagcgcg gtgccgacat cgacgcagtg gacattaaga 840
   gcggccgctc cccgctcatc cacgccgtgg aaaacaacag ccttagcatg gtgcagctgc 900
   tgctgcagca cggcgccaac gtgaacgcgc aaatgtactc cggcagctcc gccctgcact 960
   cagcgtccgg ccgcgggctc ctcccgctgg tgcgcacgct ggtccgcagc ggcgctgaca 1020
   gcagcctcaa gaactgccac aacgacacgc cgctcatggt ggcgcgcagc cgcagggtca 1080
   tcgacatcct gagggggaag gccacccggc ctgcttccac ctcccagcca gacccctccc 1140
   ctgaccggag cgccaacacc tcccccgaga gcagcagccg cctcagctcc aatggtcttc 1200
   tctccgcatc accatcctcc tcaccctccc agtctccccc cagggacccc cctggattcc 1260
   ccatggctcc tcccaatttc ttccttcctt ccccatctcc acccgccttc ctgccctttg 1320
   ctggggtcct ccgaggccct ggccggccgg tgcccccctc cccagctcca ggaggcagct 1380
   gagggggatg ggggggcaga tcttggactc atgaggaggg gcccccctgc ccagaggggt 1440
   caacccttct ggaaactgtg aagatctgac ttcgcccccc ccccccccca tcttcgggac 1500
   caggatttgc acagaagcac atgcacctac ccatacaccc cctcttctga gcacagatgt 1560
   tcccccatct cgctccctcc caggactctg accccagcat tctcaggcac cagtccctgt 1620
   ccggaatgcc acccacatct tccatttcca tgtcccctcc cagagctggt ggacccaggg 1680
   aacagccact cccctccact ctctaccaga taactgagga ggggagaggt gggccgtaac 1740
   gggcacggat cacgatgtaa attattaagc attttggttg gatttctttt gtaataaact 1800
   atttttgtac cat 1813
<210> 2
   <211> 2746
   <212> DNA
   <213> Homo sapiens
<400> 2
   ggctccgact tggactccct gctccgctgc tgccgcttcg gccccgcacg cagccagccg        60
   ccagccgccc gcccggccca gctcccgccg cggccccttg ccgcggtccc tctcctggtc       120
   ccctcccggt tggtccgggg gtgcgcaggg ggcagggcgg gcgcccaggg gaagctcgag       180
   ggacgcgcgc gcgaaggctc ctttgtggac ttcacggccg ccaacatctg ggcgcagcgc       240
   gggccaccgc tggccgtctc gccgccgcgt cgccttgggg acccgagggg gctcagcccc       300
   aaggacggag acttcgattc gggaccagcc ccccgggatg cggtagcggc cgctgtgcgg       360
   aggccgcgaa gcagctgcag ccgccgccgc gcagatccac gctggctccg tgcgccatgg       420
   tcacccacag caagtttccc gccgccggga tgagccgccc cctggacacc agcctgcgcc       480
   tcaagacctt cagctccaag agcgagtacc agctggtggt gaacgcagtg cgcaagctgc       540
   aggagagcgg cttctactgg agcgcagtga ccggcggcga ggcgaacctg ctgctcagtg       600
   ccgagcccgc cggcaccttt ctgatccgcg acagctcgga ccagcgccac ttcttcacgc       660
   tcagcgtcaa gacccagtct gggaccaaga acctgcgcat ccagtgtgag gggggcagct       720
   tctctctgca gagcgatccc cggagcacgc agcccgtgcc ccgcttcgac tgcgtgctca       780
   agctggtgca ccactacatg ccgccccctg gagccccctc cttcccctcg ccacctactg       840
   aaccctcctc cgaggtgccc gagcagccgt ctgcccagcc actccctggg agtcccccca 900
   gaagagccta ttacatctac tccgggggcg agaagatccc cctggtgttg agccggcccc 960
   tctcctccaa cgtggccact cttcagcatc tctgtcggaa gaccgtcaac ggccacctgg 1020
   actcctatga gaaagtcacc cagctgccgg ggcccattcg ggagttcctg gaccagtacg 1080
   atgccccgct ttaaggggta aagggcgcaa agggcatggg tcgggagagg ggacgcaggc 1140
   ccctctcctc cgtggcacat ggcacaagca caagaagcca accaggagag agtcctgtag 1200
   ctctgggggg aaagagggcg gacaggcccc tccctctgcc ctctccctgc agaatgtggc 1260
   aggcggacct ggaatgtgtt ggagggaagg gggagtacca cctgagtctc cagcttctcc 1320
   ggaggagcca gctgtcctgg tgggacgata gcaaccacaa gtggattctc cttcaattcc 1380
   tcagcttccc ctctgcctcc aaacagggga cacttcggga atgctgaact aatgagaact 1440
   gccagggaat cttcaaactt tccaacggaa cttgtttgct ctttgatttg gtttaaacct 1500
   gagctggttg tggagcctgg gaaaggtgga agagagagag gtcctgaggg ccccagggct 1560
   gcgggctggc gaaggaaatg gtcacacccc ccgcccaccc caggcgagga tcctggtgac 1620
   atgctcctct ccctggctcc ggggagaagg gcttggggtg acctgaaggg aaccatcctg 1680
   gtaccccaca tcctctcctc cgggacagtc accgaaaaca caggttccaa agtctacctg 1740
   gtgcctgaga gcccagggcc cttcctccgt tttaaggggg aagcaacatt tggaggggat 1800
   ggatgggctg gtcagctggt ctccttttcc tactcatact ataccttcct gtacctgggt 1860
   ggatggagcg ggaggatgga ggagacggga catctttcac ctcaggctcc tggtagagaa 1920
   gacaggggat tctactctgt gcctcctgac tatgtctggc taagagattc gccttaaatg 1980
   ctccctgtcc catggagagg gacccagcat aggaaagcca catactcagc ctggatgggt 2040
   ggagaggctg agggactcac tggagggcac caagccagcc cacagccagg gaagtgggga 2100
   gggggggcgg aaacccatgc ctcccagctg agcactggga atgtcagccc agtaagtatt 2160
   ggccagtcag gcgcctcgtg gtcagagcag agccaccagg tcccactgcc ccgagccctg 2220
   cacagccctc cctcctgcct gggtggggga ggctggaggt cattggagag gctggactgc 2280
   tgccaccccg ggtgctcccg ctctgccata gcactgatca gtgacaattt acaggaatgt 2340
   agcagcgatg gaattacctg gaacagtttt ttgtttttgt ttttgttttt gtttttgtgg 2400
   gggggggcaa ctaaacaaac acaaagtatt ctgtgtcagg tattgggctg gacagggcag 2460
   ttgtgtgttg gggtggtttt tttctctatt tttttgtttg tttcttgttt tttaataatg 2520
   tttacaatct gcctcaatca ctctgtcttt tataaagatt ccacctccag tcctctctcc 2580
   tcccccctac tcaggccctt gaggctatta ggagatgctt gaagaactca acaaaatccc 2640
   aatccaagtc aaactttgca catatttata tttatattca gaaaagaaac atttcagtaa 2700
   tttataataa agagcactat tttttaatga aaaaaaaaaa aaaaaa 2746
<210> 3
   <211> 2684
   <212> DNA
   <213> Homo sapiens
<400> 3
   cccgagagga gtcggtggca gcggcggcgg cgggaccggc agcagcagca gcagcagcag 60
   cagcaaccac tagcctcctg ccccgcggcg ctgccgcacg agccccacga gccgctcacc 120
   ccgccgttct cagcgctgcc cgaccccgct ggcgcgccct cccgccgcca gtcccggcag 180
   cgccctcagt tgtcctccga ctcgccctcg gccttccgcg ccagccgcag ccacagccgc 240
   aacgccaccc gcagccacag ccacagccac agccccaggc atagccttcg gcacagcccc 300
   ggctccggct cctgcggcag ctcctctggg caccgtccct gcgccgacat cctggaggtt 360
   gggatgctct tgtccaaaat caactcgctt gcccacctgc gcgccgcgcc ctgcaacgac 420
   ctgcacgcca ccaagctggc gcccggcaag gagaaggagc ccctggagtc gcagtaccag 480
   gtgggcccgc tactgggcag cggcggcttc ggctcggtct actcaggcat ccgcgtctcc 540
   gacaacttgc cggtggccat caaacacgtg gagaaggacc ggatttccga ctggggagag 600
   ctgcctaatg gcactcgagt gcccatggaa gtggtcctgc tgaagaaggt gagctcgggt 660
   ttctccggcg tcattaggct cctggactgg ttcgagaggc ccgacagttt cgtcctgatc 720
   ctggagaggc ccgagccggt gcaagatctc ttcgacttca tcacggaaag gggagccctg 780
   caagaggagc tggcccgcag cttcttctgg caggtgctgg aggccgtgcg gcactgccac 840
   aactgcgggg tgctccaccg cgacatcaag gacgaaaaca tccttatcga cctcaatcgc 900
   ggcgagctca agctcatcga cttcgggtcg ggggcgctgc tcaaggacac cgtctacacg 960
   gacttcgatg ggacccgagt gtatagccct ccagagtgga tccgctacca tcgctaccat 1020
   ggcaggtcgg cggcagtctg gtccctgggg atcctgctgt atgatatggt gtgtggagat 1080
   attcctttcg agcatgacga agagatcatc aggggccagg ttttcttcag gcagagggtc 1140
   tcttcagaat gtcagcatct cattagatgg tgcttggccc tgagaccatc agataggcca 1200
   accttcgaag aaatccagaa ccatccatgg atgcaagatg ttctcctgcc ccaggaaact 1260
   gctgagatcc acctccacag cctgtcgccg gggcccagca aatagcagcc tttctggcag 1320
   gtcctcccct ctcttgtcag atgcccgagg gaggggaagc ttctgtctcc agcttcccga 1380
   gtaccagtga cacgtctcgc caagcaggac agtgcttgat acaggaacaa catttacaac 1440
   tcattccaga tcccaggccc ctggaggctg cctcccaaca gtggggaaga gtgactctcc 1500
   aggggtccta ggcctcaact cctcccatag atactctctt cttctcatag gtgtccagca 1560
   ttgctggact ctgaaatatc ccgggggtgg ggggtggggg tgggtcagaa ccctgccatg 1620
   gaactgtttt cttcatcatg agttctgctg aatgccgcga tgggtcaggt aggggggaaa 1680
   caggttggga tgggatagga ctagcaccat tttaagtccc tgtcacctct tccgactctt 1740
   tctgagtgcc ttctgtgggg actccggctg tgctgggaga aatacttgaa cttgcctctt 1800
   ttacctgctg cttctccaaa aatctgcctg ggttttgttc cctatttttc tctcctgtcc 1860
   tccctcaccc cctccttcat atgaaaggtg ccatggaaga ggctacaggg ccaaacgctg 1920
   agccacctgc ccttttttct gcctccttta gtaaaactcc gagtgaactg gtcttccttt 1980
   ttggttttta cttaactgtt tcaaagccaa gacctcacac acacaaaaaa tgcacaaaca 2040
   atgcaatcaa cagaaaagct gtaaatgtgt gtacagttgg catggtagta tacaaaaaga 2100
   ttgtagtgga tctaattttt aagaaatttt gcctttaagt tattttacct gtttttgttt 2160
   cttgttttga aagatgcgca ttctaacctg gaggtcaatg ttatgtattt atttatttat 2220
   ttatttggtt cccttcctat tccaagcttc catagctgct gccctagttt tctttcctcc 2280
   tttcctcctc tgacttgggg accttttggg ggagggctgc gacgcttgct ctgtttgtgg 2340
   ggtgacggga ctcaggcggg acagtgctgc agctccctgg cttctgtggg gcccctcacc 2400
   tacttaccca ggtgggtccc ggctctgtgg gtgatgggga ggggcattgc tgactgtgta 2460
   tataggataa ttatgaaaag cagttctgga tggtgtgcct tccagatcct ctctggggct 2520
   gtgttttgag cagcaggtag cctgctggtt ttatctgagt gaaatactgt acaggggaat 2580
   aaaagagatc ttattttttt ttttatactt ggcgtttttt gaataaaaac cttttgtctt 2640
   aaaaaaaaaa aaaaaaaaaa aaaaaaaaaa aaaaaaaaaa aaaa 2684
<210> 4
   <211> 4948
   <212> DNA
   <213> Homo sapiens
<400> 4
   gtgcgcgggg cccccaagat ggcgcccgaa acccggaagt gagcggcggc agctgcgagg      60
   ctcggagaaa caggcgccgc gggctccgcg cccggccgga cccgggcccg agatcatgat     120
   gctgccgcca ccgccgccac cacggagcga gaagcccaga tagacgcccc ggcggccccg     180
   ggtcctggag tcccgccgcc tgctgcccgg ccgaggaccc caccccgcct gccgcccgat     240
   gcttgcagtg gggcccgcca tggacaggga ttacccgcag catgaacccc cgccggcggg     300
   cagcctcctg tacagcccgc cgcccctgca gagcgccatg ctgcactgcc cctactggaa     360
   caccttctcg ctgccgccat accctgcctt ctccagcgac agccgcccgt tcatgagctc     420
   cgcctccttc ctcggcagcc agccctgccc agacaccagc tatgcccccg tggccaccgc     480
   ctccagcttg ccaccaaaga cctgcgactt tgctcaggac tcctcctatt ttgaggactt     540
   ctccaacatc tccatcttct cctcgtccgt ggactccctg tcggacatcg tggacacgcc     600
   cgacttcctg ccggctgaca gcctcaacca ggtgtccacc atctgggacg ataaccctgc     660
   cccctccacc cacgataagc tgttccagct cagcaggccg tttgcaggct tcgaggactt 720
   tctgccctcc cacagcaccc cgcttctcgt cagctaccag gagcagagtg tgcagagcca 780
   gccagaggag gaggacgagg ctgaggagga ggaggcggag gagctggggc acacagagac 840
   ctacgccgac tacgtgccgt ccaagtccaa gatcgggaag cagcacccag accgcgtggt 900
   ggagaccagc acactgtcca gcgtcccacc cccagacatc acctacaccc tggccctgcc 960
   ctcggacagc ggggccctgt ctgccctgca gctagaggcc atcacctacg cctgccagca 1020
   acacgaggtc ctgctcccca gcgggcagcg cgcgggcttt ctcatcggcg atggggccgg 1080
   cgtgggcaaa ggccggacgg tggccggagt catcctggag aaccacctgc gcggccggaa 1140
   gaaagcattg tggttcagcg tctccaacga cctcaagtac gatgcggagc gcgacctgcg 1200
   ggacatcgaa gccacgggca tcgcggtgca cgcgctcagc aagatcaagt acggtgacac 1260
   cactacctca gagggcgtcc tcttcgccac ctactccgcc ctgattgggg agagccaggc 1320
   cggcggccag caccgcactc gcctccggca gatcctggac tggtgtgggg aggccttcga 1380
   gggcgtcatc gtgttcgacg agtgtcacaa agccaagaat gccggctcca ccaagatggg 1440
   caaggctgtg ctagacctgc agaacaagct gcccctggcc cgcgtggtct acgccagcgc 1500
   cacaggtgcc tctgagcctc ggaacatgat ctacatgagc cgcttgggta tctggggcga 1560
   gggcacaccc ttccggaact ttgaggagtt cctgcacgcc atcgagaaga ggggcgttgg 1620
   cgccatggag atcgtggcca tggacatgaa ggtcagcggc atgtacatcg cacgccagct 1680
   cagcttctcc ggcgtcacct tccgcatcga ggagatcccg ctggccccag ccttcgagtg 1740
   cgtctacaac cgcgcggccc tgctgtgggc cgaggccctg aacgtgttcc agcaggcggc 1800
   cgactggatc ggcctggagt cgcgcaagtc cctgtggggc cagttctggt cggcacacca 1860
   gcgcttcttc aagtatctgt gcatcgcagc caaggtgcgc cggctggtgg agctggcccg 1920
   agaggagctg gcgcgagaca agtgcgtggt catcgggctg cagtccacgg gcgaggcgcg 1980
   cacgcgggag gtgctggggg agaacgatgg gcacctcaac tgcttcgtct cggccgctga 2040
   aggcgtgttc ctgtcgctaa ttcagaagca ctttccgtcc accaagagaa agcgggacag 2100
   aggagcgggc agcaagcgga aacggcgacc tcggggacgc ggggccaaag ccccccggct 2160
   ggcgtgcgag acagcgggcg tcatccgcat cagtgacgac agcagcacgg agtcggaccc 2220
   tggcctggac agcgacttca actcctcccc cgagtccctg gtggatgacg acgttgtcat 2280
   cgttgatgca gtcgggctcc ccagtgacga ccggggaccc ctgtgcctcc tgcagagaga 2340
   cccgcatggc cccggggtcc tggagcgggt ggagcggctg aagcaggatc tgctggacaa 2400
   agtgcggcgg ctgggccggg aactgccagt caacaccctg gacgagctca tcgaccagct 2460
   gggcggcccc cagcgggtgg cggagatgac cggcaggaaa ggccgcgtgg tgtccaggcc 2520
   cgacgggacg gtggccttcg agtcgcgggc agagcagggt ctgtccatcg accacgtgaa 2580
   cctcagggag aagcagcgct tcatgagcgg cgagaagctc gtggccatca tctcggaggc 2640
   ctccagctcg ggtgtctccc tccaagccga ccgccgtgtc cagaaccagc ggcgccgcgt 2700
   gcacatgacc ttggagctgc cgtggagcgc cgaccgcgcc atccagcagt tcggccgcac 2760
   ccaccggtcc aaccaggtct ccgcgccaga gtatgtcttc ctcatctcgg agctggccgg 2820
   ggagcgccgg ttcgcctcca tcgtggccaa gcgcctggag agtctggggg ccctgaccca 2880
   cggagaccgc cgcgccacgg agtcccgtga cctcagcaag tacaactttg agaacaagta 2940
   tggcacccgg gccctgcact gtgtcctcac caccatcctg agccagactg agaacaaagt 3000
   gcctgtgccc cagggatacc ctggaggggt ccccaccttc ttccgggaca tgaagcaggg 3060
   cctgctgtct gtgggcattg gtggccggga gtcccggaat ggctgcctgg acgtggagaa 3120
   ggactgttcc atcaccaagt tcctgaaccg catcctgggg ctggaggtgc acaagcagaa 3180
   cgccctgttc cagtacttct cagacacctt cgaccacctc atcgagatgg acaagcggga 3240
   gggcaaatac gacatgggca tcctggacct tgctcccggt atcgaggaga tctacgagga 3300
   gagccagcag gtgttcctgg ctcccgggca cccgcaggac gggcaggtgg tcttctacaa 3360
   gatcagcgtg gaccgcggcc tgaagtggga ggacgccttt gccaagtcgc tggcgctgac 3420
   gggcccctat gacggcttct acctctccta caaggtccgc ggtaacaagc ccagctgcct 3480
   gctggcggag cagaaccgcg gccagttctt cacggtgtac aagcccaaca tcggccggca 3540
   gagccagctg gaggccctgg acagcctccg ccgcaagttc caccgggtca ccgcggagga 3600
   ggccaaggag ccctgggaga gtggctacgc tttgtcgctg acgcactgca gccacagcgc 3660
   ctggaaccgg cactgccggc tggcgcagga gggtaaggac tgcctgcagg ggctgcggct 3720
   gcggcaccac tacatgctgt gcggcgcgct gctgcgcgtg tggggccgca tcgccgccgt 3780
   catggccgac gtcagcagca gcagctacct gcagatcgtg cggctgaaga ccaaggacag 3840
   gaagaagcaa gtgggcatca agatccccga gggctgcgtg cgccgggtgc tgcaggagct 3900
   gcggctgatg gatgcggacg tgaagcgcag gcaggcgccc gccctgggct gccccgcccc 3960
   gcccgccccg cgcccgctgg cgctgccttg cggccccgga gaggtgctgg acctcaccta 4020
   cagccccccg gccgaggcct tcccgccgcc cccgcacttc tctttcccgg cgccgctgtc 4080
   cctggacgcc ggccccggcg tcgtgccgct gggcaccccc gacgcccagg ccgaccctgc 4140
   ggccctcgcg caccagggct gcgacatcaa cttcaaggag gtgctggagg acatgctgcg 4200
   ctcgctgcac gcggggccgc cctccgaggg cgcgctgggg gagggcgcgg gggcgggggg 4260
   cgcggcgggc ggtggtcccg agcggcagag cgtgatccag ttcagcccac ccttccccgg 4320
   cgcccaggct cctctctgac acgcctttag gcgaaacatg ccccaagaca cagggaccgt 4380
   ttctccccta ggagcagcgg tggggagcag ggccaaggtc ccctgaccac tgctcagagg 4440
   agccctaggc cctggccgca gtgccttcag cgcccgaccc gggcccccac ctggtcagcc 4500
   ctggcggggc ccactcagga cagctggggg ccggggcgtg gcagggccct ctctgtgcct 4560
   ctcctcccaa gtaggaaggg gctccgggtg gctgctctgg gactgggcac ccacaagggc 4620
   tcagtgggcc caaacccttg aaatccgtga aaccgggtgg tcccaagagc tagaaactca 4680
   ggaaacccca ggtgctcagg gccccgcgtc tcgggggctc cgtggggcag acccctgcta 4740
   atatatgcaa ttctccctcc cccagccctt ccctgacccc taagttattg cccgctcacc 4800
   tctcccaggc cccaggccgc ggagctggca gggtggcgcc tgcggtttct atgtatttat 4860
   agcaagttct gatgtacata tgtaaaggac ttttttaaat atatgtgcct tttgcctact 4920
   tcccaaaaaa aaaaaaaaaa aaaaaaaa 4948
<210> 5
   <211> 1661
   <212> DNA
   <213> Homo sapiens
<400> 5
   tgctgcagcc gctgccgccg attccggatc tcattgccac gcgcccccga cgaccgcccg 60
   acgtgcattc ccgattcctt ttggttccaa gtccaatatg gcaactctaa aggatcagct 120
   gatttataat cttctaaagg aagaacagac cccccagaat aagattacag ttgttggggt 180
   tggtgctgtt ggcatggcct gtgccatcag tatcttaatg aaggacttgg cagatgaact 240
   tgctcttgtt gatgtcatcg aagacaaatt gaagggagag atgatggatc tccaacatgg 300
   cagccttttc cttagaacac caaagattgt ctctggcaaa gactataatg taactgcaaa 360
   ctccaagctg gtcattatca cggctggggc acgtcagcaa gagggagaaa gccgtcttaa 420
   tttggtccag cgtaacgtga acatatttaa attcatcatt cctaatgttg taaaatacag 480
   cccgaactgc aagttgctta ttgtttcaaa tccagtggat atcttgacct acgtggcttg 540
   gaagataagt ggttttccca aaaaccgtgt tattggaagt ggttgcaatc tggattcagc 600
   ccgattccgt tacctgatgg gggaaaggct gggagttcac ccattaagct gtcatgggtg 660
   ggtccttggg gaacatggag attccagtgt gcctgtatgg agtggaatga atgttgctgg 720
   tgtctctctg aagactctgc acccagattt agggactgat aaagataagg aacagtggaa 780
   agaggttcac aagcaggtgg ttgagagtgc ttatgaggtg atcaaactca aaggctacac 840
   atcctgggct attggactct ctgtagcaga tttggcagag agtataatga agaatcttag 900
   gcgggtgcac ccagtttcca ccatgattaa gggtctttac ggaataaagg atgatgtctt 960
   ccttagtgtt ccttgcattt tgggacagaa tggaatctca gaccttgtga aggtgactct 1020
   gacttctgag gaagaggccc gtttgaagaa gagtgcagat acactttggg ggatccaaaa 1080
   ggagctgcaa ttttaaagtc ttctgatgtc atatcatttc actgtctagg ctacaacagg 1140
   attctaggtg gaggttgtgc atgttgtcct ttttatctga tctgtgatta aagcagtaat 1200
   attttaagat ggactgggaa aaacatcaac tcctgaagtt agaaataaga atggtttgta 1260
   aaatccacag ctatatcctg atgctggatg gtattaatct tgtgtagtct tcaactggtt 1320
   agtgtgaaat agttctgcca cctctgacgc accactgcca atgctgtacg tactgcattt 1380
   gccccttgag ccaggtggat gtttaccgtg tgttatataa cttcctggct ccttcactga 1440
   acatgcctag tccaacattt tttcccagtg agtcacatcc tgggatccag tgtataaatc 1500
   caatatcatg tcttgtgcat aattcttcca aaggatctta ttttgtgaac tatatcagta 1560
   gtgtacatta ccatataatg taaaaagatc tacatacaaa caatgcaacc aactatccaa 1620
   gtgttatacc aactaaaacc cccaataaac cttgaacagt g 1661
<210> 6
   <211> 2484
   <212> DNA
   <213> Homo sapiens
<400> 6
   catcgaacaa acaactgaga tcctgttgtg cctatcacct gttgaagttg ccagtcttaa      60
   ggaaggaatc aatttctttc gcaataagag cactggcaaa gactacgtct tgtacaagaa     120
   taagagccga ctgagggcat gcaagaatat gtgcaagcac caaggaggcc tgttcataaa     180
   agatatcgag gatttagccg gaagttgttg aaatggatga aaacaacgga cttttgcttt     240
   tagaactgaa tcctcctaac ccttgggact tacagcccag atctcctgaa gagttggctt     300
   ttggagaagt acagataaca tatctcactc atgcctgcat ggacctcaag ttaggagaca     360
   agagaatggt gtttgaccct tggttaatcg gtcctgcttt tgcccgtgga tggtggttgc     420
   tccatgagcc tccatctgat tggctggaga ggctgtgcca ggcagacctc atttacatca     480
   gtcatctgca ctcagaccac ctgagttacc ccacactgaa aaagcttgct gggagaagac     540
   cagatattcc catttatgtt ggaaacacag aaaggcctgt attttggaat ctgaatcaga     600
   gcggtgtcca gttgactaat atcaatgtcg tgccatttgg aatatggcag caggtggaca     660
   aaaatcttcg attcatgatc ttgatggacg gtgttcatcc tgagatggac acttgcatta     720
   ttgtggagta caaaggtcat aaaatactca atatagtaga ctgcaccaga cccaatgggg     780
   gaaggctgcc tatgaaggtt gctctaatga tgagtgattt tgctggagga gcatcaggct     840
   ttccaatgac tttcagtggt ggaaaattta cggaggaatg gaaagcccaa ttcattaaaa     900
   cagaaaggaa gaagctcctg aactacaagg ctcggctggt gaagaacctg caaccccgaa     960
   tttattgtcc ctttgctggg tattttgtgg aatctcaccc atcagacaag tacatcaagg    1020
   aaacaaacac caaaaatgac ccaaatgaac tcaacaatct tatcaagaaa aactctgatg    1080
   tgataacatg gacccctcga ccgggagcca cccttgatct gggaagaatg ctgaaggatc    1140
   gaacagacag caagggcatc atagagcctc cagaggggac aaaaatttac aaggattcct 1200
   gggattttga accttatttg gaaatcttga atgctgctct aggagatgaa atatttcttc 1260
   actcatcctg gataaaagaa tacttcactt gggctggatt taaagattac aaccttgtgg 1320
   tcaggatgat tgagacagat gaggacttca atccttttcc tggaggatat gactatttgg 1380
   ttgacttttt agatttatcc ttcccaaaag aaagaccaca acgagaacat ccctatgagg 1440
   aaatccatag ccgggtggat gtcatcagac acgtggtgaa gaatggtcta ctctgggatg 1500
   agttgtatat aggattccaa acacggctcc agcgggatcc tgacatatac catcacctgt 1560
   tttggaatca ttttcaaata aaactccccc tcacaccacc caactggaag tcattcctga 1620
   tgtgctgtga gcagaatggg cctgtgattt tgcaagaatg caaaaccaca tgaaaatttc 1680
   aagaattcac tgatctgatg caaaataaaa atttatcatt acatcttgaa cccaggaagc 1740
   ttacagcaaa gagactatgc tttatgacgt cagcaataga taattccacg ttgcctttgt 1800
   gatttgtata tatagcttac atttgtggat cactacatag ccagattcaa aaatatttta 1860
   cttgttccat ccacagttct ctacagaaag aaccaatgaa cccaatagga acaaattctc 1920
   tgtggaaaac aaagcatagc tgtagtagat acgaatccaa tcacagagga aacaggaaga 1980
   gaaaaacatc caagactaca gtgaaaactg gaaatggtct gttttcgtga tattcgtatg 2040
   attaagatgc aaattttttc ttaggaaaat gtgattgtta actagcattc tgttttacat 2100
   gttgacattt ctaacacaca caccactgat ttgaacttca aaatttattt tctgattata 2160
   tatgctaggt ctgattctga agatacaaga attcaatggt ggaatttgtc tcctgaaatt 2220
   tctagattca taattagaag tgtttattac caacttctca cttagattaa catcagtgat 2280
   gtttgattat tctggttaac cgctcacatg cataacaata atgctagaaa ttcaggaatt 2340
   ataattttgt gatttatact gtgtggtaaa ttattcagtt ctcactgcaa ataaatataa 2400
   tgtatatcaa tgtaatgtag gtcagaatgt ctatggtgag caaataaaag attattttaa 2460
   aatatgaaaa aaaaaaaaaa aaaa 2484
<210> 7
   <211> 1279
   <212> DNA
   <213> Homo sapiens
<400> 7
   cagcgaggag ccggcgcctc ccgcgccccg cggtcgccct ggagtaattt cggatgccca 60
   gccgcggccg ccttccccag tagacccggg agaggagttg cggccaactt gtgtgccttt 120
   cttccgcccc ggtgggagcc ggcgctgcgc gaagggctct cccggcggct catgctgccg 180
   gccctgcgcc tgcccagcct cgggtgagcc gcctccggag agacggggga gcgcggcggc 240
   gccgcgggct cggcgtgctc tcctccgggg acgcgggacg aagcagcagc cccgggcgcg 300
   cgccagaggc atggagcgct gccccagcct aggggtcacc ctctacgccc tggtggtggt 360
   cctggggctg cgggcgacac cggccggcgg ccagcactat ctccacatcc gcccggcacc 420
   cagcgacaac ctgcccctgg tggacctcat cgaacaccca gaccctatct ttgaccccaa 480
   ggaaaaggat ctgaacgaga cgctgctgcg ctcgctgctc gggggccact acgacccagg 540
   cttcatggcc acctcgcccc ccgaggaccg gcccggcggg ggcgggggtg cagctggggg 600
   cgcggaggac ctggcggagc tggaccagct gctgcggcag cggccgtcgg gggccatgcc 660
   gagcgagatc aaagggctag agttctccga gggcttggcc cagggcaaga agcagcgcct 720
   aagcaagaag ctgcggagga agttacagat gtggctgtgg tcgcagacat tctgccccgt 780
   gctgtacgcg tggaacgacc tgggcagccg cttttggccg cgctacgtga aggtgggcag 840
   ctgcttcagt aagcgctcgt gctccgtgcc cgagggcatg gtgtgcaagc cgtccaagtc 900
   cgtgcacctc acggtgctgc ggtggcgctg tcagcggcgc gggggccagc gctgcggctg 960
   gattcccatc cagtacccca tcatttccga gtgcaagtgc tcgtgctaga actcgggggc 1020
   cccctgcccg cacccggaca cttgatcgat ccccaccgac gccccctgca ccgcctccaa 1080
   ccagttccac caccctctag cgagggtttt caatgaactt tttttttttt tttttttttt 1140
   ttctgggcta cagagaccta gctttctggt tcctgtaatg cactgtttaa ctgtgtagga 1200
   atgtatatgt gtgtgtatat acggtcccag ttttaattta cttattaaaa ggtcagtatt 1260
   atacgttaaa aaaaaaaaa 1279
<210> 8
   <211> 2106
   <212> DNA
   <213> Homo sapiens
<400> 8
   agtttccctt ccgctcacct ccgcctgagc agtggagaag gcggcactct ggtggggctg      60
   ctccaggcat gcagatccca caggcgccct ggccagtcgt ctgggcggtg ctacaactgg     120
   gctggcggcc aggatggttc ttagactccc cagacaggcc ctggaacccc cccaccttct     180
   tcccagccct gctcgtggtg accgaagggg acaacgccac cttcacctgc agcttctcca     240
   acacatcgga gagcttcgtg ctaaactggt accgcatgag ccccagcaac cagacggaca     300
   agctggccgc cttccccgag gaccgcagcc agcccggcca ggactgccgc ttccgtgtca     360
   cacaactgcc caacgggcgt gacttccaca tgagcgtggt cagggcccgg cgcaatgaca     420
   gcggcaccta cctctgtggg gccatctccc tggcccccaa ggcgcagatc aaagagagcc     480
   tgcgggcaga gctcagggtg acagagagaa gggcagaagt gcccacagcc caccccagcc     540
   cctcacccag gccagccggc cagttccaaa ccctggtggt tggtgtcgtg ggcggcctgc     600
   tgggcagcct ggtgctgcta gtctgggtcc tggccgtcat ctgctcccgg gccgcacgag     660
   ggacaatagg agccaggcgc accggccagc ccctgaagga ggacccctca gccgtgcctg 720
   tgttctctgt ggactatggg gagctggatt tccagtggcg agagaagacc ccggagcccc 780
   ccgtgccctg tgtccctgag cagacggagt atgccaccat tgtctttcct agcggaatgg 840
   gcacctcatc ccccgcccgc aggggctcag ccgacggccc tcggagtgcc cagccactga 900
   ggcctgagga tggacactgc tcttggcccc tctgaccggc ttccttggcc accagtgttc 960
   tgcagaccct ccaccatgag cccgggtcag cgcatttcct caggagaagc aggcagggtg 1020
   caggccattg caggccgtcc aggggctgag ctgcctgggg gcgaccgggg ctccagcctg 1080
   cacctgcacc aggcacagcc ccaccacagg actcatgtct caatgcccac agtgagccca 1140
   ggcagcaggt gtcaccgtcc cctacaggga gggccagatg cagtcactgc ttcaggtcct 1200
   gccagcacag agctgcctgc gtccagctcc ctgaatctct gctgctgctg ctgctgctgc 1260
   tgctgctgcc tgcggcccgg ggctgaaggc gccgtggccc tgcctgacgc cccggagcct 1320
   cctgcctgaa cttgggggct ggttggagat ggccttggag cagccaaggt gcccctggca 1380
   gtggcatccc gaaacgccct ggacgcaggg cccaagactg ggcacaggag tgggaggtac 1440
   atggggctgg ggactcccca ggagttatct gctccctgca ggcctagaga agtttcaggg 1500
   aaggtcagaa gagctcctgg ctgtggtggg cagggcagga aacccctccc acctttacac 1560
   atgcccaggc agcacctcag gccctttgtg gggcagggaa gctgaggcag taagcgggca 1620
   ggcagagctg gaggcctttc aggccagcca gcactctggc ctcctgccgc cgcattccac 1680
   cccagcccct cacaccactc gggagaggga catcctacgg tcccaaggtc aggagggcag 1740
   ggctggggtt gactcaggcc cctcccagct gtggccacct gggtgttggg agggcagaag 1800
   tgcaggcacc tagggccccc catgtgccca ccctgggagc tctccttgga acccattcct 1860
   gaaattattt aaaggggttg gccgggctcc caccagggcc tgggtgggaa ggtacaggcg 1920
   ttcccccggg gcctagtacc cccgcgtggc ctatccactc ctcacatcca cacactgcac 1980
   ccccactcct ggggcagggc caccagcatc caggcggcca gcaggcacct gagtggctgg 2040
   gacaagggat cccccttccc tgtggttcta ttatattata attataatta aatatgagag 2100
   catgct 2106
<210> 9
   <211> 894
   <212> DNA
   <213> Homo sapiens
<400> 9
   tttgggtttg gacgggtgcg tttgtttttc atagtgcagg tctggagccg gactgagatg      60
   gggagtagag ggaccccttg aaacttcagg gtggatgaat atgaagtttc atcaccagct     120
   gtgatgtttg atattggact gtggttgtgc cagttgacac tcaggagtgg agactagatc     180
   tgcaatctgt tgggactgtg atgaggggat catcctgccc tcgaaccaga cccagccctg     240
   tagcccaggc actattgcct ggagttcctg ggctctcagc gccaggaaat catgaggttc     300
   agtgtctcag gcatgaggac cgactacccc aggagtgtgc tggctcctgc ttatgtgtca     360
   gtctgtctcc tcctcttgtg tccaagggaa gtcatcgctc ccgctggctc agaaccatgg     420
   ctgtgccagc cggcacccag gtgtggagac aagatctaca accccttgga gcagtgctgt     480
   tacaatgacg ccatcgtgtc cctgagcgag acccgccaat gtggtccccc ctgcaccttc     540
   tggccctgct ttgagctctg ctgtcttgat tcctttggcc tcacaaacga ttttgttgtg     600
   aagctgaagg ttcagggtgt gaattcccag tgccactcat ctcccatctc cagtaaatgt     660
   gaaagcagaa gacgttttcc ctgagaagac atagaaagaa aatcaacttt cactaaggca     720
   tctcagaaac ataggctagg gtaatatgtg taccagtaga gaagcctgag gaatttacaa     780
   aatgatgcag ctccaagcca ttgtatggcc catgtgggag actgatggga catggagaat     840
   gacagtagat tatcaggaaa taaataaagt ggtttttcca atgtaaaaaa aaaa           894
<210> 10
   <211> 1095
   <212> DNA
   <213> Homo sapiens
<400> 10
   atgggttcag cccaaggctt ggcatattgt ggatgcttcg tagaagttat tgtgggctta      60
   ctgtcagctg tgcacaaggc accaggagaa caggggctgg aggtaggaga gatgttgcca     120
   gactgtcagt tacttcttcc agttagcttt tcagttgtgt tgcagtatgc ctctggagga     180
   ggggaagtgt ctttgatgct ctggaataca accgggcccg cccccggcgc accggcccgc     240
   gcgggcacct ggcggtcgca gcggacacag caggaggagg ataggccgcg ctgggcgggc     300
   gtcgcggggc cgcgcttggt gttcctgagc cgcggccgaa ctttttccca agaagtccca     360
   cgtttggagg cccgaggagc gggacgggga ggagagactg gagttcctgg aaggagcagc     420
   agcagcagcc ccacaacatg gcctcctccg cctcctcctc tcccgcgggc accgaggact     480
   ccgctcctgc ccagggtggc tttggatcag attattcacg aagctctaga aaggtaagtg     540
   aacttggcat tggccattgc ttattatgct tcctgtctgg ctcttactca tgttgccatt     600
   ttgtggacta aaactcaagg cagttcaaaa atactgatat gcaggtgcca tggatgctgt     660
   tggagtgtgt gtcctaagag agagggtaag tgctggtttt ccattttaaa catagctcgt     720
   gatatcaagt tgccccctcc cttcattccc tgactcccgt cttcctccaa aagcggctct     780
   cttggtttag taagcatctc taccctgcaa agcggaaata atgaaaaatg agattcactt     840
   tgcaagcttg ttgggtgagc ttctggagaa taatttttac caaaagcctg aaatcatttg     900
   gcattgttgg tgttttcaat gcatctgcat cacagcacat tgcatttcac atctggccag     960
   tttggaatga aacttagaaa atcaccagac tatagtgatg taattgtcat gaagtggttt    1020
   ttgtagctta gagaaaagaa aaacacttgg ctgaaagaat ggtctgaagt cccacccaaa    1080
   gtaaaacatt ctaaa                                                     1095
<210> 11
   <211> 846
   <212> DNA
   <213> Homo sapiens
<400> 11
   acctctcaag cagccagcgc ctgcctgaat ctgttctgcc ccctccccac ccatttcacc 60
   accaccatga caccgggcac ccagtctcct ttcttcctgc tgctgctcct cacagtgctt 120
   acagttgtta cgggttctgg tcatgcaagc tctaccccag gtggagaaaa ggagacttcg 180
   gctacccaga gaagttcagt gcccagctct actgagaaga atgcttttaa ttcctctctg 240
   gaagatccca gcaccgacta ctaccaagag ctgcagagag acatttctga aatggctgtc 300
   tgtcagtgcc gccgaaagaa ctacgggcag ctggacatct ttccagcccg ggatacctac 360
   catcctatga gcgagtaccc cacctaccac acccatgggc gctatgtgcc ccctagcagt 420
   accgatcgta gcccctatga gaaggtttct gcaggtaatg gtggcagcag cctctcttac 480
   acaaacccag cagtggcagc cacttctgcc aacttgtagg ggcacgtcgc ccgctgagct 540
   gagtggccag ccagtgccat tccactccac tcaggttctt cagggccaga gcccctgcac 600
   cctgtttggg ctggtgagct gggagttcag gtgggctgct cacagcctcc ttcagaggcc 660
   ccaccaattt ctcggacact tctcagtgtg tggaagctca tgtgggcccc tgagggctca 720
   tgcctgggaa gtgttgtggt gggggctccc aggaggactg gcccagagag ccctgagata 780
   gcggggatcc tgaactggac tgaataaaac gtggtctccc actgcgccaa aaaaaaaaaa 840
   aaaaaa 846
<210> 12
   <211> 730
   <212> DNA
   <213> Homo sapiens
<220>
   <221> misc_feature
   <222> (728)..(728)
   <223> n is a, c, g, t or u
<400> 12
   attttaacag caatgagatg acatgagaat atcttcaatt catcaaggat ctgtaaagaa 60
   ggagaggtac aagatatatg aaacccaaat ctcaaaacaa tgatttagtg aatttcccat 120
   gaactttaaa cagtgattgc ttcaaaattt ccaagagcca tactctccct ccagctgctg 180
   tgtgtgtgtg tgtgtataaa tgcacactat tttaacctaa aatggtgccc tgtggctgcc 240
   attctctaac tcttgcatac ttaaacattt attcttggtc aaattaaaac ctcatgcatt 300
   tccaaagata taaatgcctt gcctggagaa gttagatctt gcaagtctca ggagggccga 360
   gatggtttgt cttatgccta tagctgttta tgtcccacca gtgggtgttt gtttcattag 420
   gtgccgtttc cagccaaatg ttctcattct tcacatcttc aatgttgagt agcaaacaga 480
   agaacatcct tcttagcata atattgcttc actggactga ttgtgaactc aaaatacctc 540
   ttgtttcttg tgaagggttt gccttttgta aacaatataa gatcactttt ggtcaaccac 600
   cctgtctgaa tttatctggg ctgctataat aaagtatcat aaactgggag gcttgtaaat 660
   aaatttattt cttacagttt tggaaagctg ggaaccccaa aatcaaggcc aatttggtgt 720
   ctggtgangg 730
<210> 13
   <211> 1834
   <212> DNA
   <213> Homo sapiens
<400> 13
   actttgacag tcttctcatg ctgcctctgc caccttctct gccagaagat accatttcaa      60
   ctttaacaca gcatgatcga aacatacaac caaacttctc cccgatctgc ggccactgga     120
   ctgcccatca gcatgaaaat ttttatgtat ttacttactg tttttcttat cacccagatg     180
   attgggtcag cactttttgc tgtgtatctt catagaaggt tggacaagat agaagatgaa     240
   aggaatcttc atgaagattt tgtattcatg aaaacgatac agagatgcaa cacaggagaa     300
   agatccttat ccttactgaa ctgtgaggag attaaaagcc agtttgaagg ctttgtgaag     360
   gatataatgt taaacaaaga ggagacgaag aaagaaaaca gctttgaaat gcaaaaaggt     420
   gatcagaatc ctcaaattgc ggcacatgtc ataagtgagg ccagcagtaa aacaacatct     480
   gtgttacagt gggctgaaaa aggatactac accatgagca acaacttggt aaccctggaa     540
   aatgggaaac agctgaccgt taaaagacaa ggactctatt atatctatgc ccaagtcacc     600
   ttctgttcca atcgggaagc ttcgagtcaa gctccattta tagccagcct ctgcctaaag     660
   tcccccggta gattcgagag aatcttactc agagctgcaa atacccacag ttccgccaaa     720
   ccttgcgggc aacaatccat tcacttggga ggagtatttg aattgcaacc aggtgcttcg     780
   gtgtttgtca atgtgactga tccaagccaa gtgagccatg gcactggctt cacgtccttt     840
   ggcttactca aactctgaac agtgtcacct tgcaggctgt ggtggagctg acgctgggag     900
   tcttcataat acagcacagc ggttaagccc accccctgtt aactgcctat ttataaccct     960
   aggatcctcc ttatggagaa ctatttatta tacactccaa ggcatgtaga actgtaataa    1020
   gtgaattaca ggtcacatga aaccaaaacg ggccctgctc cataagagct tatatatctg    1080
   aagcagcaac cccactgatg cagacatcca gagagtccta tgaaaagaca aggccattat    1140
   gcacaggttg aattctgagt aaacagcaga taacttgcca agttcagttt tgtttctttg    1200
   cgtgcagtgt ctttccatgg ataatgcatt tgatttatca gtgaagatgc agaagggaaa    1260
   tggggagcct cagctcacat tcagttatgg ttgactctgg gttcctatgg ccttgttgga    1320
   gggggccagg ctctagaacg tctaacacag tggagaaccg aaaccccccc cccccccccg    1380
   ccaccctctc ggacagttat tcattctctt tcaatctctc tctctccatc tctctctttc    1440
   agtctctctc tctcaacctc tttcttccaa tctctctttc tcaatctctc tgtttccctt    1500
   tgtcagtctc ttccctcccc cagtctctct tctcaatccc cctttctaac acacacacac    1560
   acacacacac acacacacac acacacacac acacacacac agagtcaggc cgttgctagt    1620
   cagttctctt ctttccaccc tgtccctatc tctaccacta tagatgaggg tgaggagtag    1680
   ggagtgcagc cctgagcctg cccactcctc attacgaaat gactgtattt aaaggaaatc    1740
   tattgtatct acctgcagtc tccattgttt ccagagtgaa cttgtaatta tcttgttatt    1800
   tatttttgga ataataaaga cctcttaaca ttaa                                 1834

## Description

The present invention relates to methods for the identification and diagnosis of Rheumatoid arthritis (RA), in particular for the diagnosis of anti-citrullinated peptide antibody (ACPA)-negative RA. Most particularly the invention relates to a gene expression signature comprising 12 biomarkers for use in the prognosis or diagnosis of RA.

Rheumatoid arthritis (RA) is a chronic, disabling autoimmune disease with a predilection for peripheral joints(1). The importance of prompt disease-modifying therapy in improving clinical outcomes is reinforced by international management guidelines(2). However, approximately 40% of patients with new-onset inflammatory arthritis have disease which is unclassifiable at inception, and are said to have an undifferentiated arthritis (UA)(3). Recently, a validated "prediction rule" has been developed for use amongst UA patients, whereby a composite score derived from clinical and serological data predicts risk of progression to RA(4). The scoring system relies heavily on autoantibody and, in particular, anti-citrullinated peptide antibody (ACPA) status, highlighting the specificity of circulating ACPA for RA(5). However, the diagnosis of ACPA-*negative* RA remains challenging in the early arthritis clinic, being frequently delayed despite application of the prediction rule(6).

Technological and computational advances have permitted high-throughput, "discovery-driven" routes to biomarker identification in clinical settings through whole-genome transcription profiling(7). Transcriptome analysis in RA has usually been limited to cross-sectional comparisons with normal controls(8, 9), with exceptions aiming to predict responsiveness to biologic agents in established disease(10). Recent work has demonstrated the potential for peripheral blood mononuclear cells (PBMCs) to yield clinically relevant prognostic "gene signatures" in autoimmune disease(11). The application of a similar, prospective, approach to the discovery of predictive biomarkers in UA should compliment existing diagnostic algorithms, whilst providing new insights into disease pathogenesis(12). However, the use of PBMC for transcriptional analysis may result in data that are biased by relative subset abundance (13). To address this, protocols for the rapid *ex vivo* positive selection of subsets for the purpose of transcription profiling have been validated(14), permitting scrutiny of pathophysiologically relevant cells in isolation.

Although no single cell-type is exclusively implicated in RA, many of the established and emerging genetic associations of the condition implicate the CD4+ T-cell as a key player, and anomalies in peripheral blood CD4+ T-cell phenotype are well-documented(15,16,51). For example, in addition to the long-recognised association of the disease with particular MHC class II alleles that encode a conserved sequence within the peptide binding groove ("shared epitope")(12), recent genome-wide association scans have implicated protein tyrosine phosphatase 22 (involved in T-cell receptor signalling), the IL2-receptor, the co-stimulatory molecules CD28, CTLA-4 and CD40, and the potentially lineage-defining *signal transduction and activator of transcription 4* (STAT4) molecules(17). The inventors have therefore surmised that the peripheral blood (PB) CD4+ T-cell transcriptome might therefore represent a plausible substrate for predictive biomarker discovery in early arthritis.

The following terms are used throughout this document.

A sample is any biological material obtained from an individual.

A polynucleotide is a polymeric form of nucleotides of any length. Nuceotides can be either ribonucleotides or deoxyribonucleotides. The term covers, but is not limited to, single-, double-, or multi-stranded deoxyribonucleic acid (DNA) or ribonucleic acid (RNA), mRNA, genomic DNA, cDNA, DNA-RNA hybrids, or a polymer comprising natural, chemically or biochemically modified, non-natural nucleotide bases. Such polynucleotides may include modifications such as those required to allow attachment to a solid support.

A gene is a polynucleotide sequence that comprises sequences that are expressed in a cell as RNA and control sequences necessary for the production of a transcript or precursor.

A gene expression product can be encoded by a full length coding sequence or by any portion of the coding sequence.

A probe can be a DNA molecule such as a genomic DNA or fragment thereof, an RNA molecule, a cDNA molecule or fragment thereof, a PCR product, a synthetic oligonucleotide, or any combination thereof. Said probe can be a derivative or variant of a nucleic acid molecule, such as, for example, a peptide nucleic acid molecule.

A probe can be specific for a target when it comprises a continuous stretch of nucleotides that are entirely complementary to a target nucleotide sequence (generally an RNA product of said gene, or a cDNA product thereof). However a probe can also be considered to be specific if it comprises a continuous stretch of nucleotides that are partially complementary to a target nucleotide sequence. Partially in this instance can be taken to mean that a maximum of 10% from the nucleotides in a continuous stretch of at least 20 nucleotides differs from the corresponding nucleotide sequence of a RNA product of said gene. The term complementary is well known and refers to a sequence that is related by base-pairing rules to the target sequence. Probes will generally be designed to minimise non-specific hybridization.

Where reference is made to "one or more" or "12 or more" or "X or more" genes, this can be understood to be for the purposes of illustration and are non-limiting (although may illustrate best or preferred options).

Gene-lists 1 - 9 as referenced in in the following description are provided at the end of the description. In lists 2-5, the "Illumina ID" column contains the probe address number on the Illumina WG6 (v3) BeadChip (http://www.illumina.com/support/annotation files.ilmn). Where >1 "differentially expressed" Illumina probes appearing in a given list corresponded to a single gene entity, duplicates were removed. Uncorrected p-values are given in lists 2 - 5. Official gene symbols and RefSeq accession numbers are given for identification purposes. Non-linearised fold-change values are given values >1 indicate genes up-regulated in RA relative to non-RA groups in any given comparison. (Values <1 = down-regulated). For down-regulated values, linearised data may be obtained by rendering the negative reciprocal of the non-linearised value; i.e FC of 0.75 => |FC| of -1.33. Gene lists are ranked according to FC. Any additional list-specific information is provided on the relevant pages.

In order to provide further clarity to the reader, certain sequences are provided in full herein. In particular, the following sequence data is referred to herein;

| Gene | Accession No | Sequence ID |
|---|---|---|
| BCL3 | NM_005178.2 | SEQ ID No 1 |
| SOCS3 | NM_003955.3 | SEQ ID No 2 |
| PIM1 | NM_002648.2 | SEQ ID No 3 |
| SBNO2 | NM_014963.2 | SEQ ID No 4 |
| LDHA | NM_005566.1 | SEQ ID No 5 |
| CMAH | NR_002174.2 | SEQ ID No 6 |
| NOG | NM_005450.2 | SEQ ID No 7 |
| PDCD1 | NM_005018.1 | SEQ ID No 8 |
| IGFL2 | NM_01002915.1 | SEQ ID No 9 |
| LOC731186 | XM_001128760.1 | SEQ ID No 10 |
| MUC1 | NM_001044391.1 | SEQ ID No 11 |
| GPRIN3 | CR743148 | SEQ ID No 12 |
| CD40LG | NM_000074.2 | SEQ ID No 13 |

According to the present invention there is provided a method of diagnosing Rheumatoid arthritis in a patient, the method comprising:
obtaining a sample comprising CD4+ T-cells from the patient; and
determining expression levels of all the genes from the group consisting of
BCL3
SOCS3
PIM1
SBNO2
LDHA
CMAH
NOG
PDCD1
IGFL2
LOC731186
MUC1
GPRIN3; and
comparing said expression levels to reference expression levels, wherein a difference in expression of said genes indicates an increased likelihood that the patient has Rheumatoid arthritis.

Optionally the group further consists of CD40LG.

Generally the reference expression levels are representative of levels found in samples comprising cells from a patient who does not have RA.

It has been found that an increase in expression when compared to the reference expression levels indicates an increased likelihood that the patient has rheumatoid arthritis.

The inventors' work has confirmed the utility of the signature where CD4+ T-cells of >95% purity are used, and preliminary data suggest that there is some overlap where whole blood RNA (from unpurified cells) is used as substrate.

The group may be referred to as a "12 gene signature"

Optionally the group further comprises the gene CD40LG.

This group may be referred to as a "13 gene signature"

It has been shown that a difference in expression when compared to the reference expression levels of all of said genes indicates an increased likelihood that the patient has Rheumatoid arthritis

According to the present invention there is provided an *in vitro* method for typing a sample from an individual, the method comprising:
obtaining a sample of CD4+T cells from the individual; and
determining expression levels of all the genes from the group consisting of
BCL3
SOCS3
PIM1
SBNO2
LDHA
CMAH
NOG
PDCD1
IGFL2
LOC731186
MUC1
GPRIN3; and
typing said sample on the basis of the expression levels determined; wherein said typing provides prognostic information related to the risk that the individual has rheumatoid arthritis (RA).

Optionally the group further comprises the gene CD40LG.

Most preferably expression levels are determined by determining RNA levels.

Methods for determining mRNA levels are well established, some being described herein.

The sample comprises CD4+ T cells.

Preferably the sample is peripheral whole blood.

Preferably the methods include the step of separating CD4+ T cells from peripheral whole blood.

Preferably the methods include extracting RNA from the CD4+ T cells.

Most preferably the method is for diagnosing anti-citrullinated peptide antibody (ACPA)-negative rheumatoid arthritis.

Preferably expression levels of all of the genes in the group are determined and compared to a set of reference expression levels.

Optionally the method further comprises the step of combining the results of the 12 gene signature with the results of known prediction analysis. The 13 signature could be used instead of the 12 gene signature.

Preferably the known prediction analysis is the Leiden prediction rule (Reference: van der Helm-van Mil 2008 Arthritis and Rheumatism

Using a composite of the 12 gene signature (or 13 gene signature)/Leiden prediction test maximises the specificity, precision and sensitivity of the test.

According to another aspect of the present invention there is provided a method of diagnosing rheumatoid arthritis in a patient, the method comprising:
obtaining a blood sample from the patient; and
determining expression/mRNA levels of the 12 genes from the group consisting of BCL3, SOCS3, PIM1, SBNO2, LDHA, CMAH, NOG, PDCD1, IGFL2, LOC 731186, MUC1, GPRIN3,
comparing said expression/mRNA levels to a set of reference expression/mRNA levels, wherein a difference in expression of said genes indicates an increased likelihood that the patient has Rheumatoid arthritis.

According to another aspect of the present disclosure there is provided a method of diagnosing Rheumatoid arthritis in a patient, the method comprising:
obtaining a blood sample from the patient; and
determining levels of Interleukin-6 (IL-6); and
comparing said levels to a set of reference IL-6 levels, wherein an difference in expression of IL-6 indicates an increased likelihood that the patient has Rheumatoid arthritis.

It has been found that an increase in expression of IL-6 indicates an increased likelihood that the patient has Rheumatoid arthritis.

Notably, serum IL-6 is notoriously sensitive to, for example, diurnal variation, and the inventors identified that it is useful to standardise the sampling procedure - all the samples were taken between the hours of 1300 and 1630, and frozen to -80 within 4 hours of blood draw, undergoing no more than 1 freeze-thaw cycle, for example.

Most preferably the method is for diagnosing anti-citrullinated peptide antibody (ACPA)-negative rheumatoid arthritis.

Preferably the results of the IL-6 expression analysis are combined with the results of known prediction analysis.

Further disclosed herein are:
An array comprising (a) a substrate and (b) 12 or more different elements, each element comprising at least one polynucleotide that binds to a specific mRNA transcript, said mRNA transcript being of a gene selected from the group defined in GENE LIST 2.

An array comprising (a) a substrate and (b) one or more different elements, each element comprising at least one polynucleotide that binds to a specific mRNA transcript, said mRNA transcript being of a gene selected from the group comprising
BCL3
SOCS3
PIM1
SBNO2
LDHA
CMAH
NOG
PDCD1
IGFL2
LOC731186
MUC1
GPRIN3

Optionally the group further comprises the gene CD40LG.

An array comprising (a) a substrate and (b) 12 elements, each element comprising at least one polynucleotide that binds to an mRNA transcript, said array comprising a binding element for the mRNA of each of the following group of genes
BCL3
SOCS3
PIM1
SBNO2
LDHA
CMAH
NOG
PDCD1
IGFL2
LOC731186
MUC1
GPRIN3

Optionally the array further comprises an additional element comprising at least one polynucleotide that binds to an mRNA transcript for CD40LG.

Preferably the substrate is a solid substrate,

A kit comprising an array as described above and instructions for its use.

Use of a set of probes comprising polynucleotides specific for 12 or more of the genes listed in GENE LIST 2.

The invention further relates to the use of a set of probes consisting solely of polynucleotides specific for the genes;
BCL3
SOCS3
PIM1
SBNO2
LDHA
CMAH
NOG
PDCD1
IGFL2
LOC731186
MUC1
GPRIN3
for determining the risk of an individual for suffering from rheumatoid arthritis.

Optionally the set of probes further comprises a polynucleotide specific for CD40LG.

Further disclosed herein is:
Use of a set of probes comprising primers specific for one or more of the genes selected from the list;
BCL3
SOCS3
PIM1
SBNO2
LDHA
CMAH
NOG
PDCD1
IGFL2
LOC731186
MUC1
GPRIN3
for determining the risk of an individual suffering from rheumatoid arthritis.

Optionally the set of probes further comprises a primer specific for CD40LG.

Use of a set of probes comprising primers specific for the genes selected from the list;
BCL3
SOCS3
PIM1
SBNO2
LDHA
CMAH
NOG
PDCD1
IGFL2
LOC731186
MUC1
GPRIN3
for determining the risk of an individual suffering from rheumatoid arthritis.

Optionally the set of probes further comprises a primer specific for CD40LG.

Most preferably the use of a set of probes is for determining the risk of an individual suffering from anti-citrullinated peptide antibody (ACPA)-negative rheumatoid arthritis.

According to a further aspect of the present disclosure there is provided an IL-6 receptor blocker for the treatment of RA.

This kind of biomarker would be expected to have utility in stratifying early RA patients into subgroups of therapeutic significance. For example, patients with high baseline IL-6 (and potentially also relatively highly dysregulated STAT3-inducible genes in circulating CD4+ T-cells, as a consequence), could potentially be more effectively be managed using an IL-6 signalling blocker (such as tocilizumab) or a Jak1/3 inhibitor.

Optionally the IL-6 receptor blocker is tocilizumab.

Optionally the IL-6 receptor blocker is a Jak1/3 inhibitor.

In order to provide a better understanding of the present invention further details and examples will be provided below with reference to the following figures and tables;

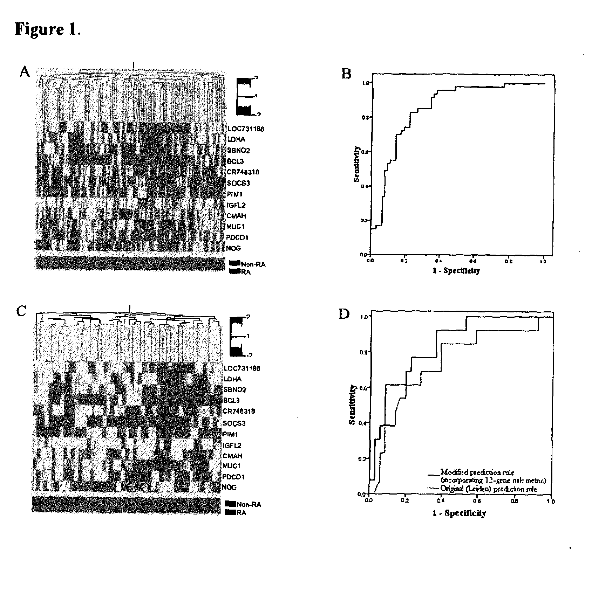

**Figure 1****.** Peripheral blood CD4+ T-cell expression of 12-gene signature is discriminatory for early RA. **A.** Hierarchical clustering of training-set samples based on similarity in gene expression. 111 samples are represented by columns and indicated individual genes by rows; the colour at each co-ordinate indicates gene-wise fold-expression relative to median, according to the colour scale to the right of the figure. Underlying colour-bar labels samples by inception diagnosis, confirmed in each case at >1 year follow-up. **B.** ROC plot from a range of cut-offs for an RA risk metric derived from normalised gene expression values in training cohort (see text). Area under curve = 0.85; Standard error of the mean = 0.04; p<0.001. **C.** Hierarchical clustering of validation UA sample set based on correlations in expression patterns of the same genes (interpretation as for Figure 1A). **D.** ROC curves comparing discriminatory value of original Leiden prediction rule (grey line) with a modified metric incorporating 12-gene signature (see text). The modified metric confers added value to the original Leiden prediction score: AU ROC curve (original Leiden prediction rule) = 0.74; SEM = 0.08, *versus* AU ROC curve (modified metric incorporating gene signature) = 0.84; SEM = 0.06. p<0.001 in both cases.

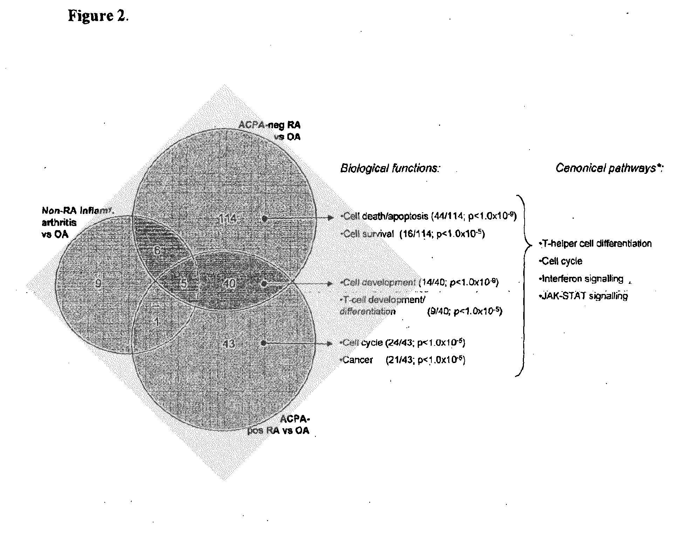

**Figure 2****.** Functional analysis of array data. Non-redundant lists of genes differentially expressed (>1.2 fold-change; p<0.05) between OA and 3 separate inflammatory comparator groups were overlapped in a Venn-diagram (see text, and *Gene-lists 2-4* for detailed list compositions). Genes uniquely de-regulated in RA (ACPA-negative, ACPA-positive or both) could thereby be identified and subjected to pathway analysis using IPA software. The top 2 over-represented *biological functions* identified for the 3 indicated sets are shown, along with the proportion of the set associated with the function in question, and a p-value relating to the likelihood of given proportions occurring by chance (Fisher's exact test). *Gene-lists* 5 - 7 summarise functionally related genes thereby identified. The 3 indicated sets were combined to identify *canonical pathways* over-represented amongst genes differentially expressed between RA and OA in general. Pathways of particular interest in the biological context are listed (genes in question are listed in *Gene-lisf 8*), *hypergeometric p-values (Fisher's exact) in each case <0.01.

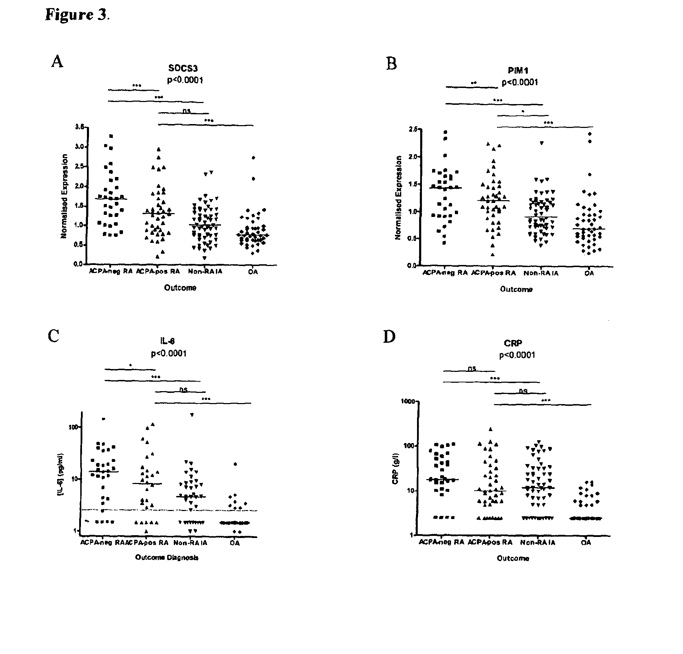

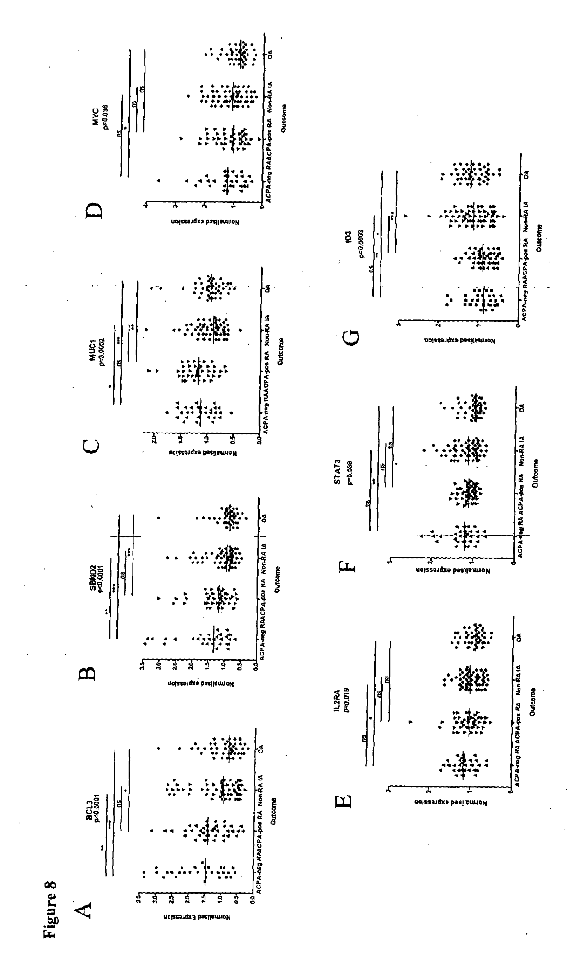

**Figure 3****. A-B.** PB CD4+ T-cell expression profiles of indicated STAT3-regulated genes across 4 comparator groups; see *Figure 8* for additional examples, and *Table 6* for characteristics of comparator groups) **C**. Comparison of serum IL-6 measurements, where available, between comparator groups (n=131). Where ELISA readout was <2.6pg/ml detection threshold (dotted line), an arbitrary value of 1.5pg/ml was recorded. **D**. Comparison of CRP measurements between comparator groups (n=173). Where read-out was <5 an arbitrary value of 2.5 was recorded. **A-F.** P-values shown are derived from non-parametric analysis of variance (Kruskall-Wallis); for *post-hoc* analyses, 1, 2 and 3 asterisks denote p< 0.05, 0.01 and 0.001 respectively (*Dunn's multiple comparison analysis).*

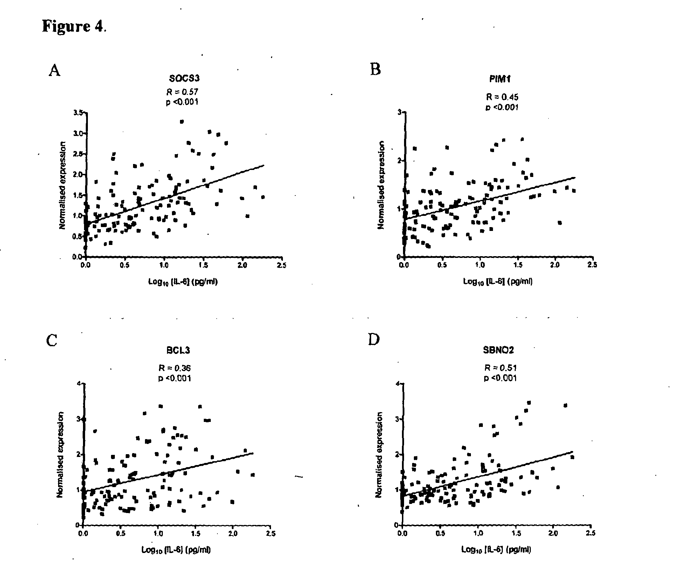

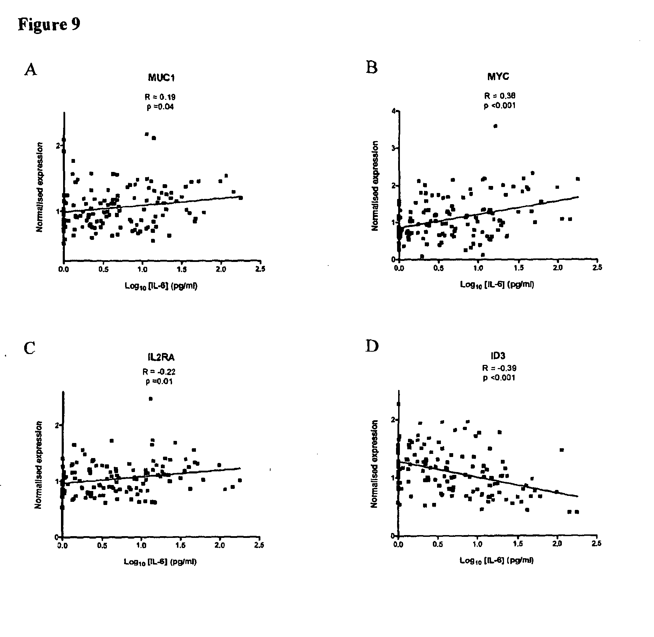

**Figure 4****.** (See *Figure 9* for additional examples). **A-D.** Serum IL-6 concentrations correlate with STAT3-inducible gene expression in PB CD4+ T-cells. Data are shown for 131 individuals in whom paired, contemporaneous samples were available; Pearson's R and associated p-values are shown.

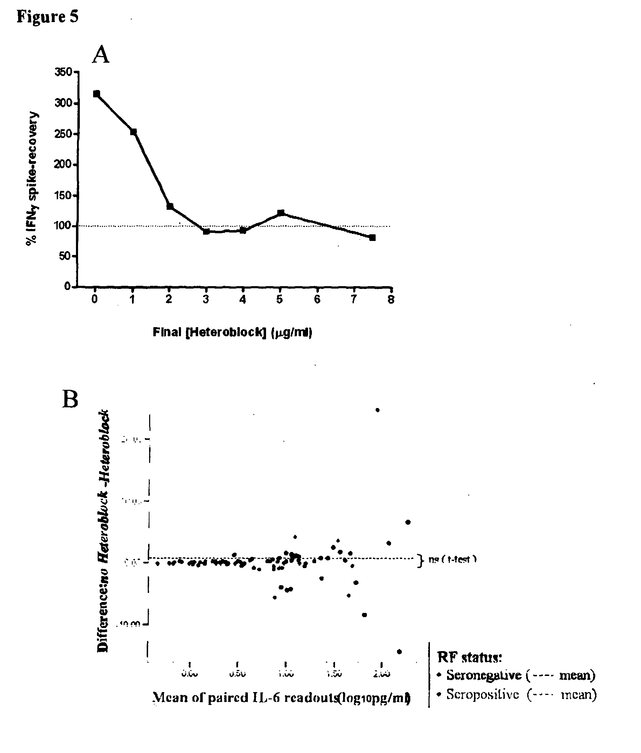

**Figure 5****. A.** Titration of proprietary cocktail of non-human sera (*Heteroblock*; see text) against IFN-γ spike recovery in exemplar RF+ human serum sample. In the absence of Heteroblock the difference in read-out between spiked and un-spiked samples ("spike recovery") is significantly greater than the known spiked IFN-γ amount (>100%), indicating spuriously high assay readout due to the presence of heterophilic RF. Addition of ≥3mg/ml final concentration of Heterblock neutralises this heterophilic effect. **B.** Bland-Altman plot of IL-6 readouts for 24 RF+ and 56 RF- serum samples obtained using MSD electrochemoluminescence platform, comparing assays performed in the presence / absence of a 3□g/ml final [Heteroblock]. No significant discrepancy is seen between RF+ and RF- samples in respect of the mean readout difference of the 2 assays. This indicates that the presence of potentially heterophilic antibodies is unlikely to affect assay readout in this system.

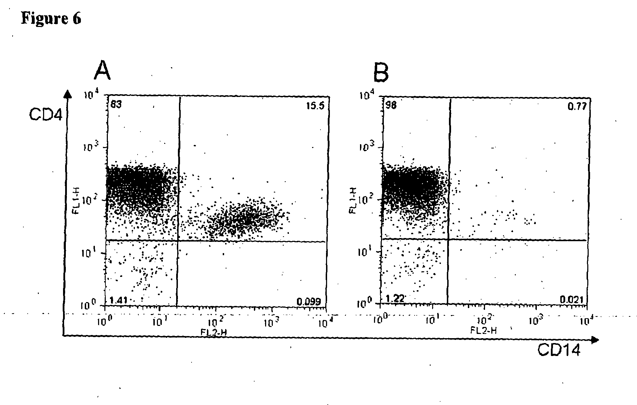

**Figure 6****:** Flow cytometric analysis of CD4+ positive-selection isolate before **(A)** and after **(B)** the monocyte-depletion step described in *Methods.* The extent of CD4+ CD14+ monocyte contamination varies, but may be as high as 15%, as in this example.

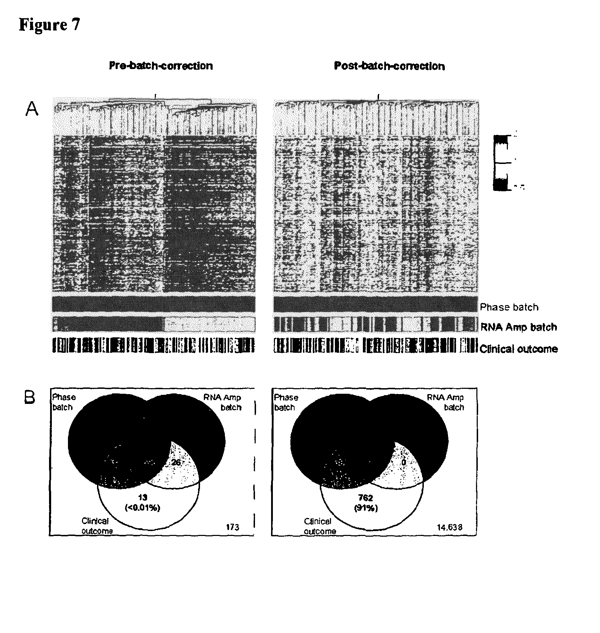

**Figure 7****.** Outputs for normalised expression data of 16,205 genes that passed filtering is shown amongst 173 samples before and after batch-correction using the method of Johnston et al (left and right panels respectively) (reference 23, amin text). **A.** Unsupervised hierarchical clustering of samples based on correlations in gene expression patterns (standard correlation, average linkage, represented by dendrogram). 173 samples are represented by columns and individual genes by rows; the colour at each co-ordinate indicates gene-wise fold-expression relative to median, according to the colour scale to the right of the figure. Underlying blue, red and yellow colour-bars label samples according to membership of phase batch (n=2), RNA amplification batch (n=6) and the clinical outcome category of interest (n=4; ACPA-negative RA, ACPA-positive RA, inflammatory or non-inflammatory controls). Artefactual clustering according to technical parameters (phase of study or within-phase RNA amplification batch) is eliminated through batch-correction, which does not of itself unmask clustering based on the clinical outcome of interest. B. Lists of genes that varied significantly (p<0.05 ANOVA) according to a sample's membership of phase batch (blue), RNA amplification batch (red) or clinical outcome of interest (yellow). Categories were generated amongst 16,205 passed genes, and overlapped in a Venn diagram. Without batch-correction virtually all genes seen to associate with clinical outcome are co-influenced by technical parameters. This potential source of technical bias is eliminated in 91% of outcome-related genes by the process of batch-correction. All genes named and discussed in this manuscript fell within this 91%.
**Figure 8****.** PB CD4+ T-cell expression profiles of indicated genes across 4 comparator groups, continued from *Figure 3*; see *Table 6* for characteristics of comparator groups. P-values shown are derived from non-parametric analysis of variance (Kruskall-Wallis); for *post-hoc* analyses, 1, 2 and 3 asterisks denote p< 0.05, 0.01 and 0.001 repectively (Dunn's multiple comparison analysis).
**Figure 9****.** Serum IL-6 concentrations correlate with STAT3-inducible gene expression in PB CD4+ T cells, continued from *Figure 4**.* Data are shown for 131 individuals in whom paired, contemporaneous samples were available; Pearson's R and associated p-values are shown.

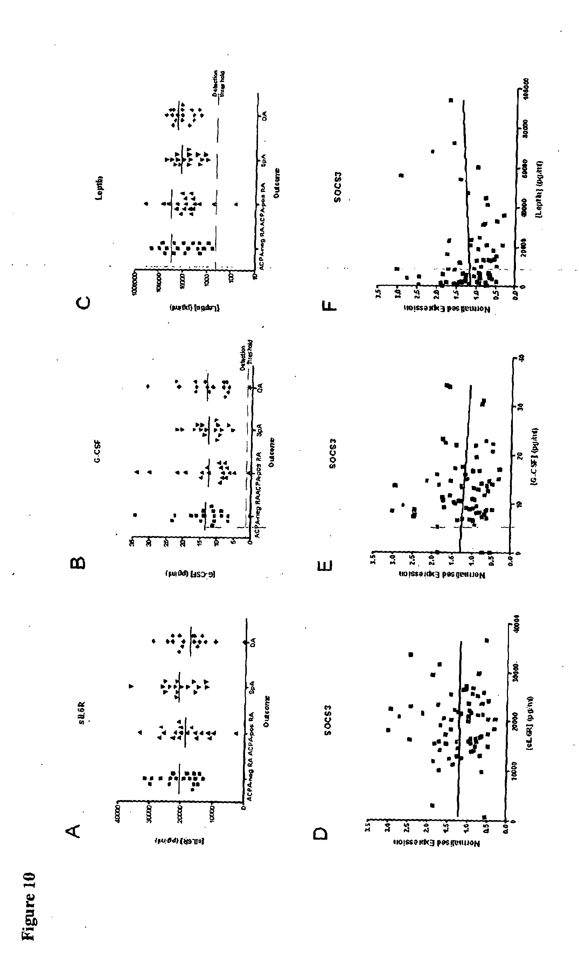

**Figure 10****. A-C.** No relationship between indicated serum analytes and diagnostic outcome amongst 80 early arthritis patients. Kruskall Wallis test; p>0.1 in all cases. D-F. Indicated serum analyte concentrations do not correlate with STAT3 gene expression (exemplar SOCS3 shown). Spearman's rank correlation; p>0.1 in all cases; and

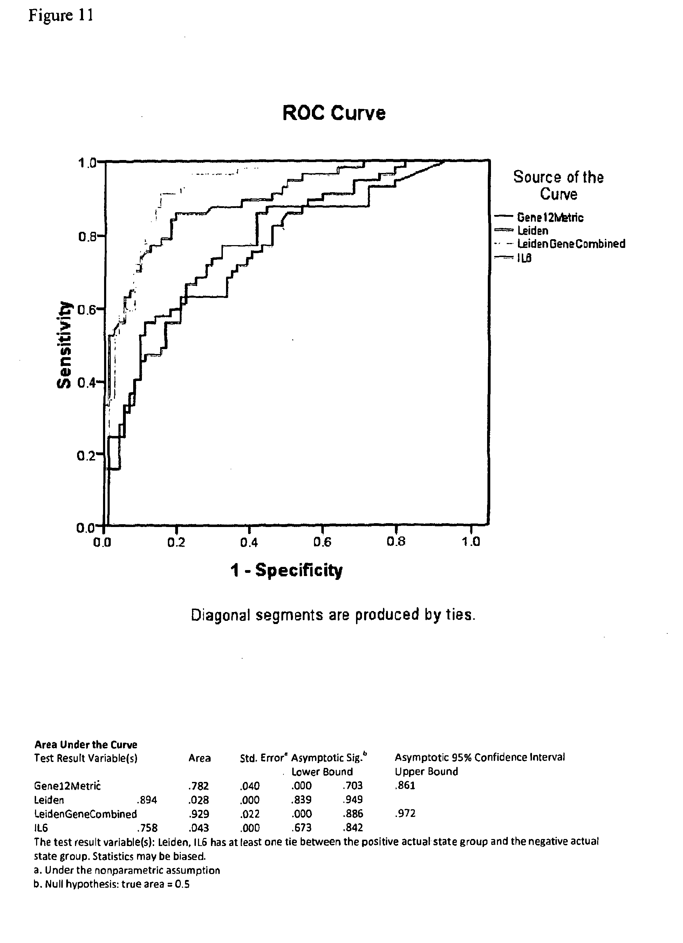

**Figure 11****.** A ROC curve for the whole cohort, including ACPA pos individuals, regardless of whether or not a diagnosis could be assigned at inception. The cohort includes 131 patients (all EA clinic attendees, including those with defined outcomes at inception); both ACPA+ and ACPA-; and

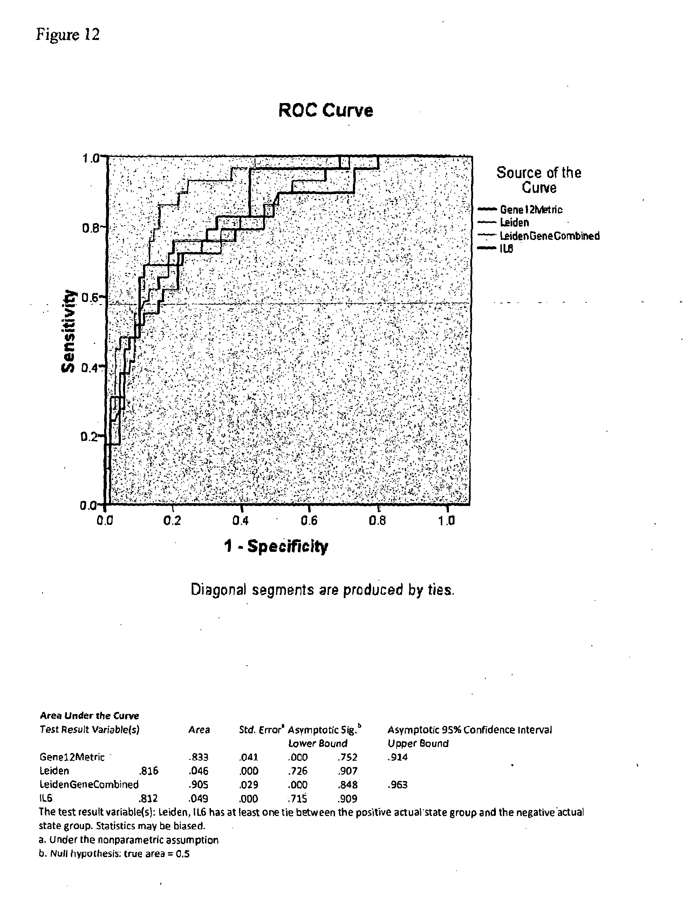

**Figure 12** **-** A ROC curve for ACPA-neg individuals, but also regardless of whether or not a diagnosis could be assigned at inception. 102 patients (all *ACPA-* EA clinic attendees, including those with defined outcomes at inception). Amongst all *ACPA-negative* early arthritis clinic attendees, an [IL-6] of ≥10pg/ml has approx. 0.89 specificity and 0.65 sensitivity for an outcome of RA; and

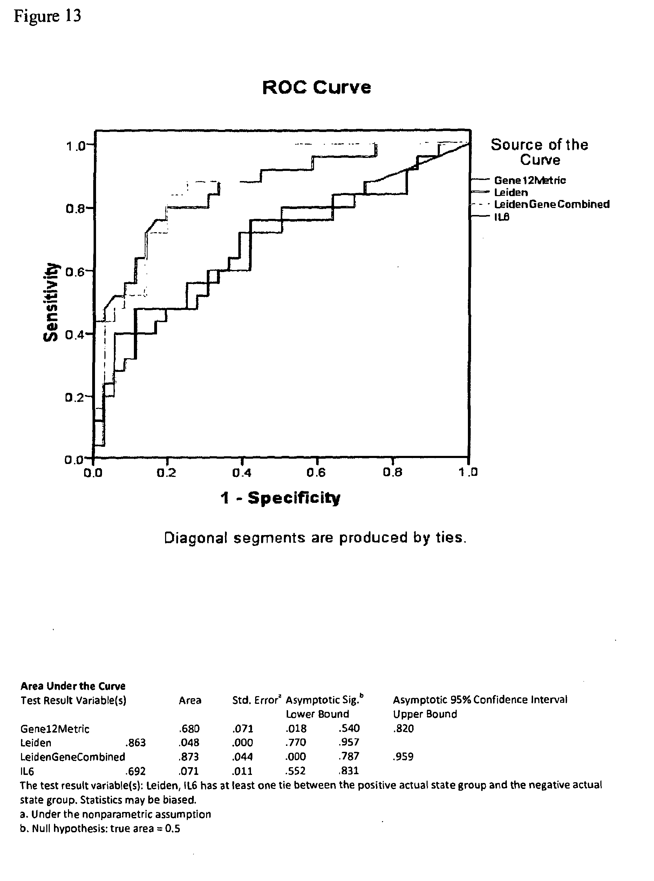

**Figure 13** **-** A ROC curve for UA patients, whether they be ACPA-pos or ACPA-neg. 61 patients (UA patients only; both ACPA+ and ACPA-); and

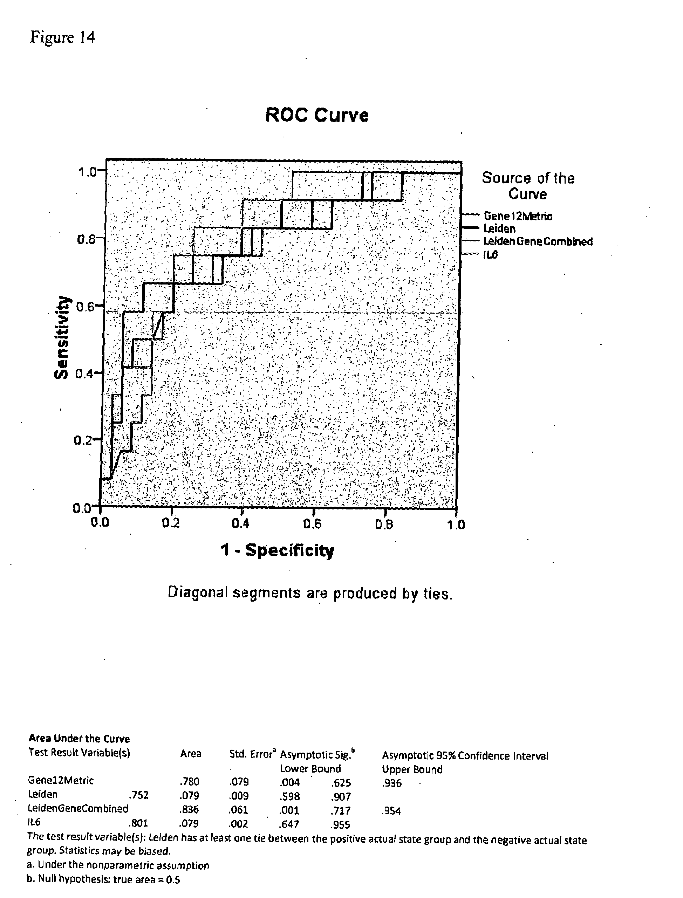

**Figure 14** **-** A ROC curve for UA patients, ACPA-neg only. 48 patients (UA patients, ACPA- only). Amongst all *ACPA-negative UA* patients, an [IL-6] of ≥10pg/ml has approx. 0.92 specificity and 0.58 sensitivity for an outcome of RA.

These examples are not to be considered as limiting.

### PATIENTS AND METHODS

**Patients.** Patients with recent onset arthritis symptoms who were naïve to disease-modifying antirheumatic drugs (DMARDs) and corticosteroids, were recruited from the Freeman Hospital early arthritis clinic (EAC), Newcastle upon Tyne, UK, between September 2006 and December 2008. A detailed clinical assessment of each patient was undertaken, including ascertainment of ACPA staus (anti-CCP2 test, Axis-Shield), along with routine baseline peripheral blood sampling. An initial working diagnosis was assigned to each patient according to a "working diagnosis proforma" (*Table 3*). RA was diagnosed only where 1987 ACR classification criteria(18) were unequivocally fulfilled, and UA was defined as a "suspected inflammatory arthritis where RA remained a possibility, but where established classification criteria for any rheumatological condition remained unmet". This working diagnosis was updated by the consulting rheumatologist at each subsequent clinic visit for the duration of the study - a median of 28 months and greater than 12 months in all cases. The diagnostic outcome of patients with UA at inception was thereby ascertained, with individuals whose arthritis remained undifferentiated at the end of the study being excluded. Patients benefitted from routine clinical care for the duration of the investigation, and all gave written informed consent before inclusion into the study, which was approved by the Local Regional Ethics Committee.

**Table 3. Categorisation of working diagnoses used amongst early arthritis patients at inception and follow-up during the course of this study. Consultant rheumatologists were asked to tick one box at each clinic visit, indicating the best description of their expert opinion of the diagnosis at a given time. See text.**

| | | | |
|---|---|---|---|
| • RA | | | □ |
| • UA | | | □ |
| • Non-RA: | "Inflammatory" | Psoriatic arthritis | □ |
| | | Reactive /self-limiting inflammatory arthritis | □ |
| | | Ankylosing spondylitis | □ |
| | | Enteropathic arthritis | □ |
| | | Undifferentiated spondyloarthritis (not RA) | □ |
| | | CTD | □ |
| | | Crystal | □ |
| | | Other | □ |
| | "Non-inflammatory" | Osteoarthritis | □ |
| | | Noninflammatory arthralgia / other. | □ |

**CD4+ T-cell RNA preparation.** Between 1300hrs and 1630hrs during the patients' EAC appointment, 15ml peripheral whole blood was drawn into EDTA tubes (Greiner Bio-One, Austria) and stored at room temperature for a maximum of 4 hours before processing. Moncytes were first depleted by immunorosetting (Rosettesep^{®} Human Monocyte depletion cocktail, Stemcell Technologies Inc., Vancouver, Canada), and remaining cells underwent positive selection using Easisep^{®} whole blood CD4+ positive selection kit reagents in conjunction with the Robosep^{®} automated cell separator (Stemcell). CD4+ T-cell purity was determined using standard flow cytometry techniques; FITC-conjugated anti-CD4 and PE-conjugated anti-CD14 antibodies were used (Beckton Dickinson, New Jersey, USA). RNA was immediately extracted from CD4+ T-cell isolates using RNeasy MINI kits® (Qiagen GmbH, Germany), incorporating an "on-column" DNA digestion step.

**Microarrays.** Microarray experiments were performed in 2 phases (phase I, 95 samples; phase II, 78 samples). In each case, total RNA quality was assessed using an Agilent 2100 Bioanalyzer (Agilent Technologies, Palo Alto, CA) according to standard protocols(19). 250ng RNA was reverse transcribed into cRNA, and biotin-UTP labeled, using the Illumina TotalPrep RNA Amplification Kit (Ambion, Texas). cRNA was hybridised to the Illumina Whole Genome 6 (version 3) BeadChip® (Illumina, San Diego, CA), following the manufacturer's protocol. Each BeadChip measured the expression of 48,804 genes (annotation file at http://www.illumina.com/support/annotation_files.ilmn) and was imaged using a BeadArray Reader (Illumina).

**Serum cytokine measurement.** During baseline clinical assessment, blood was drawn into serum / gel tubes (Greiner Bio-One, Austria), and serum separated and frozen at -80°C until use. Serum IL-6, sIL6R, TNF-a, leptin and G-CSF concentrations were measured using an immunosorbance assay platform that incorporates a highly sensitive electro-chemoluminescence detection system (Meso Scale Discovery [MSD], Gaithersberg, Maryland) according to the manufacturer's instructions. The potential for heterophilic rheumatoid factors (RFs) in sera to cross-link capture and detection antibodies and contribute to spurious read-outs (20, 21) was excluded during pilot work (*pilot Methods;* *Figure 5*).

**qRT-PCR.** CD4+ T-cell total RNA samples were reverse transcribed using Superscript II^{®} reverse transcriptase and random hexamers according to the manufacturer's instructions (Invitrogen, Carsbad, CA). For replication of microarray findings real-time PCR reactions for reported transcripts were performed as part of a custom-made TaqMan Low Density Array (7900HT real-time PCR system, Applied Biosystems, Foster City, CA). Raw data were normalized and expressed relative to the housekeeping gene beta-actin (BACT) as 2^{-□Ct} values(22). BACT was selected from a panel of 9 potential housekeeping genes, having demonstrated optimal stability for this purpose.

**General bioinformatics and statistical analysis.** Raw microarray data were imported into GeneSpring GX 7.3.1 software (Agilent Technologies), with which all statistical analyses were performed except where indicated. Phases I and II of the study were independently normalised in 2 steps: each probe measurement was first divided by the 50^{th} percentile of all measurements in its array, before being centred around its own median expression measurement across all samples in the phase. The anticipated batch-effect noted between phases on their combination, in addition to minor within-phase batch effects relating to one of the Illumina TotalPrep RNA Amplification steps, was corrected in the R statistical computing environment (http://www.r-project.org/) using the empirical Bayes method of Johnson *et al*(23). Raw and transformed data are available for review purposes at the Gene Expression Omnibus (GEO) address: httg://www.ncbi.nlm.nih.gov/geo/query/acc.cgi?token=bviftkociimgsnk&acc=G SE20098. Genes detectably expressed (detection p-value <0.01 (24)) in ≥1 sample of each study phase passed filtering of the normalised and batch-corrected data, and were included in subsequent analyses (16,205 genes). To define differential expression in this study an arbitrary fold-change cut-off of 1.2 between comparator groups was combined with a significance level cut-off of p<0.05 (Welch's t-test), corrected for multiple testing using the false-discovery-rate (FDR) method of Benjamini *et al*(25). Genes identified in this way were used to train a support vector machine (SVM) classification model (Gaussian kernel) based on known outcomes amongst a "training" sample set(26). The model's accuracy, sensitivity and specificity as a prediction tool was then assessed amongst an independent "validation" sample set. In order to obtain larger lists of differentially expressed genes for biological pathway analysis, significance thresholds were subsequently relaxed through the omission of multiple-test-correction. Ingenuity Pathways Analysis software (Ingenuity Systems, Redwood City, CA) was used for the majority of these analyses. An objectively derived list of STAT3-inducible gene set was created for additional hypergeometric statistical testing by combining lists from two publically available databases (full list given in *Gene List 1; sources;* *http:*//*www.broadinstitute.org*/*gsea*/*msigdb*/*geneset page.jsp?geneSetName*= *V$STAT3_02&keywords=stat3 htt:*//*www.broadinstitute.org*/*gsea*/*msig*d*b*/*geneset page.jsg?geneSetName*= *V$STAT3_01&keywords=stat3 http:*//*rulai.cshl.edu*/*cgi-bin*/*TRED*/*tred.cgi?process=searchTFGene&sel_type=factor_name&factor_or_ ganism=any&tx_search_terms=STAT3&target_organism=human&prom_qualit Y=1&prom_quality=2&prom_quality=3&prom_qualit=4&prom_quality=5&bind quality=0&submit=SEARCH*). Hypergeometric testing in this case was performed using Stat Trek on-line resource (http://stattrek.com). Parametric and non-parametric analyses of variance (ANOVAs), Mann-Whitney U tests, Pearson's correlation coefficients, intraclass correlations, multivariate analyses and the construction of receiver operator characteristic (ROC) curves were performed using SPSS version 15 (SPSS inc., Chicago IL).

**Derivation of risk metrics for ACPA-negative UA.** Leiden prediction scores were calculated for each member of the training cohort according to baseline clinical and laboratory data as described in reference (5). Risk metrics based on the 12-gene RA "signature" were the sum of normalised expression values for the genes therein, assigning negative charge to the value for NOG (which was down-regulated in RA). Within the training dataset, both scores were entered as independent continuous variables into a logistic regression analysis with RA *versus* non-RA outcomes as the dependent variable (*Table 4*). In the resultant model the probability of an outcome of RA is related to both variables via the *modified* metric: B₁x₁+B₂x₂, where *B₁* and *B₂* are the regression coefficients for the Leiden prediction score and 12-gene risk metric respectively (*B* values in *Table 4),* and *x₁* and *x₂* are the values for each amongst individual patients. Hence, for a given patient the modified metric is equal to: (0.98x[Leiden prediction score]) + (0.36x[12-gene risk metric/signature]).

**Table 4. Results of logistic regression analysis for RA versus non-RA diagnoses amongst 111 EA patients in the training cohort. B: regression coefficients; SE(B): standard error for B; OR: odds ratio; CI: confidence interval. The 12-gene risk metric/signature for a given patient is the sum of normalised expression values for 12 genes in the putative RA signature (value for NOG subtracted; see text). The Leiden prediction rule is calculated according to reference (5). Both scores have independent predictive value in discriminating clinical outcomes of interest. Regression coefficients for each are used for the calculation of modified risk metrics amongst the independent cohort of ACPA negative UA patients (see text).**

| **Variable** | | | | | |
|---|---|---|---|---|---|
| | **B** | SE (B) | Wald | **p**-**value** | OR (95% CI) |
| **12 gene risk metric/signature** | **0**.**36** | 0.1 | 11.0 | **0**.**001** | 1.4 (1.2-1.8) |
| **Leiden prediction rule** | **0**.**98** | 0.2 | 21.4 | **<0**.**001** | 2.5 (1.7-3.7) |
| **Constant** | -**10**.**2** | 1.8 | 30.8 | **<0**.**001** | - |

**Pilot Study Methods.** The potential for heterophilic rheumatoid factors (RFs) in sera to cross-link capture and detection antibodies and contribute to spurious read-outs was investigated in pilot work to this study. We first confirmed that a commercially available, proprietary cocktail of non-human sera (Heteroblock, Omega Biologicals Inc., Boseman, Montanna) could successfully neutralise the demonstrable heterophilic activity of native RF in human serum. A known final concentration of recombinant interferon-gamma (IFN-γ) was "spiked" into the sample and, by comparing the calculated difference in standard sandwich ELISA readout (BD Pharmingen, New Jersey, USA) between spiked and un-spiked samples with the actual spiked IFN-γ concentration, the extent of heterophilic activity could be ascertained, and the neutralising effect of varying concetrations of Heteroblock determined *(**Figure* 5A)(reference 21, main text). We next measured IL-6 concentration in 24 RF+ serum samples (median RF by nephelometry = 165 IU) and 56 RF-negative samples, using the MSD platform, in each case running parallel assays with and without an optimised final concentration of Heteroblock. For the RF+ samples, excellent correlation was seen between assays performed with and without heteroblock (intraclass correlation coefficient = 0.98 [95% CI=0.95-0.99]). A Bland-Altman plot confirmed that any such discrepancy that did exist was no less evident in RF-negative samples, suggesting that interference by heterophilic RFs in sera analysed using this platform is inconsequential *(**Figure 5B*). All serum measurements reported in the current study were therefore carried out using the MSD platform in the absence of Heteroblock.

### RESULTS

**Patient groups.** 173 patient samples were retrospectively selected for microarray analysis. 111 of these originated from patients who could be assigned definitive diagnoses at inception, which were confirmed at a median follow-up of 28 months (minimum 1 year); an RA versus non-RA discriminatory "signature" was derived from this "training cohort" alone. The remaining 62 samples, all representing UA patients, formed an independent "validation cohort" for testing the utility of the "signature" according to diagnostic outcomes as they evolved during the same follow-up period. As expected, the characteristics of the UA cohort in respect of age, acute phase response, joint counts etc. fell between the equivalent measurements in the RA and control sample sets within the training cohort *(Table 1)*. For subsequent pathway analysis, all 173 samples were pooled before being divided into four categories based on diagnostic outcome at the end of the study (*Table 5*).

**Table 1. Clinical characteristics of the RA and Control comparator groups used to generate list of differentially-expressed genes, which together comprise a training cohort for machine-learning (total n=111), and the independent UA validation cohort (n=62). Values are mean (1 SD range), median (IQR) or % for normally-distributed, skewed or dichotomous data respectively. ^{A}Statistical tests for significant difference between RA and Non-RA groups; t-test, Mann-Whitney U or Fisher's exact test for normally-distributed, skewed or dichotomous data respectively. Seroneg spond: seronegative sponyloarthropathy; CRP: C-reactive protein; RF: rheumatoid factor; DAS28: disease activity score (incorporating 28- swollen / tender joint counts).**

| | **Training cohort** | | | **Test cohort** |
|---|---|---|---|---|
| | **RA** | **Non-RA** | | **UA** |
| | (n=47) | (n=64) | p*^{A}* | (n=62) |
| **Age** (years; mean, SD range) | **60** (46-74) | **48 (34-62)** | <0.01 | 52 (37-67) |
| **% Female** | **65** | **61** | NS | **77** |
| **% White Caucasian** | **96** | **92** | NS | **90** |
| **Symptom duration** (weeks; median, IQR) | **12** (8-24) | **21** (10.5-52) | 0.026 | **14** (12-26) |
| **Tender joint count** (median, IQR) | **10** (4-15) | **7** (2-14) | 0.246 | **8** (3-16.5) |
| **Swollen joint count** (median, IQR) | **6** (2-10) | **0** (0-2) | <0.001 | **1** (0-3) |
| **Morning stiffness** (hours; median, IQR) | **1** (0.75-2) | **0.75** (0.1-2) | 0.007 | **1** (0.5-2) |
| **ESR** (s; median, IQR) | **56** (30-78) | **24** (14-52) | <0.001 | **30** (18-60) |
| **CRP** (g/l; median, IQR) | **17** (9-62) | **5** (2.5-19) | <0.001 | **8.5** (0-17.) |
| **%ACPA+** | **69** | **0** | - | **21** |
| **%RF+** | **77** | **6** | - | **32** |
| **DAS28** (median, IQR) | **5.37** | n/a | - | |
| **Leiden prediction score** (median, IQR) | n/a | n/a | | **6.4** (5-7.6) |
| **Outcome Diagnosis (%)** | | | | |
| RA | 100 | 0 | - | 40 |
| Seroneg Spond | - | 34 | - | 13 |
| Self-limiting inflam. | - | 19 | - | 15 |
| Other inflam | - | 5 | - | 3 |
| OA/non-inflam. | - | 42 | - | 29 |

**Table 5. Clinical characteristics of subjects as used in pathway analysis of pooled sample-set (n=173), divided into 4 comparator groups by outcome at >1 year follow-up: ACPA-negative RA, ACPA-positive RNA, inflammatory and non-inflammatory control groups. Values are mean (1 SD range), median (IQR) or % for normally-distributed, skewed or dichotomous data respectively. ^{A}statistical tests for significant variance between 3 inflammatory comparator groups (ACPA-negative RA, ACPA-positive RA and non-RA inflammatory arthritis); ANOVA, Krukskall-Wallis or Chi-square test normally-distributed, skewed or dichotomous data respectively. ^{B}statistical tests for significant variance between all 4 inflammatory comparator groups; ANOVA, Kruskall-Wallis or Chi square test for normally-distributed, skewed or dichotomous data respectively.**

| | **ACPA-** | **ACPA-** | **Non-RA** | **Non-RA (OA /** | p*^{A}* | p*^{B}* |
|---|---|---|---|---|---|---|
| | **neg RA** | **pos RA** | **Inflam^{y}.** | **non-intlam^{y}.**) | (3xInflam^{y}.) | (4xgroups) |
| | (n=31) | (n=41) | (n=56) | (n=45) | | |
| **Age** | **61** | **56** | **44** | **52** | | |
| (years; mean, SD) | (46-77) | (44-70) | (30-60) | (40-64) | <0001 | <0.0001 |
| **% Female** | **66** | **61** | **62** | **80** | NS | NS |
| **Symptom durn.** | **12** | **12** | **12** | **32** | | |
| (wk; median, IQR) | (10-20) | (9-22) | (8-25) | (20-89) | NS | <0.0001 |
| **Tender joint count** | **10.5** | **10** | **5** | **9** | | |
| (median, IQR) | (5-15.5) | (3.5-16.5) | (2-13) | (2.5-19) | NS | NS |
| **Swollen joint count** | **4** | **3** | **1** | **0** | | |
| (median, IQR) | (1-4) | (0.5-7.5) | (0-4) | (0-0.5) | <0.001 | <0.0001 |
| **Morning stiffness** | **1** | **1** | **1** | **0.5** | | |
| (hrs; median, IQR) | (1-3.6) | (0.6-2.5) | (0.25-2) | (0.2-1.6) | NS | 0.005 |
| **ESR** | **48** | **54** | **34** | **20** | | |
| (s; median, IQR) | (27-68) | (27-73) | (20-72) | (9.5-30) | NS | <0.0001 |
| **CRP** | **18** | **10** | **13** | **2.5** | | |
| (g/l; median, IQR) | (10-57) | (5-35) | (5-23) | (2.5-6) | NS | <0.0001 |
| **%ACPA+** | **0** | **100** | **0** | **0** | - | - |
| %**RF**+ | **25** | **93** | **11** | **12** | - | - |
| **DAS28** | 4.9 | 5.2 | | | | |
| (median, IQR) | (4.5-5.9) | (4.1-6.0) | - | - | - | - |

**CD4+ T-cell purity and quality control.** Flow cytometric analysis was completed for 148/173 (86%) of samples, and a median CD4+ CD14- purity of 98.9% was achieved (range 95 - 99.7%), with minimal CD4+ CD14+ monocyte contamination (median 0.32%; range 0.01 - 2.98%). Pilot work had demonstrated that incorporation of the monocyte depletion step described was required to achieve this (*Figure 6A* *and B*). RNA integrity numbers (RINs) for all 173 samples were calculated based on Agilent 2100 Bioanalyser(19), and all were of adequate quality for inclusion into microarray experiments (median RIN number 9.5). After normalisation of the raw data and filtering of expressed genes, technical bias relating to processing batches was successfully eliminated using the method of Johnson *et al* (*Figure 7*)(23).

**RA transcription "signature" most accurate in ACPA-negative UA.** Using a significance threshold robust to multiple test correction (false discovery rate p<0.05)(25), 12 non-redundant genes were shown to be differentially expressed (>1.2-fold) in PB CD4+ T-cells between 47 "training cohort" EAC patients with a confirmed diagnosis of RA, and 64 who could be assigned non-RA diagnoses *(Table 2).* An extended list, obtainable by omitting multiple-test correction, is given in *Gene-List 2.* Supervised hierarchical cluster analysis of the resultant multidimensional dataset (111 samples, 12 genes), demonstrated a clear tendency for EAC patients diagnosed with RA to cluster together based on this transcription profile *(**Figure 1A*). Quantitative real-time PCR (qRT-PCR) was used to analyse expression of seven of the differentially expressed genes in a subset of 73 samples. Despite the reduced power to detect change in this smaller dataset, robust differential expression was confirmed for six of the seven genes *(Table 2).*

**Table 2. Fold-change and significance level for genes differentially expressed at inception amongst PB CD4+ T-cells between EAC patients with inception diagnoses of RA and Non-RA (confirmed at >1 year; median 28 months follow-up). The official gene symbol and RefSeq accession number are given as identifiers, in boldface for 12 genes included in statistically most robust "RA signature", and in regular text for additional STAT3-regulated genes referred to in text. ND: not done. |FC|: linearised fold-change expression in RA relative to Non-RA (i.e. negative values represent genes down-regulated in RA relative to non-RA by n-fold). ^{A}Calculations based on normalised expression values of array data; Welch's t-test, raw and multiple-test-corrected p-values given (see methods). ^{B}Calculations based on expression data normalised to the house-keeping gene beta-actin (2^{-□Ct}); Mann-Whitney U test (see methods). ^{C}note that the transcript CR743148 (Illumina Probe ID 6370082) has been retired from NCBI, but the expressed sequence tag corresponds to splice variant(s) within the GPRIN3 gene (chromosome 4.90).**

| | Microarray data | | | qRT-PCR data | |
|---|---|---|---|---|---|
| | (47 RA *vs*. 64 non-RA) | | | (32 RA vs. 41 non-RA) | |
| Gene (Accn. No.) | \|FC\| | Uncorr. p^{A} | Corr. p^{A} | \|FC\| | p^{B} |
| ***12-Gene RA Signature:*** | | | | | |
| **BCL3 (NM_005178)** | 1.59 | 2.6x10⁻⁵ | 0.03 | 2.15 | 0.005 |
| **SOCS3 (NM_003955)** | 1.55 | 3.4x10⁻⁶ | 0.03 | 1.83 | 0.002 |
| **P1M1 (NM_002648)** | 1.52 | 6.8x10⁻⁶ | 0.03 | 1.67 | 0.001 |
| **SBNO2 (NM_014963)** | 1.47 | 1.2x10⁻⁵ | 0.03 | 1.13 | 0.158 |
| **LDHA (NM_005566)** | 1.23 | 3.8x10⁻⁵ | 0.04 | 1.25 | 0.003 |
| **CMAH (NR_002174)** | 1.2 | 1.7x10⁻⁵ | 0.03 | 1.40 | 0.003 |
| **NOG (NM_005450)** | -1.32 | 3.1x10⁻⁵ | 0.03 | -1.59 | 0.004 |
| **PDCD1 (NM_005018)** | 1.42 | 1.0x10⁻⁵ | 0.03 | ND | ND |
| **IGFL2 (NM-001002915)** | 1.31 | 1.1x10⁻⁷ | 0.002 | ND | ND |
| **LOC731186 (XM_001128760)** | 1.28 | 2.3x10⁻⁵ | 0.03 | ND | ND |
| **MUC1 (NM_001044391)** | 1.26 | 2.0x10⁻⁵ | 0.03 | ND | ND |
| **GPRIN3 (CR743148) ^{C}** | 1.32 | 2.1x10⁻⁴ | 0.049 | ND | ND |
| *Additional STAT3-Regulated:* | | | | | |
| ID3 (NM_002167) | -1.3 | 5.2x10⁻⁴ | 0.16 | ND | ND |
| MYC (NM_002467) | 1.2 | 0.04 | 0.75 | 1.29 | 0.01 |

To derive a metric denoting risk of RA progression, the sum of normalised expression values for the 12-gene RA "signature" was calculated for each individual in the training cohort (see methods). A receiver operator characteristic (ROC) curve, plotting sensitivity versus [1-specificity] for a range of cut-offs of this *risk metric,* was then constructed, the area under which (0.85; standard error of mean [SEM]=0.04) suggested a promising discriminatory utility *(**Figure 1B*). When the optimum discriminatory cut-off value for this metric based on the training cohort was applied to classify members of the validation cohort, RA could be predicted amongst UA patients with sensitivity, specificity, positive and negative likelihood ratios (95% CIs) accuracy of 0.64 (0.45-0.80), 0.70 (0.54-0.82), 2.2 (1.2-3.8) and 0.5(0.3-0.9) respectively. An alternative machine-learning methodology, a support vector machine (SVM), was also tested as a classification tool in our cohorts. Use of the SVM prediction model led to a modest improvement in UA classification accuracy over that of the ROC model, with sensitivity, specificity, positive and negative likelihood ratios (95% CIs) accuracy of 0.68 (0.48-0.83), 0.70 (0.60-0.87), 2.2 (1.2-3.8) and 0.4(0.2-0.8) respectively. However, we observed that of 13 ACPA-positive UA patients, 12 progressed to RA, indicating that autoantibody status alone was a much more sensitive predictor of RA in this subset. In contrast, when applied exclusively to the ACPA-*negative* subset of the UA validation cohort (n=49), the SVM classification model provided a sensitivity of 0.85 (0.58-0.96) and a specificity of 0.75 for progression to RA, thereby performing best in this diagnostically most challenging patient group. Hierarchical clustering of the ACPA-negative UA samples based on their 12-gene RA "signature" expression profiles further illustrates molecular similarities within the ACPA-negative RA outcome group *(**Figure 1C*).

The inventors have also found that a third gene could be included to make a 13 gene signature. Effectively, all genes are included as per the original 13 gene signature, but an additional down-regulated gene CD40LG is also included; this provides further specificity to the test giving an area under ROC curve of 0.835.

**Gene signature adds value to existing tools in diagnosing ACPA-negative UA.** Next, we tested the potential additive diagnostic value of our 12-gene signature in comparison to the existing "Leiden *prediction rule"* as a predictor of RA amongst UA patients (4). Whilst the discriminatory utility achieved by the prediction rule in our UA cohort was comparable to that previously reported (n=62; AU ROC curve =0.86; SEM=0.05, data not shown), its performance diminished amongst the ACPA-*negative* sub-cohort (n=49; AU ROC curve = 0.74; SEM=0.08; *Figure 1D**.* Employing a 12-gene risk metric as described above, equivalent discriminatory utility was found in this sub-cohort (AU ROC curve = 0.78; SEM=0.08, data not shown). However, by deriving a *modified risk metric,* which combined all features of the Leiden prediction rule with our 12-gene risk signature (or 13 gene signature) (see *Methods),* and applying it to the independent ACPA-negative UA cohort, we could improve the utility of the prediction rule in this most diagnostically challenging patient group (AU ROC=0.84; SEM=0.06; *Figure 1D*)*.*

**A STAT3 transcription profile is most prominent in ACPA-negative RA.** All 173 patients studied were now grouped into 4 categories based on outcome diagnosis alone: ACPA-positive RA, ACPA-negative RA, inflammatory non-RA controls and osteoarthritis (OA); their demographic and clinical characteristics are presented for comparison in *Table 5.* Three lists of differentially expressed genes could then be generated by comparing each of the "inflammatory" groups (which themselves exhibited comparable acute phase responses) with the OA group (>1.2 fold change; uncorrected p<0.05; *Gene-lists 3-5).* The 3 lists were overlapped on a Venn diagram *(**Figure 2**).*

A highly significant over-representation of genes involved in the cell cycle was identified in association with ACPA-positive RA (24/46; p<1.0x10⁻⁵); *Figure 2**; Gene-list 6).* In addition, genes involved in the regulation of apoptosis were particularly over-represented in ACPA-negative RA patients, and RA was in general characterised by genes with functional roles in T-cell maturation *(**Figure 2**; gene-lists 6-9)*. Interestingly, within the highly significant 12-gene RA "signature" several genes (PIM1, SOCS3, SBNO2, BCL3 and MUC1) were noted to be STAT3-inducible based on literature sources (27-32). The majority of these were most markedly differentially expressed in ACPA-negative RA when compared to ACPA-positive RA *(**Figures 3A**-B* and *Figures 8A**-C)*. Additional STAT3-inducible genes (MYC, IL2RA; (27, 33, 34)) exhibited similar expression patterns, and there was a trend for STAT3 itself to be up-regulated in ACPA-negative RA compared to ACPA-positive RA *(**Figures 8D-F*). Moreover, a reciprocal pattern of expression across outcome groups was observed for the dominant negative helix-loop-helix protein-encoding gene *inhibitor of DNA-binding* 3 (ID3) (*Figure 7G*), consistent with its putative regulatory role in STAT3 signalling(35). MYC and ID3, although not included in the discriminatory RA signature under the stringent significance thresholds used, were nonetheless also seen to exhibit robust differential expression between RA and non-RA patients within the training cohort alone (*Table 2).* Finally, in relation to both the 12-gene signature, and the extended list of genes *exclusively* deregulated in ACPA-*negative RA (Gene list 7),* overlap with independently predicted STAT3-inducible gene sets *(methods* and *Gene List 1*) confirmed a preponderance of STAT3-inducible genes (hypergeometric p-values <0.005 in both cases) - which was not seen for genes deregulated only in *ACPA-positive* RA (p=0.19).

**Serum IL-6 is highest in ACPA-negative RA, and independently predicts CD4+ STAT3-inducible gene expression.** Since one classical mechanism of STAT3 phosphorylation is via gp130 co-receptor ligation(36), we hypothesised that increased systemic levels of a key gp130 ligand and proinflammatory cytokine, IL-6, may be responsible for the STAT3-mediated transcriptional programme in early RA patients. Baseline serum IL-6 was measured in 131/173 EAC patients, subsequently grouped according to their ultimate diagnosis (ACPA-negative RA, ACPA-positive RA, non-RA inflammatory arthropathy or OA). IL-6 levels were low overall (generally <100pg/ml), but were highest in the ACPA-negative RA group *(**Figure 3C**).* Indeed, unlike the generic marker of systemic inflammation C-reactive protein (CRP), IL-6 had discriminatory value for ACPA-negative RA compared with non-RA inflammatory arthritides (*Figures 3C and 3D*). Furthermore, amongst individuals for whom paired and contemporaneous serum IL-6 and PB CD4+ T-cell RNA samples were obtained, a clear correlation was present between IL-6 and the normalised expression of a range of STAT3-inducible genes *(**Figures 4A**-D;* *Figures 9A-D*); for example, serum IL-6 measurements correlated with normalised SOCS3 expression: Pearson's R=0.57, p<0.001 *(**Figure 4A**).* To exclude the possibility that this observation merely reflected systemic inflammation, multivariate analysis was carried out to measure the relative contribution of three related serum variables on STAT3-inducible gene expression. Hence, of CRP, IL-6 and the alternative pro-inflammatory cytokine tumour necrosis factor alpha (TNF-α, which does not signal via STAT3), *only* IL-6 *independently* predicted PB CD4+ T-cell SOCS3 expression amongst 131 early arthritis patients (β=0.53; p<0.001; *Table 6*). Finally, we found no similar relationship between alternative gp130 ligands measurable in sera (G-CSF and leptin) and PB CD4+ T-cell STAT3-inducible gene expression *(**Figure 10**).*

**Table 6. Results of standard linear regression analysis to identify related serum variables independently associated with STAT-3 inducible gene expression amongst 131 EA clinic patients. The dependent variable was Log₁₀(normalised SOCS3 gene expression). SE (B): standard error for B; CI: confidence interval. All variables underwent prior transformation in order to satisfy normality conditions of standard linear regression. Only serum [IL-6] is independently associated with CD4+ T-cell SOCS3 expression (p<0.001; see text).**

| **Serum Variable** | Unstandardised coefficients: | | **Standardised coefficients :** | **p**-**value** | 95% CI (B) (lower, upper) |
|---|---|---|---|---|---|
| | B | SE (B) | β | | |
| Log₁₀[**IL-6**] | 0.21 | 0.05 | **0.53** | **<0.001** | 0.12, 0.30 |
| Log₁₀[**CRP**] | 0.06 | 0.04 | **0.13** | **0.18** | -0.03, 0.15 |
| Log₁₀[**TNF**α] | -0.09 | 0.09 | **-0.08** | **0.32** | -0.27, 0.09 |
| Constant | -0.12 | 0.05 | - | **0.026** | -0.23, -0.02 |

### DISCUSSION.

We present a unique analysis of the *ex-vivo* PB CD4+ T-cell transcriptome in a well-characterised inception cohort of early arthritis patients. We have minimised confounding by including only patients naive to disease-modifying therapy, focussing on a single PB cell subset, collecting and processing samples expeditiously under standardised conditions, and employing careful quality control. In terms of a potential diagnostic tool, it is pleasing that our 12-gene "RA expression signature" *(Table 2)* performed best amongst the diagnostically challenging ACPA-negative UA patient group. These findings support the involvement of CD4+ T-cells in both ACPA positive and negative disease. The observation that both RA serotypes differed from a non-inflammatory control group to a greater extent than a non-RA inflammatory control group *(**Figure 2*), further supports this concept.

The signature's sensitivity and specificity (0.85 and 0.75) for predicting subsequent RA in seronegative UA patients equate to a positive likelihood ratio (LR+) of 3.4, indicating that a prior probability of 25% for RA progression amongst this cohort (13/49 patients progressed to RA) doubles to 53% for an individual assigned a positive SVM classification (posterior probability; [3.4 x {0.25/0.75}]/[1+{3.4 x (0.25/0.75)}](37)). Moreover, of the 13 ACPA-negative UA patients who progressed to RA in our cohort, 8 fell into an "intermediate" risk category for RA progression according to the validated Leiden prediction score(4), thereby remaining subject to delayed diagnosis. Encouragingly, all but one of these patients were correctly classified based on their 12-gene expression profiles. Our *proof-of-concept* that this approach might add value to existing algorithms in the diagnosis of ACPA-negative UA is further supported by the construction of ROC curves comparing the Leiden prediction rule with a modified risk metric that amalgamates the features of our gene signature with those of the prediction rule *(**Figure 1D*). Further validation of the RA signature in well-defined ACPA-negative UA populations is now a priority.

Our data indicate that PB CD4+ T-cells in early RA are characterised by a predominant up-regulation of biological pathways involved in cell cycle progression (ACPA-positive) and survival (ACPA-negative) *(**Figure* 2 and *Gene-lists 6 -* 7). Pathway analysis also suggested that T-cell development and differentiation were de-regulated in both RA serotypes (Gene*-list 8*)*.* These findings are consistent with previous observations of impaired T-cell homeostasis in RA, characterised by increased turnover, telomere shortening and immunosenescence (38). Intriguingly, such observations may be associated with carriage of HLA-DRB1 *shared epitope* alleles(39), which have themselves since been defined as risk-factors for ACPA positivity (40), consistent with the more marked CD4+ T-cell-cycling programme in seropositive individuals suggested by our study (*Figure 2*)*.*

Given the well-characterised importance of the STAT3 signalling pathway in both oncogenesis and T-cell survival pathways, it was notable that 5 genes from our statistically robust 12-gene RA signature are reportedly induced following STAT3 phosphorylation(27-32). This up-regulation was generally most pronounced in ACPA-negative RA (*Figures 3A-B* and *Figure 8A-C*), potentially explaining why the predictive utility of the 12-gene signature was optimal in this disease subset. Additional STAT3-inducible genes (including IL2RA and MYC(27, 33, 34)), along with STAT3 itself, exhibited similar, albeit less statistically robust, expression patterns across the clinical comparator groups, and a reciprocal pattern was seen for ID3 expression, consistent with a proposed regulatory function of its product with respect to STAT3 signalling (35) *(**Figure 8D**-G;* see also *Gene-list* 7). Our observation that increased serum IL-6 levels amongst early arthritis clinic attendees may predict a diagnosis of RA versus alternative arthritides is consistent with findings of previous biomarker studies(41, 42), but ours is, to our knowledge, the first demonstration of a particular association with ACPA-negative disease *(**Figure 3C**).*

Striking correlations were seen between PB CD4+ T-cell expression of several STAT3-inducible genes and paired, contemporaneous serum IL-6 concentrations *(**Figures 4 A-D;* *Figures 9A-D).* Although IL-6 measurements also correlated with systemic inflammation in general (measured as CRP), as well as serum levels of an alternative, non-STAT3-signalling pro-inflammatory cytokine, TNF-α (data not shown), multivariate analysis confirmed IL-6 to be the sole independent predictor of STAT3 gene expression (*Table 6*)*.* STAT3 phosphorylation and downstream transcription is initiated by ligation of the cell-surface gp130 co-receptor by a range of ligands including IL-6 (43). We measured IL-6 in particular because of its recognised role as a pro-inflammatory cytokine in RA(44). However, given that only 30-50% of PB CD4+ T-cells are thought to express membrane-bound IL6R(45), it was possible that the critical *in vivo* determinant of at least some STAT-3 inducible gene-expression in this setting might be circulating sIL6-R rather than IL-6 levels. Trans-signalling of IL-6 via the soluble form of the receptor is crucial for IL-6 mediated responses in IL-6R-negative T-cells. We therefore measured baseline serum sIL-6R concentrations in a subset of 80 early arthritis patients from the current study, comprising 20 of each diagnostic outcome defined in Table 2. In contrast to IL-6, no relationship with diagnostic outcome was detected, and neither was there a correlation between serum sIL-6R concentration and STAT3-inducible gene expression (*Figures 10A* and D).

The inventors also studied two other gp130 ligands , seeking a potential role for them in STAT3 pathway induction; Granulocyte colony stimulating factor (G-CSF) and leptin have both been implicated in RA pathogenesis(46, 47), but their levels in sera from the same subset of study patients neither correlated with diagnostic outcome nor STAT3 gene expression (Figures *10 B-C, E-F*). Finally, IL-10, which is also known to signal through STAT3(48), was undetectable in the majority of sera (*data not shown*)*.* It therefore seems likely that the findings in relation to a STAT3 inducible gene expression signature as part of an early arthritis biomarker for seronegative RA are largely specific to IL-6 signalling.

The inventors also reviewed ROC curves to look at the discriminatory utility of various scoring systems in their cohort, and subsets thereof. 42 patients were excluded from the analysis for whom no IL-6 measurements were available, however only one of these presented with UA.

Figures 11-14, look at:
Figure 11 - The whole cohort, including ACPA pos individuals, regardless of whether or not a diagnosis could be assigned at inception.
Figure 12 - ACPA-neg individuals, but also regardless of whether or not a diagnosis could be assigned at inception.
Figure 13 - UA patients, whether they be ACPA-pos or ACPA-neg
Figure 14 - UA patients, ACPA-neg only.

In each cohort / sub-cohort, 4 ROC curves are compared: the Leiden prediction rule, the 12-gene risk metric we discussed, the composite Leiden/12-gene metric mentioned in the manuscript, and IL-6 alone.

In slides 12 and 14 (excluding ACPA-positive patients), there are given sensitivities / specificities for an example cut-off of 10pg/ml serum [IL-6].

The results suggest that IL-6 is a useful parameter for predicting outcome in early ACPA-negative disease in particular. The most effective prediction appears to be given by a composite of the Leiden prediction score and the 12-gene metric.

In conclusion, the data provides strong evidence for the induction of an IL-6-mediated STAT3 transcription programme in PB CD4+ T-cells of early RA patients, which is most prominent in ACPA-negative individuals, and which contributes to a gene expression "signature" that may have diagnostic utility. Such a pattern of gene expression amongst CD4+ T-cells at this critical early phase in the natural history of inflammatory arthritis could have a defining role in the switch from potentially self-limiting inflammation to T-cell-perpetuated chronic autoimmunity - a model which may not be limited to the example of RA. In any event, the findings could pave the way for a novel treatment paradigm in early arthritis, whereby drugs targeting the *IL-6-gp130-STAT3* "axis" find a rational niche as first choice biologic agents in the management of ACPA-negative RA. One such agent, already available in the clinic, is the IL-6 receptor blocker tocilizumab, whose efficacy is already established in RA(49); others include janus kinase inhibitors currently undergoing phase III clinical trials for the disease (50). Studies such as ours should ultimately contribute to the realisation of true "personalised medicine" in early inflammatory arthritis, in which complex heterogeneity is stratified into pathophysiologically and therapeutically relevant subsets, with clear benefits in terms of clinical outcome and cost.

### Gene List 1 - STAT3-INDUCIBLE GENES on ILLUMINA ARRAY

| **Symbol (Illumina)** | **RefSeq** | **Symbol** (source database, if different) |
|---|---|---|
| A2M | NM_000014.4 | |
| ACCN1 | NM_001094.4 | |
| ACCN4 | NM_018674.3 | |
| ADM2 | NM_024866.4 | |
| ALKBH6 | NM_198867.1 | MGC14376 |
| AP1S2 | NM_003916.3 | |
| AP2B1 | NM_001282.2 | |
| APBA1 | NM_001163.2 | |
| ARF3 | NM_001659.1 | |
| ARHGAP8 | NM_181335.2 | |
| ARL6IP6 | NM_152522.3 | MGC33864 |
| ARX | NM_139058.1 | |
| ASXL1 | NM_015338.4 | |
| ATG3 | NM_022488.3 | |
| AZIN1 | NM_015878.4 | OAZIN |
| B3GAT3 | NM_012200.2 | |
| BCAM | NM_005581.3 | LU |
| BCL2 | NM_000633.2 | |
| BCL2L1 | NM_138578.1 | |
| BCL7A | NM_001024808.1 | |
| BIRC5 | NM_001168.2 | |
| BMI1 | NM_005180.5 | |
| BMP4 | NM_130851.1 | |
| BNC1 | NM_001717.2 | |
| BTBD1 | NM_001011885.1 | |
| C14orf179 | NM_052873.1 | MGC16028 |
| C16orf85 | NM_001001682.1 | FLJ45530 |
| C17orf91 | NM_001001870.1 | MGC 14376 |
| C5orf41 | NM_153607.1 | LOC153222 |
| CA10 | NM_020178.3 | |
| CALU | NM_001219.2 | |
| CAPZA1 | NM_006135.1 | |
| CCL2 | NM_002982.3 | |
| CCND1 | NM_053056.2 | |
| CCND3 | NM_001760.2 | |
| CD40 | NM_152854.2 | TNFRSF5 |
| CDKN1A | NM_000389.2 | |
| CEBPB | NM_005194.2 | |
| CENTD1 | NM_139182.1 | |
| CHRM1 | NM_000738.2 | |
| CISH | NM_145071.1 | |
| CLDN5 | NM_003277.2 | |
| COL4A3BP | NM_005713.1 | |
| CPA4 | NM_016352.2 | |
| CPLX2 | NM_006650.3 | |
| CRTAC1 | NM_018058.4 | |
| CSRP1 | NM_004078.1 | |
| CTGF | NM_001901.2 | |
| CXorf36 | NM_024689.1 | FLJ14103 |
| CYP19A1 | NM_000103.2 | |
| DDIT3 | NM_004083.4 | |
| DERL2 | NM_016041.3 | F-LANA |
| EGR1 | NM_001964.2 | |
| EGR3 | NM_004430.2 | |
| EHHADH | NM_001966.2 | |
| EIF4E | NM_001968.2 | |
| EIF4G1 | NM_198244.1 | |
| E1F5A | NM_001970.3 | |
| ELMO1 | NM_130442.2 | |
| EPHA7 | NM_004440.2 | |
| EXOC3 | NM_007277.4 | SEC6L1 |
| FAS | NM_152872.1 | TNFRSF6 |
| FASN | NM_004104.4 | |
| FBN2 | NM_001999.3 | |
| FBXL3 | NM_012158.1 | FBXL3A |
| FCGR1A | NM_000566.2 | |
| FLJ33387 | NM_182526.1 | |
| FLRT1 | NM_013280.4 | |
| FOS | NM_005252.2 | |
| FOSB | NM_006732.1 | |
| FOXO4 | NM_005938.2 | MLLT7 |
| FUT8 | NM_178156.1 | |
| GABRB1 | NM_000812.2 | |
| GEN1 | NM_182625.2 | FLJ40869 |
| GPC3 | NM_004484.2 | |
| GPHN | NM_001024218.1 | |
| GRIN2D | NM_000836.2 | |
| HEYL | NM_014571.3 | |
| HMOX1 | NM_002133.1 | |
| HNRPR | NM_005826.2 | |
| HOXB13 | NM_006361.5 | |
| HOXB4 | NM_024015.3 | |
| HOXB9 | NM_024017.3 | |
| HOXC4 | NM_014620.4 | |
| HOXC6 | NM_153693.3 | |
| HS6ST3 | NM_153456.2 | |
| HSP90AA1 | NM_001017963.2 | HSPCA |
| HSP90AB1 | NM_007355.2 | HSPCB |
| ICAM1 | NM_000201.1 | |
| IGF1 | NM_000618.2 | |
| IL10 | NM_000572.2 | |
| IL18BP | NM_005699.2 | |
| IL2RA | NM_000417.1 | |
| IL6 | NM_000600.1 | |
| IL6ST | NM_002184.2 | |
| IRF1 | NM_002198.1 | |
| IRX5 | NM_005853.4 | |
| JAK3 | NM_000215.2 | |
| JUN | NM_002228.3 | |
| KAZALD1 | NM_030929.3 | |
| KCNH3 | NM_012284.1 | |
| KCNN2 | NM_021614.2 | |
| KCNN3 | NM_002249.4 | |
| KCNT2 | NM_198503.2 | SLICK |
| KIAA0146 | NM_001080394.1 | |
| KIAA0913 | NM_015037.2 | |
| KIRREL3 | NM_032531.2 | |
| KPNB1 | NM_002265.4 | |
| LBP | NM_004139.2 | |
| LRP2 | NM_004525.2 | |
| LTA | NM_000595.2 | |
| LTBP1 | NM_000627.2 | |
| MAFF | NM_012323.2 | |
| MAML1 | XM_937023.1 | |
| MATN4 | NM_030592.1 | |
| MBD6 | NM_052897.3 | |
| MCL1 | NM_021960.3 | |
| MEIS2 | NM_172315.1 | |
| MIA2 | NM_054024.3 | |
| MID1IP1 | NM_021242.4 | MIG12 |
| MIS12 | NM_024039.1 | |
| MLL | NM_005933.2 | |
| MNT | NM_020310.2 | |
| MOBKL2C | NM_201403.2 | |
| MTMR14 | NM_001077525.1 | FLJ22405 |
| MUC1 | NM_002456.4 | |
| MUC4 | NM_018406.3 | |
| MYC | NM_002467.3 | |
| MYT1 | NM_004535.2 | |
| NAPB | NM_022080.1 | |
| NAV2 | NM_145117.3 | |
| NCAM1 | NM_001076682.2 | |
| NCOA5 | NM_020967.2 | |
| NDST2 | NM_003635.2 | |
| NELL2 | NM_006159.1 | |
| NFAM1 | NM_145912.5 | |
| NOL3 | NM_003946.3 | |
| NOS2A | NM_000625.3 | |
| NPAS4 | NM_178864.2 | NXF |
| NR1D1 | NM_021724.2 | |
| NR4A1 | NM_002135.3 | |
| OSM | NM_020530.3 | |
| OXTR | NM_000916.3 | |
| PAPD1 | NM_018109.2 | |
| PCBP4 | NM_033009.1 | |
| PGF | NM_002632.4 | |
| PIM1 | NM_002648.2 | |
| PPFIA2 | NM_003625.2 | |
| PRF1 | NM_005041.4 | |
| PROS1 | NM_000313.1 | |
| PTMS | NM_002824.4 | |
| RBPJ | NM_203283.1 | PRBPSUH |
| RBPJL | NM_014276.2 | RBPSUHL |
| REG1A | NM_002909.3 | |
| REM2 | NM_173527.2 | FLJ38964 |
| RGS3 | NM_134427.1 | |
| RIMS1 | NM_014989.3 | |
| RND1 | NM_014470.2 | |
| RNF213 | NM_020914.3 | C17ORF27 |
| RORA | NM_002943.2 | |
| RPUSD4 | NM_032795.1 | FLJ14494 |
| RSPO4 | NM_001040007.1 | R-SPONDIN |
| S100A14 | NM_020672.1 | |
| SCUBE3 | NM_152753.2 | |
| SDC1 | NM_002997.4 | |
| SENP3 | NM_015670.4 | |
| SERPING1 | NM_001032295 | |
| SET | NM_003011.2 | |
| SGMS1 | NM_147156.3 | TMEM23 |
| SHOX2 | NM_006884.2 | |
| SLC35A5 | NM_017945.2 | |
| SLC38A5 | NM_033518.1 | |
| SLCO5A1 | NM_030958.1 | |
| SMG7 | NM_201569.1 | C1ORF16 |
| SOCS1 | NM_003745.1 | |
| SOCS3 | NM_003955.3 | |
| SOS1 | NM_005633.2 | |
| SP6 | NM_199262.2 | |
| SPON1 | NM_006108.2 | |
| SPTBN2 | NM_006946.1 | APG3 |
| ST7L | NM_138729.2 | |
| STRA13 | NM_144998.2 | |
| SV2B | NM_014848.3 | |
| TAOK1 | NM_020791.1 | TAO1 |
| TCF7L2 | NM_030756.2 | |
| TIMP1 | NM_003254.2 | |
| TIMP3 | NM_000362.4 | |
| TJAP1 | NM_080604.1 | TJP4 |
| TLR2 | NM_003264.3 | |
| TM9SF1 | NM_006405.5 | |
| TMEM158 | NM_015444.2 | |
| TMEM180 | NM_024789.3 | C10ORF77 |
| TMEM37 | NM_183240.2 | PR1 |
| TNF | NM_000594.2 | |
| TNFRSF8 | NM_001243.3 | |
| TNFSF18 | NM_005092.2 | |
| TNRC6A | NM_014494.2 | TNRC6 |
| TRAF4 | NM_004295.3 | |
| TRH | NM_007117.1 | |
| TRIB2 | NM_021643.1 | |
| TRIP10 | NM_004240.2 | |
| TSC22D4 | NM_030935.3 | THG-1 |
| UBE4B | NM_006048.2 | |
| UBR1 | NM_174916.1 | |
| UBR5 | NM_015902.4 | DD5 |
| UBTF | NM_14233.2 | |
| UPK2 | NM_006760.2 | |
| VCL | NM_014000.2 | |
| VEGFA | NM_003376.4 | VEGF |
| VEZF1 | NM_007146.2 | ZNF161 |
| VIP | NM_194435.1 | |
| VSNL1 | NM_003385.4 | |
| WDR81 | NM_152348.1 | FLJ33817 |
| WEE1 | NM_003390.2 | |
| WNT4 | NM_030761.3 | |
| YY1 | NM_003403.3 | |
| ZBTB11 | NM_014415.2 | |
| ZBTB17 | NM_003443.1 | ZNF151 |
| ZBTB25 | NM_006977.2 | ZNF46 |
| ZBTB9 | NM_152735.3 | |
| ZC3H18 | NM_144604.2 | LOC124245 |
| ZFP112 | NM_001083335.1 | ZNF228 |
| ZFYVE9 | NM_007324.2 | |
| ZHX2 | NM_014943.3 | |
| ZNF296 | NM_145288.1 | ZNF342 |
| ZNF395 | NM_018660.2 | PBF |

| | | |
|---|---|---|
| Note, for hypergeometric probabilities, total number of "non-redundant" genes used as "population size" = 37,847 | | |

### Gene-List 2: RA vs Non-RA, Training samples (n=111)

| ***Red/Bold/Italicised entries => p<0.05 when corrected for multiple-testing (FDR)** | | | | |
|---|---|---|---|---|
| ****transcript CR743148 (Illumina Probe ID 6370082) has been retired from NCBI, but the EST corresponds to splice variant(s) within the GPRIN3 gene (chromosome 4.90).** | | | | |
| **Illumina ID** | **Symbol** | **RefSeq** | **FC** | **uncorrected p*** |
| 2070168 | CX3CR1 | NM_001337.3 | 1.657 | 0.0308 |
| **6330725** | ***BCL3*** | ***NM_005178.2*** | ***1.59*** | ***2.63E-05*** |
| ***4230102*** | ***SOCS3*** | ***NM_003955.3*** | ***1.55*** | ***3.36E-06*** |
| 430438 | MIAT | NR_003491.1 | 1.531 | 0.000239 |
| ***3130301*** | ***PIM1*** | ***NM*_002648.2** | ***1.515*** | ***6.80E-06*** |
| ***3190609*** | ***SBNO2*** | ***NM_014963.2*** | ***1.465*** | ***1.15E-05*** |
| ***6280170*** | ***PDCD1*** | ***NM_005018.1*** | ***1.423*** | ***1.04E-05*** |
| 6220288 | PRDM1 | NM_001198.2 | 1.415 | 0.000242 |
| 60470 | STX11 | NM_003764.2 | 1.386 | 0.00117 |
| 6620689 | MTHFD2 | NM_001040409.1 | 1.375 | 7.88E-05 |
| 1510553 | DACT1 | NM_016651.5 | 1.37 | 0.00763 |
| 4670193 | PRF1 | NM_005041.4 | 1.351 | 0.0465 |
| 1710070 | ITGAM | NM_000632.3 | 1.339 | 0.0249 |
| 5700753 | CEACAM1 | NM_001024912.1 | 1.334 | 0.000235 |
| 160292 | APOBEC3H | NM_181773.2 | 1.332 | 0.0017 |
| 6220195 | BATF | NM_006399.2 | 1.326 | 0.000953 |
| *6370082* | *GPRIN3*** | *CR743148* | *1.321* | *0.00021* |
| 7200301 | ARID5A | NM_212481.1 | 1.321 | 0.00388 |
| ***4730523*** | ***IGFL2*** | *NM_001002915.1* | ***1.307*** | ***1.12E-07*** |
| 4060358 | ABCA1 | NM_005502.2 | 1.299 | 0.00162 |
| 6550600 | MYC | NM_002467.3 | 1.295 | 0.0425 |
| 6770673 | SOCS2 | NM_003877.3 | 1.287 | 0.011 |
| 670576 | ***LOC731186*** | *XM_001128760.1* | 1.283 | 2.28E-05 |
| 650452 | PLCH2 | NM_014638.2 | 1.275 | 0.00242 |
| 7560731 | SNORA64 | NR_002326.1 | 1.265 | 0.000326 |
| 1430598 | FBXO32 | NM_058229.2 | 1.264 | 0.00127 |
| 2070037 | ICOS | NM_012092.2 | 1.263 | 0.00318 |
| 5490068 | MCOLN2 | NM_153259.2 | 1.26 | 0.0131 |
| 3800647 | UGCG | NM_003358.1 | 1.258 | 0.00242 |
| 4230228 | CDK5RAP3 | NM_176095.1 | 1.257 | 0.0126 |
| ***7650026*** | ***MUC1*** | ***NM***_***001044391***.***1*** | *1.255* | ***1.95E-05*** |
| 2070288 | MT1E | NM_175617.3 | 1.253 | 0.0171 |
| 1410408 | ARID5B | NM_032199.1 | 1.248 | 0.000716 |
| 1510424 | S100P | NM_005980.2 | 1.246 | 0.000653 |
| 3420128 | AP3M2 | NM_006803.2 | 1.246 | 0.00305 |
| 3190148 | DDIT4 | NM_019058.2 | 1.245 | 0.0281 |
| 160494 | AQP9 | NM_020980.2 | 1.243 | 0.0275 |
| 870202 | TNFSF10 | NM_003810.2 | 1.24 | 0.00949 |
| 2320129 | CSDA | NM_003651.3 | 1.235 | 0.0405 |
| 2100215 | MAF | NM_001031804.1 | 1.233 | 0.00222 |
| 6420731 | SLC20A1 | NM_05415.3 | 1.231 | 9.77E-05 |
| 10333 | LOC731682 | XM_001129369.1 | 1.229 | 0.0356 |
| 4540376 | FAM13A1 | NM_001015045.1 | 1.228 | 0.043 |
| 1850554 | NPDC1 | NM_015392.2 | 1.227 | 0.000194 |
| *3190092* | ***LDHA*** | *NM_005566.1* | 1.226 | ***3***.***82E***-***05*** |
| 4260372 | GTSCR1 | XM_496277.2 | 1.225 | 0.049 |
| 6250010 | GPRIN3 | NM_198281.2 | 1.223 | 0.0136 |
| 3840470 | ST6GALNAC1 | NM_018414.2 | 1.222 | 0.0141 |
| 4590446 | MSL3L1 | NM_078628.1 | 1.22 | 0.00221 |
| 5890524 | LINS1 | NM_181740.1 | 1.22 | 0.000796 |
| 7570600 | FLJ33590 | NM_173821.1 | 1.22 | 0.00361 |
| 3940390 | TBXAS1 | NM_001061.2 | 1.218 | 0.0186 |
| 450615 | MT2A | NM_005953.2 | 1.216 | 0.000379 |
| 520278 | FAM100B | NM_182565.2 | 1.214 | 0.00367 |
| 7050326 | CDKN2D | NM_079421.2 | 1.214 | 0.000125 |
| 1400601 | C20orf100 | NM_032883.1 | 1.212 | 0.0142 |
| 5130382 | CLDN5 | NM_003277.2 | 1.212 | 0.0321 |
| 5700735 | PARP9 | NM_031458.1 | 1.211 | 0.00126 |
| 5910364 | TYMS | NM_001071.1 | 1.211 | 0.00678 |
| 5900471 | PTGER2 | NM_000956.2 | 1.21 | 0.0115 |
| 2850291 | GARNL4 | NM_015085.3 | 1.209 | 0.00831 |
| 4180301 | ZNF365 | NM_014951.2 | 1.209 | 0.0192 |
| 4200541 | FAM113B | NM_138371.1 | 1.209 | 0.000487 |
| 5090754 | KIAA0101 | NM_014736.4 | 1.208 | 0.00994 |
| 7100372 | PRPF4B | NM_003913.3 | 1.206 | 0.0112 |
| 5420538 | TP53INP1 | NM_033285.2 | 1.205 | 0.016 |
| 1510364 | GBP5 | NM_052942.2 | 1.204 | 0.0182 |
| 3800168 | SLC2A3 | NM_006931.1 | 1.204 | 0.0331 |
| 3840053 | UGP2 | NM_006759.3 | 1.204 | 0.000214 |
| 4610201 | SNORA10 | NR_002327.1 | 1.203 | 8.50E-05 |
| 6200168 | PIM2 | NM_006875.2 | 1.203 | 0.000135 |
| 2190689 | OSBPL5 | NM_020896.2 | 1.202 | 0.0208 |
| 2760112 | P2RY5 | NM_005767.4 | 1.201 | 0.00774 |
| 4670603 | ELMO2 | NM_133171.2 | 1.201 | 0.0101 |
| 3460008 | TMEM173 | NM_198282.1 | 1.2 | 0.000813 |
| 3780161 | TMEM70 | NM_017866.4 | 1.2 | 0.00548 |
| *5550066* | ***CMAH*** | ***NR***_***002174***.***2*** | 1.2 | ***1.69E-05*** |
| 3170703 | LY9 | NM_001033667.1 | 0.837 | 0.0043 |
| 5810746 | MATN2 | NM_002380.3 | 0.837 | 0.00284 |
| 5080615 | IL16 | NM_172217.2 | 0.835 | 0.0262 |
| 160242 | C13orf15 | NM_014059.2 | 0.834 | 0.00866 |
| 6200019 | KLRB1 | NM_002258.2 | 0.831 | 0.0393 |
| 6280504 | LOC100008589 | NR_003287.1 | 0.83 | 0.0364 |
| 6280243 | DNTT | NM_001017520.1 | 0.829 | 0.000589 |
| 5130692 | DDX17 | NM_030881.3 | 0.821 | 0.0105 |
| 2970730 | MYADM | NM_001020820.1 | 0.819 | 0.0171 |
| 870056 | FAM119B | NM_015433.2 | 0.816 | 0.00801 |
| 1580477 | C11orf74 | NM_138787.2 | 0.815 | 7.68E-05 |
| 2710309 | ELA1 | NM_001971.4 | 0.783 | 0.00177 |
| 50706 | CD40LG | NM_000074.2 | 0.78 | 0.000441 |
| 1470762 | AUTS2 | NM_015570.1 | 0.779 | 0.0224 |
| 7570324 | ID3 | NM_002167.2 | 0.775 | 0.000522 |
| 2320253 | USMG5 | NM_032747.2 | 0.774 | 0.0236 |
| *6770603* | *NOG* | ***NM_005450.2*** | *0.755* | *3.14E-05* |
| 130609 | FCGBP | NM_003890.1 | 0.707 | 0.00124 |
| 5080192 | SERPINE2 | NM_006216.2 | 0.657 | 0.00192 |

### Gene-List 3: ACPA-neg RA vs OA, Pooled dataset (n=173)

| ***Red/Bold/italicised entries => p<0.05 when corrected for multiple-testing (FDR)** | | | | |
|---|---|---|---|---|
| ****transcript CR743148 (Illumina Probe ID 6370082) has been retired from NCBI, but the EST corresponds to splice variant(s) within the GPRIN3 gene (chromosome 4.90).** | | | | |
| **Illumina ID** | **Symbol** | **RefSeq** | **FC** | **uncorrected P*** |
| ***4230102*** | ***SOCS3*** | ***NM_003955.3*** | ***1.916*** | ***9.17E-09*** |
| ***6330725*** | ***BCL3*** | ***NM_005178.2*** | ***1.797*** | ***1.64E-05*** |
| ***3130301*** | ***PIM1*** | ***NM_002648.2*** | ***1.715*** | ***4.06E-06*** |
| 510079 | HLA-DRB4 | NM_021983.4 | 1.701 | 0.0231 |
| ***3190609*** | ***SBNO2*** | ***NM_014963.2*** | 1.618 | ***1***.***06E***-***05*** |
| 1820594 | HBEGF | **NM_001945.1** | 1.607 | 0.00284 |
| 6220195 | ***BATF*** | ***NM_006399.2*** | ***1.594*** | ***1.40E-05*** |
| *6620689* | ***MTHFD2*** | ***NM_001040409.1*** | *1.561* | ***1.42E-05*** |
| 6290270 | MNDA | NM_002432.1 | 1.558 | 0.0499 |
| 670010 | LOC650298 | XM_939387.1 | 1.555 | 0.033 |
| 60470 | STX11 | NM_003764.2 | 1.553 | 0.000765 |
| 6220288 | PRDM1 | NM_001198.2 | 1.531 | 0.000238 |
| 6550600 | MYC | NM_002467.3 | 1.527 | 0.00645 |
| 6590377 | RPS26 | NM_001029.3 | 1.527 | 0.0216 |
| ***3800647*** | *UGCG* | ***NM_003358.1*** | 1.518 | ***1.99E-05*** |
| 4230201 | CDKN1A | NM_000389.2 | 1.505 | 0.0136 |
| 1240152 | CFD | NM_001928.2 | 1.499 | 0.0314 |
| 4670048 | RPS26L | NR_002225.2 | 1.499 | 0.0372 |
| 1990300 | SOCS1 | NM_003745.1 | 1.49 | 0.0155 |
| 6270307 | LOC644934 | XM_930344.2 | 1.476 | 0.0265 |
| 6370082 | *GPRIN3"* | CR743148 | 1.457 | 8.53E-05 |
| 4060358 | ABCA1 | NM_005502.2 | 1.454 | 0.000771 |
| 7200301 | ARID5A | NM_212481.1 | 1.448 | 0.00212 |
| ***3780161*** | ***TMEM70*** | ***NM_017866.4*** | ***1.442*** | ***3.84E-06*** |
| 6770673 | SOCS2 | NM_003877.3 | 1.441 | 0.00609 |
| 2070037 | ICOS | NM_012092.2 | 1.438 | 0.000297 |
| **670576** | ***LOC731186*** | ***XM_001128760.1*** | 1.435 | ***6.02E-06*** |
| 430438 | MIAT | NR_003491.1 | 1.428 | 0.00396 |
| 6560376 | RPS26L1 | NR_002309.1 | 1.423 | 0.0367 |
| 6250010 | GPRIN3 | NM_198281.2 | 1.419 | 0.00082 |
| 1440736 | LDLR | NM_000527.2 | 1.415 | 0.00256 |
| 870202 | TNFSF10 | NM_003810.2 | 1.404 | 0.000859 |
| 3710397 | EFNA1 | NM_004428.2 | 1.402 | 0.000713 |
| 2650192 | C6orf105 | NM_032744.1 | 1.399 | 0.000885 |
| 5870692 | GPR132 | NM_013345.2 | 1.399 | 0.0278 |
| 50672 | GSTM1 | NM_000561.2 | 1.394 | 0.0462 |
| 1510553 | DACT1 | NM_016651.5 | 1.374 | 0.0373 |
| 670255 | GADD45A | NM_001924.2 | 1.373 | 0.0411 |
| 2320129 | CSDA | NM_003651.3 | 1.369 | 0.0161 |
| 3940438 | NCF1 | NM_000265.4 | 1.369 | 0.0486 |
| 6960195 | LOC650646 | XM_942527.2 | 1.369 | 0.0456 |
| 6280458 | BCL6 | NM_001706.2 | 1.363 | 0.00517 |
| 520278 | FAM100B | NM_182565.2 | 1.352 | 0.000191 |
| 160494 | AQP9 | NM_020980.2 | 1.349 | 0.0216 |
| 7400747 | FAM89A | NM_198552.1 | 1.336 | 0.000375 |
| 6860347 | FAM46C | NM_017709.3 | 1.334 | 0.0429 |
| 1430598 | FBXO32 | NM_058229.2 | 1.331 | 0.0017 |
| 2570291 | IFNGR2 | NM_005534.2 | 1.329 | 0.000131 |
| 3800168 | SLC2A3 | NM_006931.1 | 1.329 | 0.00763 |
| 3840470 | ST6GALNAC1 | NM_018414.2 | 1.329 | 0.00551 |
| 4670603 | ELMO2 | NM_133171.2 | 1.322 | 0.00404 |
| 270152 | SLC7A5 | NM_003486.5 | 1.321 | 0.0382 |
| 5700753 | CEACAM1 | NM_001024912.1 | 1.321 | 0.00359 |
| 1050068 | ***F2RL1*** | ***NM_005242.3*** | 1.316 | ***0***.***00015*** |
| 4810520 | TRIB1 | NM_025195.2 | 1.316 | 0.0366 |
| 5670465 | ADM | NM_001124.1 | 1.313 | 0.0187 |
| ***7050326*** | ***CDKN2D*** | ***NM_079421.2*** | 1.313 | ***2.36E-05*** |
| 4730411 | SFXN1 | NM_022754.4 | 1.312 | 0.00143 |
| 5270097 | LOC653853 | XM_936029.1 | 1.309 | 0.00641 |
| 1510424 | S100P | NM_005980.2 | 1.305 | 0.00177 |
| ***3190092*** | ***LDHA*** | ***NM_005566.1*** | *1.302* | ***4.95E-05*** |
| 5890524 | LINS1 | NM_181740.1 | 1.298 | 0.000974 |
| 3420128 | AP3M2 | NM_006803.2 | 1.296 | 0.00528 |
| 1030102 | RGS16 | NM_002928.2 | 1.295 | 0.000337 |
| 3190148 | DDIT4 | NM_019058.2 | 1.291 | 0.0213 |
| 3460008 | TMEM173 | NM_198282.1 | 1.287 | 0.000248 |
| 6280672 | TMEM49 | NM_030938.2 | 1.284 | 0.000283 |
| 5820020 | PRDX3 | NM_006793.2 | 1.282 | 0.0019 |
| 2470348 | NFKBIZ | NM_001005474.1 | 1.281 | 0.00621 |
| 2470358 | IFNGR1 | NM_000416.1 | 1.279 | 0.00205 |
| 7560731 | SNORA64 | NR_002326.1 | 1.278 | 0.00259 |
| 1230201 | CTLA4 | NM_005214.3 | 1.277 | 0.00417 |
| 450348 | GNG10 | NM_001017998.2 | 1.276 | 9.60E-05 |
| 380056 | B3GNT2 | NM_006577.5 | 1.274 | 0.000643 |
| 1990753 | SLA | NM_006748.1 | 1.271 | 0.0131 |
| 2600735 | TLR6 | NM_006068.2 | 1.271 | 0.00765 |
| 3840053 | UGP2 | NM_006759.3 | 1.271 | 0.000201 |
| 2070288 | MT1E | NM_175617.3 | 1.27 | 0.0397 |
| 6270554 | LGALS8 | NM_201545.1 | 1.27 | 0.000789 |
| 2640341 | FKBP5 | NM_004117.2 | 1.269 | 0.00778 |
| 6660630 | TP53INP1 | NM_033285.2 | 1.266 | 0.00444 |
| 4590446 | MSL3L1 | NM_078628.1 | 1.265 | 0.00248 |
| 4610201 | SNORA10 | NR_002327.1 | 1.265 | 0.00016 |
| 4010097 | FBXO5 | NM_012177.2 | 1.264 | 0.000131 |
| 1260086 | ID2 | NM_002166.4 | 1.263 | 0.014 |
| 7320041 | GALNAC4S-6ST | NM_015892.2 | 1.262 | 0.0289 |
| 4670414 | TMEM140 | NM_018295.2 | 1.261 | 0.00276 |
| 3870706 | FURIN | NM_002569.2 | 1.259 | 0.00518 |
| 1410408 | ARID5B | NM_032199.1 | 1.258 | 0.00417 |
| 4200541 | FAM113B | NM_138371.1 | 1.258 | 0.000767 |
| 4590228 | GLRX | NM_002064.1 | 1.258 | 0.00719 |
| 20446 | CEBPB | NM_005194.2 | 1.257 | 0.0108 |
| 1850554 | NPDC1 | NM_015392.2 | 1.257 | 0.00132 |
| 5550343 | PDCL | NM_005388.3 | 1.253 | 0.000603 |
| 840554 | RYBP | NM_012234.4 | 1.251 | 0.00531 |
| 1340075 | BAG3 | NM_004281.3 | 1.251 | 0.000391 |
| 4230554 | REXO2 | NM_015523.2 | 1.251 | 0.00173 |
| 5420564 | NFIL3 | NM_005384.2 | 1.251 | 0.0156 |
| 6220543 | HIF1A | NM_001530.2 | 1.251 | 0.0068 |
| 70167 | LY96 | NM_015364.2 | 1.249 | 0.00105 |
| 4230619 | GCA | NM_012198.2 | 1.249 | 0.0104 |
| 2760112 | P2RY5 | NM_005767.4 | 1.247 | 0.0114 |
| 6420731 | SLC20A1 | NM_005415.3 | 1.247 | 0.000324 |
| ***7150280*** | ***LOC145853*** | ***XM***_***096885***.***9*** | *1.247* | ***1.21E-05*** |
| 3420593 | LMNB1 | NM_005573.2 | 1.245 | 0.00222 |
| 4590349 | ACVR2A | NM_001616.3 | 1.245 | 0.0009 |
| 630167 | SDCBP | NM_001007067.1 | 1.244 | 0.0149 |
| 2750719 | DDX21 | NM_004728.2 | 1.243 | 0.00255 |
| 5130382 | CLDN5 | NM_003277.2 | 1.241 | 0.0235 |
| 1010653 | POLR1C | NM_203290.1 | 1.239 | 0.00639 |
| 10630 | IL21R | NM_181078.1 | 1.237 | 0.00733 |
| 5270167 | GNL3 | NM_206826.1 | 1.237 | 0.00236 |
| 6200168 | PIM2 | NM_006875.2 | 1.237 | 0.000751 |
| 3190112 | SERPINB1 | NM_0306662 | 1.236 | 0.000149 |
| 6280170 | PDCD1 | NM_005018.1 | 1.236 | 0.0285 |
| 670086 | MXD1 | NM_002357.2 | 1.235 | 0.0026 |
| 2230379 | NAMPT | NM_005746.2 | 1.232 | 0.0132 |
| 3890326 | SOD2 | NM_001024465.1 | 1.232 | 0.0147 |
| 4050681 | NDUFV2 | NM_21074.1 | 1.232 | 0.00154 |
| 6370414 | CLECL1 | NM_172004.2 | 1.232 | 0.0459 |
| 2060615 | ACVR1B | NM_020328.2 | 1.23 | 0.000616 |
| 2710709 | FCGR1B | NM_001017986.1 | 1.229 | 0.0434 |
| 5270110 | EIF4A3 | NM_014740.2 | 1.228 | 0.000397 |
| 4920110 | GADD45B | NM_015675.2 | 1.227 | 0.00136 |
| 6380112 | GRAMD4 | NM_015124.2 | 1.227 | 0.000299 |
| 2190452 | PIM3 | XM_938171.2 | 1.225 | 0.00047 |
| 5900274 | EDA | NM_001005611.1 | 1.225 | 0.000448 |
| 2630400 | CSTF2T | NM_015235.2 | 1.224 | 0.00143 |
| 7150176 | MAT2A | NM_005911.4 | 1.224 | 0.00517 |
| 5080021 | BIRC3 | NM_001165.3 | 1.22 | 0.00923 |
| 4260019 | NGRN | NM_016645.2 | 1.218 | 0.0008 |
| 4810615 | SLC25A44 | NM_014655.1 | 1.218 | 0.00342 |
| 5870307 | LOC440359 | XM_496143.2 | 1.218 | 0.0223 |
| 1990630 | TRIB3 | NM_021158.3 | 1.217 | 0.0221 |
| 2600059 | GNPDA1 | NM_005471.3 | 1.215 | 0.0146 |
| 5900471 | PTGER2 | NM_000956.2 | 1.213 | 0.0365 |
| 3930390 | SMAP2 | NM_022733.1 | 1.212 | 0.00839 |
| 3420241 | SLC2A14 | NM_153449.2 | 1.211 | 0.0374 |
| 5360079 | GIMAP5 | NM_018384.3 | 1.211 | 0.00847 |
| 130452 | GP5 | NM_004488.1 | 1.21 | 0.000553 |
| 5810504 | METRNL | NM_001004431.1 | 1.21 | 0.0219 |
| 4120131 | KISS1R | NM_032551.3 | 1.209 | 0.00165 |
| 1030646 | FLJ43692 | NM_001003702.1 | 1.208 | 0.00793 |
| 4480504 | ZNF828 | NM_032436.1 | 1.207 | 0.00585 |
| 270601 | HIAT1 | NM_033055.2 | 1.206 | 0.0116 |
| 990735 | RNF149 | NM_173647.2 | 1.205 | 0.0033 |
| 3170091 | GIMAP7 | NM_153236.3 | 1.203 | 0.00101 |
| 3390612 | TLR8 | NM_016610.2 | 1.203 | 0.0404 |
| 1940524 | STS-1 | NM_032873.3 | 1.202 | 0.00732 |
| 4900575 | PTRH2 | NM_016077.3 | 1.202 | 0.0103 |
| 130021 | IL2RA | NM_000417.1 | 1.2 | 0.00378 |
| 2100484 | STAT3 | NM_139276.2 | 1.2 | 0.00317 |
| 60079 | DNAJB1 | NM_006145.1 | 0.831 | 0.00833 |
| 3170703 | LY9 | NM_001033667.1 | 0.83 | 0.0178 |
| 7610440 | XAF1 | NM_199139.1 | 0.827 | 0.0389 |
| 4200475 | MAST3 | NM_015016.1 | 0.824 | 0.0359 |
| 770161 | C10orf73 | XM_096317.11 | 0.813 | 0.00616 |
| 870056 | FAM119B | NM_015433.2 | 0.811 | 0.0225 |
| 3610300 | CCDC58 | NM_001017928.2 | 0.811 | 0.0149 |
| 5080615 | IL16 | NM_172217.2 | 0.806 | 0.0306 |
| 3990170 | IFI27 | NM_005532.3 | 0.799 | 0.0267 |
| 6770603 | NOG | NM_005450.2 | 0.779 | 0.000954 |
| 7570324 | ID3 | NM_002167.2 | 0.75 | 0.00109 |
| 50706 | CD40LG | NM_000074.2 | 0.739 | 0.000205 |
| 2230538 | LRRN3 | NM_001099660.1 | 0.578 | 0.0308 |

### Gene-List 4: ACPA-pos RA vs OA, Pooled dataset (n-173)

| ***Red/Bold/italicised entries => p<0.05 when corrected for multiple-testing (FDR)** | | | | |
|---|---|---|---|---|
| **Illumina ID** | **Symbol** | **RefSeq** | **FC** | **uncorrected p*** |
| 5080692 | HLA-A29.1 | NM_001080840.1 | 1.961 | 0.0149 |
| 430438 | MIAT | NR_003491.1 | 1.546 | 0.000406 |
| 3130301 | PIM1 | NM_002648.2 | 1.536 | 0.000107 |
| 6220288 | PRDM1 | NM_001198.2 | 1.485 | 0.000136 |
| 4230102 | SOCS3 | NM_003955.3 | 1.452 | 0.000601 |
| 6330725 | BCL3 | NM_005178.2 | 1.434 | 0.00186 |
| 6280170 | PDCD1 | NM_005018.1 | 1.427 | 1.73E-05 |
| 1400601 | C20orf100 | NM_032883.1 | 1.381 | 0.000272 |
| ***4730523*** | ***IGFL2*** | ***NM_001002915.1*** | ***1.381*** | ***1.69E-08*** |
| 6220195 | BATF | NM_006399.2 | 1.372 | 0.00029 |
| 2070037 | ICOS | NM_012092.2 | 1.37 | 7.70E-05 |
| 3190609 | SBNO2 | NM_014963.2 | 1.369 | 0.000182 |
| 5090754 | KIAA0101 | NM_014736.4 | 1.337 | 0.000717 |
| 5910364 | TYMS | NM_001071.1 | 1.334 | 0.00037 |
| 6620689 | MTHFD2 | NM_001040409.1 | 1.332 | 0.000143 |
| 6370082 | | CR743148 | 1.331 | 6.90E-05 |
| 1990300 | SOCS1 | NM_003745.1 | .1.328 | 0.0283 |
| 1230201 | CTLA4 | NM_005214.3 | 1.326 | 0.000122 |
| 60470 | STX11 | NM_003764.2 | 1.311 | 0.00495 |
| 2070520 | CDCA7 | NM_031942.4 | 1.311 | 0.00086 |
| 3800647 | UGCG | NM_003358.1 | 1.308 | 0.000353 |
| 130022 | CDCA5 | NM_080668.2 | 1.305 | 0.000177 |
| 7560731 | SNORA64 | NR_002326.1 | 1.3 | 6.93E-05 |
| 5420538 | TP53INP1 | NM_033285.2 | 1.29 | 0.00336 |
| 6250010 | GPRIN3 | NM_198281.2 | 1.288 | 0.00228 |
| 2570253 | BTN3A2 | NM_007047.3 | 1.285 | 0.017 |
| 4060558 | ASCA1 | NM_005502.2 | 1.262 | 0.00522 |
| 4260368 | UBE2C | NM_181800.1 | 1.278 | 0.00038 |
| 10333 | LOC731682 | XM_001129369.1 | 1.274 | 0.00834 |
| 160292 | APOBEC3H | NM_181773.2 | 1.274 | 0.0101 |
| 4230228 | CDK5RAP3 | NM_176095.1 | 1.273 | 0.0141 |
| 5420095 | MYC | NM_002467.3 | 1.273 | 0.00207 |
| 670576 | *LOC731186* | ***XM_001128760.1*** | ***1***.***272*** | 5.19E-06 |
| 1850554 | NPDC1 | NM_015392.2 | 1.272 | 0.000954 |
| 3990619 | TOP2A | NM_001067.2 | 1.269 | 0.000693 |
| 5360070 | CCNB2 | NM_004701.2 | 1.258 | 0.000841 |
| 3840470 | ST6GALNAC1 | NM_018414.2 | 1.256 | 0.00534 |
| 3780161 | TMEM70 | NM_017866.4 | 1.255 | 0.000225 |
| 520278 | FAM100B | NM_182565.2 | 1.252 | 0.00317 |
| 1430598 | FBXO32 | NM_058229.2 | 1.246 | 0.00115 |
| 5890524 | LINS1 | NM_181740.1 | 1.246 | 0.000377 |
| 3610440 | MAF | NM_005360.3 | 1.245 | 0.00787 |
| 6350189 | MGC4677 | NM_052871.3 | 1.239 | 0.0214 |
| 1500010 | CDC20 | NM_001255.2 | 1.236 | 0.000944 |
| 2320170 | CDC45L | NM_003504.3 | 1.236 | 4.66E-05 |
| 5700753 | CEACAM1 | NM_001024912.1 | 1.236 | 0.0226 |
| 5340246 | CRIP2 | NM_001312.2 | 1.235 | 0.0179 |
| 1410408 | ARID5B | NM_032199.1 | 1.234 | 0.00269 |
| 1690692 | SOCS2 | NM_003877.3 | 1.233 | 0.0309 |
| 2470348 | NFKBIZ | NM_001005474.1 | 1.232 | 0.00421 |
| 4880646 | FKSG30 | NM_001017421.1 | 1.232 | 0.0191 |
| 1450056 | CPA5 | NM_080385.3 | 1.231 | 0.00564 |
| 1500553 | NUSAP1 | NM_018454.5 | 1.23 | 0.00215 |
| 1710019 | ICA1 | NM_004968.2 | 1.23 | 0.0016 |
| 4730411 | SFXN1 | NM_022754.4 | 1.225 | 0.000836 |
| 4810520 | TRIB1 | NM_025195.2 | 1.224 | 0.0324 |
| 4890750 | DDX11 | NM_030653.3 | 1.224 | 0.0376 |
| 2490161 | CLEC2B | NM_005127.2 | 1.222 | 0.0378 |
| 10414 | PTTG1 | NM_004219.2 | 1.219 | 0.00173 |
| 1510364 | GBP5 | NM_052942.2 | 1.218 | 0.0161 |
| 380056 | B3GNT2 | NM_006577.5 | 1.216 | 0.000916 |
| 2940110 | UHRF1 | NM_001048201.1 | 1.216 | 0.00165 |
| 3190092 | LDHA | NM_005566.1 | 1.215 | 9.71E-05 |
| 3420241 | SLC2A14 | NM_153449.2 | 1.21 | 0.00873 |
| 6100408 | NLRC5 | NM_032206.3 | 1.21 | 0.00475 |
| 7570600 | FLJ33590 | NM_173821.1 | 1.21 | 0.012 |
| 7650026 | MUC1 | NM_001044391.1 | 1.21 | 0.0017 |
| 450615 | MT2A | NM_005953.2 | 1.209 | 0.00164 |
| 1450280 | NCAPG | NM_022346.3 | 1.208 | 0.000123 |
| 160097 | MELK | NM_014791.2 | 1.207 | 2.95E-05 |
| 4730196 | TK1 | NM_003258.2 | 1.207 | 0.000283 |
| 5090528 | CYorf15B | NM_032576.2 | 1.207 | 0.0415 |
| 6480053 | ATF4 | NM_001675.2 | 1.206 | 0.0166 |
| 4610189 | HERPUD1 | NM_001010990.1 | 1.205 | 0.0217 |
| 4830056 | ARPC5L | NM_030978.1 | 1.204 | 6.82E-05 |
| 3800168 | SLC2A3 | NM_006931.1 | 1.203 | 0.0313 |
| 5260600 | ZNF655 | NM_001009957.1 | 1.203 | 0.00963 |
| 7200301 | ARID5A | NM_212481.1 | 1.202 | 0.0349 |
| 2600735 | TLR6 | NM_006068.2 | 1.201 | 0.0157 |
| 2760112 | P2RY5 | NM_005767.4 | 1.201 | 0.0133 |
| 2970730 | MYADM | NM_001020820.1 | 0.818 | 0.0113 |
| 3610300 | CCDC58 | NM_001017928.2 | 0.815 | 0.0182 |
| 50706 | CD40LG | NM_000074.2 | 0.81 | 0.00637 |
| 6770603 | NOG | NM_005450.2 | 0.802 | 0.00116 |
| 7570324 | ID3 | NM_002167.2 | 0.757 | 8.06E-05 |
| 130609 | FCGBP | NM_003890.1 | 0.688 | 0.00193 |
| 2230538 | LRRN3 | NM_001099660.1 | 0.679 | 0.0483 |
| 5080192 | SERPINE2 | NM_006216.2 | 0.628 | 0.0069 |
| 7050021 | PRKAR1A | NM_002734.3 | 0.522 | 0.00561 |

### Gene-List 5: Non-RA inflam. vs OA, Pooled dataset (n=173)

| **Illumina ID** | **Symbol** | **RefSeq** | **FC** | **uncorrected p*** |
|---|---|---|---|---|
| 3610743 | SF1 | NM_201997.1 | 1.658 | 0.0427 |
| 6960661 | FAM118A | NM_017911.1 | 1.595 | 0.0349 |
| 1430113 | LOC728505 | XM_001127580.1 | 1.366 | 0.000994 |
| 1690440 | XIST | NR_001564.1 | 1.358 | 0.013 |
| 6560376 | RPS26L1 | NR_002309.1 | 1.319 | 0.045 |
| 4060358 | ABCA1 | NM_005502.2 | 1.307 | 0.00185 |
| 5420095 | MYC | NM_002467.3 | 1.302 | 0.00377 |
| 6960195 | LOC650646 | XM_942527.2 | 1.301 | 0.0432 |
| 3710397 | EFNA1 | NM_004428.2 | 1.251 | 0.0055 |
| 2570253 | BTN3A2 | NM_007047.3 | 1.246 | 0.0272 |
| 1940632 | NCAPG2 | NM_017760.5 | 1.238 | 0.0261 |
| 3800647 | UGCG | NM_003358.1 | 1.233 | 0.00373 |
| 6590377 | RPS26 | NM_001029.3 | 1.226 | 0.0457 |
| 5490408 | CEBPD | NM_005195.3 | 1.222 | 0.0436 |
| 3130296 | AMY2A | NM_000699.2 | 1.221 | 0.0485 |
| 160370 | TPM2 | NM_213674.1 | 1.209 | 0.0474 |
| 3130301 | PIM1 | NM_002648.2 | 1.203 | 0.0173 |
| 1440736 | LDLR | NM_000527.2 | 1.2 | 0.0263 |
| 6250010 | GPRIN3 | NM_198281.2 | 0.833 | 0.0147 |
| 1260086 | ID2 | NM_002166.4 | 0.814 | 0.0158 |
| 5890730 | RPS26L | XR_017804.1 | 0.805 | 0.0405 |

### Gene List 6 - Uniquely deregulated in ACPA-pos RA vs OA

| **Symbol** | **RefSeq** | **FC** |
|---|---|---|
| HLA-A29.1 | NM_001080840.1 | 1.961 |
| C20orf100 | NM_032883.1 | 1.381 |
| IGFL2 | NM_001002915.1 | 1.381 |
| KIAA0101 | NM_014736.4 | 1.337 |
| TYMS | NM_001071.1 | 1.334 |
| CDCA7 | NM_031942.4 | 1.311 |
| CDCA5 | NM_080668.2 | 1.305 |
| UBE2C | NM_181800.1 | 1.278 |
| APOBEC3H | NM_181773.2 | 1.274 |
| LOC731682 | XM_001129369.1 | 1.274 |
| CDK5RAP3 | NM_176095.1 | 1.273 |
| TOP2A | NM_001067.2 | 1.269 |
| CCNB2 | NM_004701.2 | 1.258 |
| MGC4677 | NM_052871.3 | 1.239 |
| CDC20 | NM_001255.2 | 1.236 |
| CDC45L | NM_003504. 3 | 1.236 |
| CRIP2 | NM_001312.2 | 1.235 |
| FKSG30 | NM_001017421.1 | 1.232 |
| CPA5 | NM_080385.3 | 1.231 |
| ICA1 | NM_004968.2 | 1.23 |
| NUSAP1 | NM_018454.5 | 1.23 |
| DDX11 | NM_030653.3 | 1.224 |
| CLEC2B | NM_005127.2 | 1.222 |
| MAF | NM_001031804.1 | 1.219 |
| PTTG1 | NM_004219.2 | 1.219 |
| GBP5 | NM_052942.2 | 1.218 |
| UHRF1 | NM_001048201.1 | 1.216 |
| FLJ33590 | NM_173821.1 | 1.21 |
| MUC1 | NM_001044391.1 | 1.21 |
| NLRC5 | NM_032206.3 | 1.21 |
| MT2A | NM_005953.2 | 1.209 |
| NCAPG | NM_022346.3 | 1.208 |
| CYort15B | NM_032576.2 | 1.207 |
| MELK | NM_014791.2 | 1.207 |
| TK1 | NM_003258.2 | 1.207 |
| ATF4 | NM_001675.2 | 1.206 |
| HERPUD1 | NM_001010990.1 | 1.205 |
| ARPC5L | NM_030978.1 | 1.204 |
| ZNF655 | NM_001009957.1 | 1.203 |
| MYADM | NM_001020820.1 | 0.818 |
| FCGBP | NM_003890.1 | 0.688 |
| SERPINE2 | NM_006216.2 | 0.628 |
| PRKAR1A | NM_002734.3 | 0.522 |

Biological Functions: proportion of genes in a given gene list assigned particular biological function is given, along with p-value...

| **"Cell cycle " subset.** | **"Cancer" subset.** |
|---|---|
| 24/43 (p<10e-6) | 21/43 (p<10e-6) |
| | |
| CCNB2 | CCNB2 |
| CDC20 | CDC20 |
| CDC45L | CDCA5 |
| COCA5 | CDCA7 |
| CDCA7 | FCGBP |
| CDK5RAP3 (includes EG:80279) | KIAA0101 |
| DDX11 | MAF |
| FCGBP | MELK |
| KIAA0101 | MT2A |
| MAF | MUC1 |
| MELK | NCAPG (includes EG:64151) |
| MT2A | PRKAR1A |
| MUC1 | PTTG1 |
| NCAPG (includes EG:64151) | SERPINE2 |
| NUSAP1 | TK1 |
| PRKAR1A | TOP2A |
| PTTG1 | TP53INP1 |
| SERPINE2 | TYMS |
| TK1 | UBE2C |
| TOP2A | UHRF1 |
| TYMS | UHRF1 |
| UBE2C | |
| UHRF1 | |
| ZNF655 | |

### Gene List 7 - Uniquely deregulated in ACPA-neg RA vs OA

| **SYMBOL** | **RefSeq** | **FC** |
|---|---|---|
| HLA-DRB4 | NM_021983.4 | 1.701 |
| HBEGF | NM_001945.1 | 1.607 |
| MNDA | NM_002432.1 | 1.558 |
| LOC650298 | XM_939387.1 | 1.555 |
| CDKN1A | NM_000389.2 | 1.505 |
| CFD | NM_001928.2 | 1.499 |
| LOC644934 | XM_930344.2 | 1.476 |
| TNFSF10 | NM_003810.2 | 1.404 |
| C6orf105 | NM_032744.1 | 1.399 |
| GPR132 | NM_013345.2 | 1.399 |
| GSTM1 | NM_000561.2 | 1.394 |
| DACT1 | NM_016651.5 | 1.374 |
| GADD45A | NM_001924.2 | 1.373 |
| CSDA | NM_003651.3 | 1.369 |
| NCF1 | NM_000265.4 | 1.369 |
| BCL6 | NM_001706.2 | 1.363 |
| RPS26L | XR_017804.1 | 1.362 |
| AQP9 | NM_020980.2 | 1.349 |
| FAM46C | NM_017709.3 | 1.334 |
| IFNGR2 | NM_005534.2 | 1.329 |
| SLC7A5 | NM_003486.5 | 1.321 |
| F2RL1 | NM_005242.3 | 1.316 |
| ADM | NM_001124.1 | 1.313 |
| LOC653853 | XM_936029.1 | 1.309 |
| S100P | NM_005980.2 | 1.305 |
| AP3M2 | NM_006803.2 | 1.296 |
| CDKN2D | NM_001800.3 | 1.296 |
| RGS16 | NM_002928.2 | 1.295 |
| DDIT4 | NM_019058.2 | 1.291 |
| TMEM173 | NM_198282.1 | 1.287 |
| TMEM49 | NM_030938.2 | 1.284 |
| PRDX3 | NM_006793.2 | 1.282 |
| IFNGR1 | NM_000416.1 | 1.279 |
| GNG10 | NM_001017998.2 | 1.276 |
| UGP2 | NM_006759.3 | 1.271 |
| MT1E | NM_175617.3 | 1.27 |
| FKBP5 | NM_004117.2 | 1.269 |
| MSL3L1 | NM_078628.1 | 1.265 |
| SNORA10 | NR_002327.1 | 1.265 |
| FBXO5 | NM_012177.2 | 1.264 |
| GALNAC4S-6ST | NM_015892.2 | 1.262 |
| SLA | NM_001045556.1 | 1.262 |
| TMEM140 | NM_018295.2 | 1.261 |
| FURIN | NM_002569.2 | 1.259 |
| FAM113B | NM_138371.1 | 1.258 |
| GLRX | NM_002064.1 | 1.258 |
| CEBPB | NM_005194.2 | 1.257 |
| PDCL | NM_005388.3 | 1.253 |
| ELM02 | NM_182764.1 | 1.252 |
| BAG3 | NM_004281.3 | 1.251 |
| HIF1A | NM_001530,2 | 1.251 |
| NFIL3 | NM_005384.2 | 1.251 |
| REXO2 | NM_015523.2 | 1.251 |
| RYBP | NM_012234.4 | 1.251 |
| GCA | NM_012198.2 | 1.249 |
| LY96 | NM_015364.2 | 1.249 |
| LOC145853 | XM_096885.9 | 1.247 |
| SLC20A1 | NM_005415.3 | 1.247 |
| ACVR2A | NM_001616.3 | 1.245 |
| LMNB1 | NM_005573.2 | 1.245 |
| SDCBP | NM_001007067.1 | 1.244 |
| DDX21 | NM_004728.2 | 1.243 |
| CLDN5 | NM_003277.2 | 1.241 |
| POLR1C | NM_203290.1 | 1.239 |
| GNL3 | NM_206826.1 | 1.237 |
| IL21R | NM_181078.1 | 1.237 |
| PIM2 | NM_006875.2 | 1.237 |
| SERPINB1 | NM_030666.2 | 1.236 |
| MXD1 | NM_002357.2 | 1.235 |
| CLECL1 | NM_172004.2 | 1.232 |
| NAMPT | NM_005746.2 | 1.232 |
| NDUFV2 | NM_021074.1 | 1.232 |
| ACVR1B | NM_020328.2 | 1.23 |
| FCGR1B | NM_001017986.1 | 1.229 |
| EIF4A3 | NM_014740.2 | 1.228 |
| GADD45B | NM_015675.2 | 1.227 |
| GRAMD4 | NM_015124.2 | 1.227 |
| EDA | NM_001005611.1 | 1.225 |
| PIM3 | XM_938171.2 | 1.225 |
| CSTF2T | NM_015235.2 | 1.224 |
| MAT2A | NM_005911.4 | 1.224 |
| BIRC3 | NM_001165.3 | 1.22 |
| LOC44035 | NM_496143.2 | 1.218 |
| NGRN | NM_016645.2 | 1.218 |
| SLC25A44 | NM_014655.1 | 1.218 |
| TRIB3 | NM_021158.3 | 1.217 |
| GNPDA1 | NM_005471.3 | 1.215 |
| SOD2 | NM_001024466.1 | 1.215 |
| PTGER2 | NM_000956.2 | 1.213 |
| LGALS8 | NM_006499.3 | 1.212 |
| SMAP2 | NM_022733.1 | 1.212 |
| GIMAP5 | NM_018384.3 | 1.211 |
| FAM89A | NM_198552.1 | 1.21 |
| GP5 | NM_004488.1 | 1.21 |
| METRNL | NM_001004431 | 1.21 |
| KISS1R | NM_032551.3 | 1.209 |
| FLJ43692 | NM_001003702.1 | 1.208 |
| ZNF828 | NM_032436.1 | 1.207 |
| HIAT1 | NM_033055.2 | 1.206 |
| RNF149 | NM_173647.2 | 1.205 |
| GIMAP7 | NM_153236.3 | 1.203 |
| TLR8 | NM_016610.2 | 1.203 |
| PTRH2 | NM_016077.3 | 1.202 |
| STS-1 | NM_032873.3 | 1.202 |
| IL2RA | NM_000417.1 | 1.2 |
| STAT3 | NM_139276.2 | 1.2 |
| DNAJB1 | NM_006145.1 | 0.831 |
| LY9 | NM_001033667.1 | 0.83 |
| XAF1 | NM_199139.1 | 0.827 |
| MAST3 | NM_015016.1 | 0.824 |
| C10orf73 | XM_096317.11 | 0.813 |
| FAM119B | NM_015433.2 | 0.811 |
| IL16 | NM_172217.2 | 0.806 |
| IFI27 | NM_005532.3 | 0.799 |

### Gene List 8 Deregulated in (ACPA-neg AND ACPA-pos RA) vs OA

| **Symbol** | **RefSeq** | ACPA-neg RA vs OA | ACPA-pos RA vs OA |
|---|---|---|---|
| SOCS3 | NM_003955.3 | 1.916 | 1.452 |
| BCL3 | NM_005178.2 | 1.797 | 1.434 |
| SBNO2 | NM_014963.2 | 1.618 | 1.369 |
| BATF | NM_006399.2 | 1.594 | 1.372 |
| MTHFD2 | NM_001040409.1 | 1.561 | 1.332 |
| STX11 | NM_003764.2 | 1.553 | 1.311 |
| SOCS1 | NM_003745.1 | 1.49 | 1.328 |
| *GRIN3** | CR743148 | 1.457 | 1.331 |
| ARID5A | NM_212481.1 | 1.448 | 1.202 |
| TMEM70 | NM_017866.4 | 1.442 | 1.255 |
| ICOS | NM_012092.2 | 1.438 | 1.37 |
| LOC731186 | XM_001128760.1 | 1.435 | 1.272 |
| MIAT | NR_003491.1 | 1.428 | 1.546 |
| SOCS2 | NM_003877.3 | 1.403 | 1.233 |
| FAM100B | NM_182565.2 | 1.352 | 1.252 |
| FBXO32 | NM_058229.2 | 1.331 | 1.246 |
| SLC2A3 | NM_006931.1 | 1.329 | 1.203 |
| ST6GALNAC1 | NM_018414.2 | 1.329 | 1.256 |
| PRDM1 | NM_182907.1 | 1.328 | 1.335 |
| CEACAM1 | NM_001024912.1 | 1.321 | 1.236 |
| TRIB1 | NM_025195.2 | 1.316 | 1.224 |
| SFXN1 | NM_022754.4 | 1.312 | 1.225 |
| LDHA | NM_005566.1 | 1.302 | 1.215 |
| LINS1 | NM_181740.1 | 1.298 | 1.246 |
| NFKBIZ | NM_001005474.1 | 1.281 | 1.232 |
| SNORA64 | NR_002326.1 | 1.278 | 1.3 |
| CTLA4 | NM_005214.3 | 1.277 | 1.326 |
| B3GNT2 | NM_006577.5 | 1.274 | 1.216 |
| TLR6 | NM_006068.2 | 1.271 | 1.201 |
| TP53INP1 | NM_033285.2 | 1.266 | 1.278 |
| ARID5B | NM_032199.1 | 1.258 | 1.234 |
| NPDC1 | NM_015392.2 | 1.257 | 1.272 |
| P2RY5 | NM_005767.4 | 1.247 | 1.201 |
| PDCD1 | NM_005018.1 | 1.236 | 1.427 |
| SLC2A14 | NM_153449.2 | 1.211 | 1.21 |
| CCDC58 | NM_001017928.2 | 0.811 | 0.815 |
| NOG | NM_005450.2 | 0.779 | 0.802 |
| ID3 | NM_002167.2 | 0.75 | 0.757 |
| CD40LG | NM_000074.2 | 0.739 | 0.81 |
| LRRN3 | NM_001099660.1 | 0.578 | 0.679 |

| | | | |
|---|---|---|---|
| "transcript CR743148 (Illumina Probe ID 6370082) has been retired from NCBI, but the EST corresponds to splice variant(s) within the GPRIN3 gene (chromosome 4.90). | | | |

Biological Functions: proportion of genes in a given gene list assigned particular biological function is given, along with p-value...

| **Cell development** | **T-lymphocyte differentiation** | **T-lymphocyte development** |
|---|---|---|
| 14/40 (p<10e-10) | 7/40 (2.6e-7) | 9/40 (3.14e-7) |
| | | |
| BATF | BCL3 | BCL3 |
| BCL3 | CD40LG | CD40LG |
| CD40LG | CTLA4 | CTLA4 |
| CEACAM1 | ICOS | ICOS |
| CTLA4 | ID3 | ID3 |
| ICOS | NOG | NOG |
| ID3 | SOCS3 | PDCD1 |
| NOG | | SOCS1 |
| PDCD1 | | SOCS3 |
| PRDM1 | | |
| SFXN1 | | |
| SOCS1 | | |
| SOCS2 | | |
| SOCS3 | | |

### Gene List 9 Lists of Functionally-related Genes based on Pathway analysis of Lists 5, 6 and 7 combined (n=197) (Uniquely de-regulated in RA vs OA, but not in inflammatory controls)

### Canonical Pathways. Proportion of genes listed in particular pathway that appear is given in each case, along with p-value for significance...

| T Helper Cell | Cell cycle | Interferon signalling |
|---|---|---|
| Differentiation | | |
| | | |
| 6/41 (p=2.63e-4) | (G2/M DNA damage checkpoint regn) 4/43(p=3.25e-4) | 3/30 (p=1.8e-3) |
| ICA1 | CCB2 | IFNGR1 |
| IFNGR1 | CDKN1A | IFNGR2 |
| IFNGR2 | GADD45A | SOCS1 |
| SOCS1 | TOP2A | |
| SOCS2 | | |
| SOCS3 | | |
| | | |
| IL-9 signalling | JAK/STAT signalling | |
| | | |
| 3/37 (p=2.44e-03) | 4/64 (p=1.97e-03) | |
| | | |
| BCL3 | CDKN1A | |
| SOCS2 | SOCS1 | |
| SOCS3 | SOCS2 | |
| | SOCS3 | |

### Biological Functions: proportion of genes in a given gene list assigned particular biological function is given, along with p-value...

| Cell death 97/197 (p=2.31e-23) | Cell Survival 79/197 (p=2.97e-7) | Cell Proliferation 67/197 (p=2.28e-20) | T-cell proliferation 17/197 (p=2.27e-7) |
|---|---|---|---|
| ACVR1B | ACVR2A | ACVR2A | B3GNT2 |
| ACVR2A | ADM | ADM | BATF |
| ADM | AP3M2 | ARID5B | CD40LG |
| AP3M2 | ATF4 | ATF4 | CDKN1A |
| BAG3 | BCL3 | B3GNT2 | CEACAM1 |
| BCL3 | BCL6 | BATF | CLECL1 |
| BCL6 | CCNB2 | BCL3 | CTLA4 |
| BIRC3 | CD40LG | BCL6 | F2RL1 |
| CCNB2 | CDC20 | CD40LG | GADD45A |
| CD40LG | CDCA5 | CDC45L | ICOS |
| CDC20 | CDCA7 | CDCA7 | IFNGR1 |
| CDC45L | CDKN1A | CDKN1A | IL2RA |
| CDCA5 | CDKN2D | CDKN2D | PDCD1 |
| CDCA7 | CEACAM1 | CEACAM1 | PRDM1 |
| CDK5RAP3 (inct EG:B0279) | CEBPB | CEBPB | SOCS1 |
| CDKN1A | CFD | CLECL1 | SOCS3 |
| CDKN2D | CTLA4 | CRIP2 | TNFSF10 |
| CEACAM1 | DDIT4 | CSDA | |
| CEBPB | FCGBP | CTLA4 | |
| CFD | FKBP5 | DDX11 | |
| CSDA | FURIN | DDX21 | |
| CTLA4 | GADD45A | F2RL1 | |
| DDIT4 | GLRX | FKBP5 | |
| DNAJB1 | GPR132 | FURIN | |
| EDA | GSTM1 | GADD45A | |
| FBXO32 | HBEGF | GNL3 | |
| FCGBP | HERPUD1 | GPR132 | |
| FKBP5 | HIF1A | GSTM1 | |
| FURIN | HLA-DRB4 | HBEGF | |
| GADD45A | ICOS | HIF1A | |
| GIMAP5 | IFI27 | ICOS | |
| GLRX | IFNGR1 | ID3 | |
| GNL3 | IFNGR2 | IFNGR1 | |
| GPR132 | IL2RA | IL16 | |
| GSTM1 | KIAA0101 | IL21R | |
| HBEGF | LDHA | IL2RA | |
| HERPUD1 | LGALS8 | KIAA0101 | |
| HIF1A | MAF | KISS1R | |
| HLA-DRB4 | MAT2A | LDHA | |
| ICOS | MELK | LY96 | |
| ID3 | MT1E | MT2A | |
| IFI27 | MT2A | MUC1 | |
| IFNGR1 | MTHFD2 | MXD1 | |
| IFNGR2 | MUC1 | NAMPT | |
| IL2RA | MXD1 | NCF1 | |
| KIAA0101 | NAMPT | NOG | |
| LDHA | NCAPG (includes EG.64151) | NPDC1 | |
| LGALS8 | NDUFV2 | PDCD1 | |
| LMNB1 | NFIL3 | PIM2 (includes EG:11040) | |
| MAF | NOG | PRDM1 | |
| MAT2A | NPDC1 | PRDX3 | |
| MELK | PIM2 (includes EG:11040) | PRKAR1A | |
| MT1E | PRDX3 | PTGER2 | |
| MT2A | PRKAR1A | PTTG1 | |
| MTHFD2 | PTGER2 | S100P | |
| MUC1 | PTTG1 | SERPINE2 | |
| MXD1 | S100P | SLC7A5 | |
| NAMPT | SDCBP | SOCS1 | |
| NCAPG (includes EG:64151) | SERPINB1 | SOCS2 | |
| NCF1 | SERPINE2 | SOCS3 | |
| NDUFV2 | SLC2A3 | SOD2 | |
| NFIL3 | SLC2A14 | TNFSF10 | |
| NFKBIZ | SLC7A5 | TP53INP1 | |
| NOG | SOCS1 | TRIB1 | |
| NPDC1 | SOCS2 | TYMS | |
| PDCD1 | SOCS3 | UBE2C | |
| PIM2 (includes EG:11040) | SOD2 | UHRF1 | |
| PRDM1 | TK1 | | |
| PRDX3 | TNFSF10 | | |
| PRKAR1A | TOP2A | | |
| PTGER2 | TP53INP1 | | |
| PTRH2 | TRIB1 | | |
| PTTG1 | TYMS | | |
| RYBP | UBE2C | | |
| S100P | UHRF1 | | |
| SDCBP | XAF1 | | |
| SERPINB1 | | | |
| SERPINE2 | | | |
| SLC2A3 | | | |
| SLC2A14 | | | |
| SLC7A5 | | | |
| SOCS1 | | | |
| SOCS2 | | | |
| SOCS3 | | | |
| SOD2 | | | |
| TK1 | | | |
| TLR6 | | | |
| TMEM173 | | | |
| TNFSF10 | | | |
| TOP2A | | | |
| TP53INP1 | | | |
| TRIB1 | | | |
| TRIB3 | | | |
| TYMS | | | |
| UBE2C | | | |
| UHRF1 | | | |
| XAF1 | | | |

| Blood cell differentiation 25/197 (p=4.5e-12) | T-cell differentiation 15/197 (p=3.3e-09) |
|---|---|
| ACVR1B | BCL3 |
| ACVR2A | BCL6 |
| BCL3 | CD40LG |
| BCL26 | CEBPB |
| CD40LG | CTLA4 |
| CDKN2D | GIMAP5 |
| CEBPB | ICOS |
| CTLA4 | ID3 |
| GIMAP5 | IFNGR2 |
| HIF1A | IL21R |
| ICOS | IL2RA |
| ID3 | MAF |
| IFNGR2 | MUC1 |
| IL21R | NOG |
| IL2RA | SOCS3 |
| MAF | |
| MUC1 | |
| NOG | |
| PDCD1 | |
| PRDM1 | |
| PRDX3 | |
| SFXN1 | |
| SOCS1 | |
| SOCS3 | |
| TNFSF10 | |

### REFERENCES

1. Klareskog L, Catrina Al, Paget S, Klareskog L, Catrina Al, Paget S. Rheumatoid arthritis. Lancet 2009;373(9664):659-72.
2. Combe B, Landewe R, Lukas C, Bolosiu HD, Breedveld F, Dougados M, et al. EULAR recommendations for the management of early arthritis: report of a task force of the European Standing Committee for International Clinical Studies Including Therapeutics (ESCISIT).[see comment]. Annals of the Rheumatic Diseases 2007:66(1):34-45.
3. van Gaalen FA, Linn-Rasker SP, van Venrooij WJ, de Jong BA, Breedveld FC, Verweij CL, et al. Autoantibodies to cyclic citrullinated peptides predict progression to rheumatoid arthritis in patients with undifferentiated arthritis: a prospective cohort study. Arthritis & Rheumatism 2004;50(3):709-15.
4. van Der Helm-van Mil AHM, Detert J, Cessie SL, Filer A, Bastian H, Burmester GR, et al. Validation of a prediction rule for disease outcome in patients with recent-onset undifferentiated arthritis: Moving toward individualized treatment decision-making. Arthritis & Rheumatism 2008;58(8):2241-7.
5. Nishimura K, Sugiyama D, Kogata Y, Tsuji G, Nakazawa T, Kawano S, et al. Meta-analysis: diagnostic accuracy of anti-cyclic citrullinated peptide antibody and rheumatoid factor for rheumatoid arthritis.[see comment]. Annals of Internal Medicine 2007;146(11):797-808.
6. Pratt AG, Isaacs JD, Wilson G. The clinical utility of a rule for predicting rheumatoid arthritis in patients with early undifferentiated arthritis: comment on the article by van der Helm-van Mil et al.[comment]. Arthritis & Rheumatism 2009;60(3):905; author reply 906.
7. van't Veer LJ, Bernards R, van't Veer LJ, Bernards R. Enabling personalized cancer medicine through analysis of gene-expression patterns. Nature 2008;452(7187):564-70.
8. Pascual V, Chaussabel D, Banchereau J. A genomic approach to human autoimmune diseases. Annual Review of Immunology 2010;28:535-71.
9. Toonen EJ, Barrera P, Radstake TR, van Riel PL, Scheffer H, Franke B, et al. Gene expression profiling in rheumatoid arthritis: current concepts and future directions. Annals of the Rheumatic Diseases 2008;67(12):1663-9.
10. Lequerre T, Gauthier-Jauneau A-C, Bansard C, Derambure C, Hiron M, Vittecoq O, et al. Gene profiling in white blood cells predicts infliximab responsiveness in rheumatoid arthritis. Arthritis Research & Therapy 2006;8(4):R105.
11. McKinney EF, Lyons PA, Carr EJ, Hollis JL, Jayne DR, Willcocks LC, et al. A CD8+ T cell transcription signature predicts prognosis in autoimmune disease. Nature Medicine 2010;16(5):586-91.
12. van Baarsen LGM, Bos WH, Rustenburg F, van der Pouw Kraan TCTM, Wolbink GJJ, Dijkmans BAC, et al. Gene expression profiling in autoantibody-positive patients with arthralgia predicts development of arthritis. Arthritis & Rheumatism 2010;62(3):694-704.
13. Batliwalla FM, Baechler EC, Xiao X, Li W, Balasubramanian S, Khalili H, et al. Peripheral blood gene expression profiling in rheumatoid arthritis. Genes & Immunity 2005;6(5):388-97.
14. Lyons PA, Koukoulaki M, Hatton A, Doggett K, Woffendin HB, Chaudhry AN, et al. Microarray analysis of human leucocyte subsets: the advantages of positive selection and rapid purification. BMC Genomics 2007;8:64.
15. Koetz K, Bryl E, Spickschen K, O'Fallon WM, Goronzy JJ, Weyand CM. T cell homeostasis in patients with rheumatoid arthritis. Proceedings of the National Academy of Sciences of the United States of America 2000;97(16):9203-8.
16. Ponchel F, Morgan AW, Bingham SJ, Quinn M, Such M, Verburg RJ, et al. Dysregulated lymphocyte proliferation and differentiation in patients with rheumatoid arthritis. Blood 2002;100(13):4550-6.
17. McInnes IB, O'Dell JR. State-of-the-art: rheumatoid arthritis. Annals of the Rheumatic Diseases 2010;69(11):1898-906.
18. Arnett FC, Edworthy SM, Bloch DA, McShane DJ, Fries JF, Cooper NS, et al. The American Rheumatism Association 1987 revised criteria for the classification of rheumatoid arthritis. Arthritis & Rheumatism 1988;31(3):315-24.
19. Schroeder A, Mueller O, Stocker S, Salowsky R, Leiber M, Gassmann M, et al. The RIN: an RNA integrity number for assigning integrity values to RNA measurements. BMC Molecular Biology 2006;7:3.
20. de Jager W, Prakken BJ, Bijlsma JWJ, Kuis W, Rijkers GT. Improved multiplex immunoassay performance in human plasma and synovial fluid following removal of interfering heterophilic antibodies. Journal of Immunological Methods 2005;300(1-2):124-35.
21. Hueber W, Tomooka BH, Zhao X, Kidd BA, Drijfhout JW, Fries JF, et al. Proteomic analysis of secreted proteins in early rheumatoid arthritis: anti-citrulline autoreactivity is associated with up regulation of proinflammatory cytokines. Annals of the Rheumatic Diseases 2007;66(6):712-9.
22. Livak KJ, Schmittgen TD, Livak KJ, Schmittgen TD. Analysis of relative gene expression data using real-time quantitative PCR and the 2(-Delta Delta C(T)) Method. Methods (Duluth) 2001;25(4):402-8.
23. Johnson WE, Li C, Rabinovic A. Adjusting batch effects in microarray expression data using empirical Bayes methods. Biostatistics 2007;8(1):118-27.
24. Du P, Kibbe WA, Lin SM. lumi: a pipeline for processing Illumina microarray. Bioinformatics 2008;24(13):1547-8.
25. Benjamini Y, Hochberg Y. Controlling the false discovery rate: a practical and powerful approach to multiple testing. Journal of the Royal Statistical Society. Series B (Methodological) 1995;57(1):289-300.
26. Cortes C, Vapnik V. Support-vector networks. Machine Learning 1995;20(3):273-97.
27. Owaki T, Asakawa M, Morishima N, Mizoguchi I, Fukai F, Takeda K, et al. STAT3 is indispensable to IL-27-mediated cell proliferation but not to IL-27-induced Th1 differentiation and suppression of proinflammatory cytokine production. Journal of Immunology 2008;180(5):2903-11.
28. Starr R, Willson TA, Viney EM, Murray LJ, Rayner JR, Jenkins BJ, et al. A family of cytokine-inducible inhibitors of signalling. Nature 1997;387(6636):917-21.
29. EI Kasmi KC, Smith AM, Williams L, Neale G, Panopoulos AD, Watowich SS, et al. Cutting edge: A transcriptional repressor and corepressor induced by the STAT3-regulated anti-inflammatory signaling pathway.[erratum appears in J Immunol. 2008 Mar 1;180(5):3612 Note: Panopolous, Athanasia [corrected to Panopoulos, Athanasia D]]. Journal of Immunology 2007;179(11):7215-9.
30. Brocke-Heidrich K, Ge B, Cvijic H, Pfeifer G, Loffler D, Henze C, et al. BCL3 is induced by IL-6 via Stat3 binding to intronic enhancer HS4 and represses its own transcription. Oncogene 2006;25(55):7297-304.
31. Richard M, Louahed J, Demoulin JB, Renauld JC, Richard M, Louahed J, et al. Interleukin-9 regulates NF-kappaB activity through BCL3 gene induction. Blood 1999;93(12):4318-27.
32. Gao J, McConnell MJ, Yu B, Li J, Balko JM, Black EP, et al. MUC1 is a downstream target of STAT3 and regulates lung cancer cell survival and invasion. International Journal of Oncology 2009;35(2):337-45.
33. Akaishi H, Takeda K, Kaisho T, Shineha R, Satomi S, Takeda J, et al. Defective IL-2-mediated IL-2 receptor alpha chain expression in Stat3-deficient T lymphocytes. International Immunology 1998;10(11):1747-51.
34. Matikainen S, Sareneva T, Ronni T, Lehtonen A, Koskinen PJ, Julkunen I, et al. Interferon-alpha activates multiple STAT proteins and upregulates proliferation-associated IL-2Ralpha, c-myc, and pim-1 genes in human T cells. Blood 1999;93(6):1980-91.
35. Nichane M, Ren X, Bellefroid EJ. Self-regulation of Stat3 activity coordinates cell-cycle progression and neural crest specification. EMBO Journal;29(1):55-67.
36. Hirano T, Ishihara K, Hibi M, Hirano T, Ishihara K, Hibi M. Roles of STAT3 in mediating the cell growth, differentiation and survival signals relayed through the IL-6 family of cytokine receptors. Oncogene 2000;19(21):2548-56.
37. Altman DG, Bland JM. Diagnostic tests 2: Predictive values. BMJ 1994;309(6947):102.
38. Goronzy JJ, Weyand CM. Rheumatoid arthritis. Immunological Reviews 2005;204:55-73.
39. Schonland SO, Lopez C, Widmann T, Zimmer J, Bryl E, Goronzy JJ, et al. Premature telomeric loss in rheumatoid arthritis is genetically determined and involves both myeloid and lymphoid cell lineages. Proceedings of the National Academy of Sciences of the United States of America 2003; 100(23): 13471-6.
40. van der Helm-van Mil AHM, Verpoort KN, Breedveld FC, Huizinga TWJ, Toes REM, de Vries RRP. The HLA-DRB1 shared epitope alleles are primarily a risk factor for anti-cyclic citrullinated peptide antibodies and are not an independent risk factor for development of rheumatoid arthritis. Arthritis & Rheumatism 2006;54(4):1117-21.
41. Kokkonen H, Soderstrom I, Rocklov J, Hallmans G, Lejon K, Rantapaa Dahlqvist S. Up-regulation of cytokines and chemokines predates the onset of rheumatoid arthritis. Arthritis & Rheumatism;62(2):383-91.
42. Karlson EW, Chibnik LB, Tworoger SS, Lee IM, Buring JE, Shadick NA, et al. Biomarkers of inflammation and development of rheumatoid arthritis in women from two prospective cohort studies. Arthritis & Rheumatism 2009;60(3):641-52.
43. Schindler CW. Series introduction. JAK-STAT signaling in human disease. Journal of Clinical Investigation 2002;109(9):1133-7:
44. Fonseca JE, Santos MJ, Canhao H, Choy E. Interleukin-6 as a key player in systemic inflammation and joint destruction. Autoimmunity Reviews 2009;8(7):538-42.
45. Nowell MA, Williams AS, Carty SA, Scheller J, Hayes AJ, Jones GW, et al. Therapeutic targeting of IL-6 trans signaling counteracts STAT3 control of experimental inflammatory arthritis. Journal of Immunology 2009; 182(1):613-22.
46. Eyles JL, Hickey MJ, Norman MU, Croker BA, Roberts AW, Drake SF, et al. A key role for G-CSF-induced neutrophil production and trafficking during inflammatory arthritis. Blood 2008;112(13):5193-201.
47. Rho YH, Solus J, Sokka T, Oeser A, Chung CP, Gebretsadik T, et al. Adipocytokines are associated with radiographic joint damage in rheumatoid arthritis. Arthritis & Rheumatism 2009;60(7):1906-14.
48. El Kasmi KC, Smith AM, Williams L, Neale G, Panopoulos AD, Watowich SS, et al. Cutting edge: A transcriptional repressor and corepressor induced by the STAT3-regulated anti-inflammatory signaling pathway.[Erratum appears in J Immunol. 2008 Mar 1;180(5):3612 Note: Panopolous, Athanasia [corrected to Panopoulos, Athanasia D]]. Journal of Immunology 2007;179(11):7215-9.
49. Nishimoto N, Miyasaka N, Yamamoto K, Kawai S, Takeuchi T, Azuma J. Long-term safety and efficacy of tocilizumab, an anti-IL-6 receptor monoclonal antibody, in monotherapy, in patients with rheumatoid arthritis (the STREAM study): evidence of safety and efficacy in a 5-year extension study. Annals of the Rheumatic Diseases 2009;68(10):1580-4.
50. Cohen S, Fleischmann R. Kinase inhibitors: a new approach to rheumatoid arthritis treatment. Current Opinion in Rheumatology 2010;22(3):330-5.
51. Li J. et al. Altered microRNA expression profile with miR-146a upregulated in CD4+ Tcells from patients with rheumatoid arthritis. Arthritis Research & Therapy 2010, 12:R81.

## Claims

1. A method of diagnosing Rheumatoid arthritis in a patient, the method comprising:
obtaining a sample of CD4+ T cells from the patient; and
determining expression levels of all of the genes from the group consisting of
BCL3
SOCS3
PIM1
SBNO2
LDHA
CMAH
NOG
PDCD1
IGFL2
LOC731186
MUC1
GPRIN3; and
comparing said expression levels to reference expression levels, wherein
a difference in expression of said genes indicates an increased likelihood that the patient has Rheumatoid arthritis (RA).

2. A method as in Claim 1 wherein the group further comprises the gene CD40LG.

3. A method as in Claims 1 or 2 wherein the reference expression levels are representative of levels found in samples comprising cells from a patient who does not have Rheumatoid arthritis (RA).

4. A method for typing a sample from an individual, the method comprising:
obtaining a sample of CD4+ T cells from the individual; and
determining expression levels of all of the genes from the group consisting of
BCL3
SOCS3
PIM1
SBNO2
LDHA
CMAH
NOG
PDCD1
IGFL2
LOC731186
MUC1
GPRIN3; and
typing said sample on the basis of the expression levels determined;
wherein said typing provides prognostic information related to the risk that the individual has rheumatoid arthritis (RA).

5. A method as in Claim 4 wherein the group further comprises the gene CD40LG.

6. A method as in any of the previous Claims wherein expression levels are determined by determining RNA levels.

7. A method as in any of the previous Claims wherein the CD4+ T cells sample is obtained from peripheral whole blood.

8. A method as in Claim 7 which includes the step of separating CD4+ T cells from peripheral whole blood.

9. A method as in Claim 7 which includes the step of extracting RNA from the CD4+ T cells.

10. Methods as in any of the previous Claims further comprising the step of combining the results with the results of known prediction analysis.

11. A method as in Claim 10 wherein the known prediction analysis is the Leiden prediction rule.

12. A method of diagnosing rheumatoid arthritis in a patient, the method comprising:
obtaining a blood sample from the patient; and
determining expression/mRNA levels of all of the genes from the group consisting of
BCL3
SOCS3
PIM1
SBNO2
LDHA
CMAH
NOG
PDCD1
IGFL2
LOC731186
MUC1
GPRIN3; and
comparing said expression/mRNA levels to a set of reference expression/mRNA levels, wherein a difference in expression of said genes indicates an increased likelihood that the patient has Rheumatoid arthritis.

13. Use of a set of probes consisting solely of polynucleotides specific for the genes
BCL3
SOCS3
PIM1
SBNO2
LDHA
CMAH
NOG
PDCD1
IGFL2
LOC731186
MUC1
GPRIN3
for determining the risk of an individual for suffering from rheumatoid arthritis.

14. Use of a set of probes as in claim 13 said probes further comprising a polynucleotide specific for CD40LG.

## Patentansprüche

1. Ein Verfahren zur Diagnose von rheumatoider Arthritis bei einem Patienten, wobei das Verfahren Folgendes beinhaltet:
Erhalten einer Probe von CD4+ T-Zellen von dem Patienten; und
Bestimmen der Expressionsniveaus aller Gene aus der Gruppe, bestehend aus BCL3
SOCS3
PIM1
SBNO2
LDHA
CMAH
NOG
PDCD1
IGFL2
LOC731186
MUC1
GPRIN3; und
Vergleichen der Expressionsniveaus mit Referenzexpressionsniveaus, wobei ein Unterschied in der Expression der Gene eine erhöhte Wahrscheinlichkeit, dass der Patient rheumatoide Arthritis (RA) hat, angibt.

2. Verfahren gemäß Anspruch 1, wobei die Gruppe ferner das Gen CD40LG beinhaltet.

3. Verfahren gemäß Anspruch 1 oder 2, wobei die Referenzexpressionsniveaus repräsentativ für Niveaus sind, die in Proben gefunden werden, welche Zellen von einem Patienten beinhalten, der nicht rheumatoide Arthritis (RA) hat.

4. Ein Verfahren zur Typisierung einer Probe von einem Individuum, wobei das Verfahren Folgendes beinhaltet:
Erhalten einer Probe von CD4+ T-Zellen von dem Individuum; und
Bestimmen der Expressionsniveaus aller Gene aus der Gruppe, bestehend aus BCL3
SOCS3
PIM1
SBNO2
LDHA
CMAH
NOG
PDCD1
IGFL2
LOC731186
MUC1
GPRIN3; und
Typisieren der Probe auf der Basis der bestimmten Expressionsniveaus;
wobei die Typisierung prognostische Informationen in Bezug auf das Risiko liefert, dass das Individuum rheumatoide Arthritis (RA) hat.

5. Verfahren gemäß Anspruch 4, wobei die Gruppe ferner das Gen CD40LG beinhaltet.

6. Verfahren gemäß einem der vorhergehenden Ansprüche, wobei die Expressionsniveaus durch Bestimmen der RNA-Niveaus bestimmt werden.

7. Verfahren gemäß einem der vorhergehenden Ansprüche, wobei die Probe der CD4+ T-Zellen aus peripherem Vollblut erhalten wird.

8. Verfahren gemäß Anspruch 7, das den Schritt des Trennens von CD4+ T-Zellen von peripherem Vollblut umfasst.

9. Verfahren gemäß Anspruch 7, das den Schritt des Extrahierens von RNA aus den CD4+ T-Zellen umfasst.

10. Die Verfahren gemäß einem der vorhergehenden Ansprüche, die ferner den Schritt des Kombinierens der Ergebnisse mit den Ergebnissen bekannter Vorhersageanalysen beinhalten.

11. Verfahren gemäß Anspruch 10, wobei die bekannte Vorhersageanalyse die Leiden-Vorhersageregel ist.

12. Ein Verfahren zur Diagnose von rheumatoider Arthritis bei einem Patienten, wobei das Verfahren Folgendes beinhaltet:
Erhalten einer Blutprobe von dem Patienten; und
Bestimmen der Expression/mRNA-Niveaus aller Gene aus der Gruppe, bestehend aus BCL3
SOCS3
PIM1
SBNO2
LDHA
CMAH
NOG
PDCD1
IGFL2
LOC731186
MUC1
GPRIN3; und
Vergleichen der Expression/mRNA-Niveaus mit einem Satz Referenz-Expression/mRNA-Niveaus, wobei ein Unterschied in der Expression der Gene eine erhöhte Wahrscheinlichkeit, dass der Patient rheumatoide Arthritis hat, angibt.

13. Eine Verwendung eines Satzes Sonden, bestehend ausschließlich aus Polynukleotiden, die für die folgenden Gene spezifisch sind:
BCL3
SOCS3
PIM1
SBNO2
LDHA
CMAH
NOG
PDCD1
IGFL2
LOC731186
MUC1
GPRIN3,
zum Bestimmen des Risikos, dass ein Individuum an rheumatoider Arthritis leidet.

14. Verwendung eines Satzes Sonden gemäß Anspruch 13, wobei die Sonden ferner einen Polynukleotiden beinhalten, der für CD40LG spezifisch ist.

## Revendications

1. Une méthode de diagnostic d'une polyarthrite rhumatoïde chez un patient, la méthode comprenant :
l'obtention d'un échantillon de cellules T CD4+ prélevées sur le patient ; et
la détermination de niveaux d'expression de tous les gènes du groupe consistant en BCL3
SOCS3
PIM1
SBNO2
LDHA
CMAH
NOG
PDCD1
IGFL2
LOC731186
MUC1
GPRIN3 ; et
la comparaison desdits niveaux d'expression avec des niveaux d'expression de référence, dans laquelle une différence dans l'expression desdits gènes indique une possibilité accrue que le patient présente une polyarthrite rhumatoïde (RA).

2. Une méthode telle que dans la revendication 1 dans laquelle le groupe comprend en sus le gène CD40LG.

3. Une méthode telle que dans les revendications 1 ou 2 dans laquelle les niveaux d'expression de référence sont représentatifs de niveaux trouvés dans des échantillons comprenant des cellules prélevées sur un patient qui ne présente pas de polyarthrite rhumatoïde (RA).

4. Une méthode pour typer un échantillon provenant d'un individu, la méthode comprenant :
l'obtention d'un échantillon de cellules T CD4+ prélevées sur l'individu ; et
la détermination de niveaux d'expression de tous les gènes du groupe consistant en BCL3
SOCS3
PIM1
SBNO2
LDHA
CMAH
NOG
PDCD1
IGFL2
LOC731186
MUC1
GPRIN3 ; et
le typage dudit échantillon sur la base des niveaux d'expression déterminés ;
dans laquelle ledit typage fournit une information de prognostic concernant le risque que l'individu présente une polyarthrite rhumatoïde (RA).

5. Une méthode telle que dans la revendication 4, dans laquelle le groupe comprend en sus le gène CD40LG.

6. Une méthode telle que dans n'importe lesquelles des revendications précédentes dans laquelle des niveaux d'expression sont déterminés en déterminant des niveaux d'ARN.

7. Une méthode telle que dans n'importe lesquelles des revendications précédentes dans laquelle l'échantillon de cellules T CD4+ est obtenu à partir de sang périphérique entier.

8. Une méthode telle que dans la revendication 7 qui inclut l'étape consistant à séparer des cellules T CD4+ du sang périphérique entier.

9. Une méthode telle que dans la revendication 7 qui inclut l'étape consistant à extraire l'ARN des cellules T CD4+.

10. Des méthodes telles que dans n'importe lesquelles des revendications précédentes comprenant en sus l'étape consistant à combiner les résultats avec les résultats d'analyse de prédiction connue.

11. Une méthode telle que dans la revendication 10 dans laquelle l'analyse de prédiction connue est la régle de prédiction de Leiden.

12. Une méthode de diagnostic d'une polyarthrite rhumatoïde chez un patient, la méthode comprenant :
l'obtention d'un échantillon de sang prélevé sur le patient ; et
la détermination de niveaux d'expression/d'ARNm de tous les gènes du groupe consistant en
BCL3
SOCS3
PIM1
SBNO2
LDHA
CMAH
NOG
PDCD1
IGFL2
LOC731186
MUC1
GPRIN3 ; et
la comparaison desdits niveaux d'expression/d'ARNm avec un jeu de niveaux d'expression/d'ARNm de référence, dans laquelle une différence dans l'expression desdits gènes indique une possibilité accrue que le patient présente une polyarthrite rhumatoïde.

13. Utilisation d'un jeu de sondes consistant uniquement en polynucléotides spécifiques pour les gènes
BCL3
SOCS3
PIM1
SBNO2
LDHA
CMAH
NOG
PDCD1
IGFL2
LOC731186
MUC1
GPRIN3
pour déterminer le risque pour un individu de souffrir de polyarthrite rhumatoïde.

14. Utilisation d'un jeu de sondes telle que dans la revendication 13, lesdites sondes comprenant en sus un polynucléotide spécifique pour CD40LG.
